(19) Europäisches Patentamt / European Patent Office / Office européen des brevets

(11) EP 1 841 464 B1

(12) EUROPEAN PATENT SPECIFICATION

(45) Date of publication and mention of the grant of the patent: **27.06.2012 Bulletin 2012/26**

(21) Application number: **06733815.2**

(22) Date of filing: **24.01.2006**

(51) Int Cl.: *C12N 15/113* (2010.01) *A61P 25/00* (2006.01)

(86) International application number: **PCT/US2006/002315**

(87) International publication number: **WO 2006/081192 (03.08.2006 Gazette 2006/31)**

(54) **RNAI MODULATION OF THE NOGO-L OR NOGO-R GENE AND USES THEREOF**

RNAI-MODULATION DES NOGO-L ODER NOGO-R-GENS UND DESSEN VERWENDUNGEN

MODULATION ARNI DU GENE NOGO-L OU NOGO-R ET UTILISATIONS

(84) Designated Contracting States: **AT BE BG CH CY CZ DE DK EE ES FI FR GB GR HU IE IS IT LI LT LU LV MC NL PL PT RO SE SI SK TR**

(30) Priority: **24.01.2005 US 646353 P**
**21.07.2005 US 701470 P**
**14.10.2005 US 726838 P**
**07.12.2005 US 748316 P**

(43) Date of publication of application: **10.10.2007 Bulletin 2007/41**

(73) Proprietor: **Alnylam Pharmaceuticals Inc.**
**Cambridge, MA 02142 (US)**

(72) Inventors:
- **SOUTSCHEK, Juergen**
  **95359 Kasendorf (DE)**
- **VORNLOCHER, Hans-Peter**
  **95448 Bayreuth (DE)**

(74) Representative: **Vossius & Partner**
**Siebertstrasse 4**
**81675 München (DE)**

(56) References cited:
**WO-A2-01/59103** **WO-A2-02/081628**
**WO-A2-2004/096846** **US-A1- 2005 182 008**
**US-A1- 2005 261 212**

- **AHMED Z ET AL: "Disinhibition of neurotrophin-induced dorsal root ganglion cell neurite outgrowth on CNS myelin by siRNA-mediated knockdown of NgR, p75<NTR> and Rho-A" MOLECULAR AND CELLULAR NEUROSCIENCES, SAN DIEGO, US LNKD- DOI: 10.1016/J.MCN.2004.11.002, vol. 28, no. 3, 1 March 2005 (2005-03-01), pages 509-523, XP004767191 ISSN: 1044-7431**

EP 1 841 464 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

Printed by Jouve, 75001 PARIS (FR)

## Description

## TECHNICAL FIELD

The invention relates to compositions and methods for modulating the expression of Nogo-L or Nogo-R, and more particularly to the downregulation ofNogo-L or Nogo-R mRNA and Nogo-L or Nogo-R protein levels by oligonucleotides via RNA interference, *e.g.*, chemically modified oligonucleotides.

## BACKGROUND

RNA interference or "RNAi" is a term initially coined by Fire and co-workers to describes the observation that double-stranded RNA (dsRNA) can block gene expression when it is introduced into worms (Fire et al., Nature 391: 806-811, 1998). Short dsRNA directs gene-specific, post-transcriptional silencing in many organisms, including vertebrates, and has provided a new tool for studying gene function.

Numerous myelin-derived axon growth inhibitors have been characterized (see, for review, David et al., W0995394547, 1999; Bandman et al. U.S. Pat No. 5,858,708, 1999; Schwab, Neurochem. Res. 21:755-761, 1996). Several components of CNS white matter, NI35, NI250 (Nogo) and Myelin-associated glycoprotein (MAG), which have inhibitory activity for axonal extension, have been described as well (Schwab et al., WO9005191, 1990; Schwab et al., U.S. Pat No. 5,684,133,1997). In particular, Nogo is a 250 kDa myelin-associated axon growth inhibitor that was originally characterized based on the effects of the purified protein *in vitro* and monoclonal antibodies that neutralize the protein's activity (Schwab, *Exp. Neurol.* 109:2-5). The Nogo cDNA was first identified through random analysis of brain cDNA and had no suggested function (Nagase et al., DNA Res. 5:355-364, 1998). The identification of this Nogo cDNA as the cDNA encoding the 250 kDa myelin-associated axon growth inhibitor was discovered only recently (GrandPre *et al.,* 403:439-444, 2000; Chen et al., Nature 403:434-439, 2000; Prinjha et al., Nature 403:383-384, 2000).

Recently, the cloning of NOGO-A (Genbank Accession No. AJ242961), the rat complementary DNA (cDNA) encoding NI-220/250 has been reported (Chen et al., Nature, 403:434-439, 2000). The NOGO gene encodes at least three major protein products (NOGO-A, NOGO-B, and NOGO-C) resulting from both alternative promoter usage and alternative splicing. Recombinant NOGO-A inhibits neurite outgrowth from dorsal root ganglia and the spreading of 3T3 fibroblasts. Monoclonal antibody IN-1 recognizes NOGO-A and neutralizes NOGO-A inhibition of neuronal growth *in vitro.* Evidence supports the proposal that NOGO-A is the previously described rat NI-250 since NOGO-A contains all six peptide sequences obtained from purified bNI-220, the bovine equivalent of rat NI-250 (Chen *et al., supra*).

Prinjha et al. (Nature 403:383-384, 2000), report the cloning of the human NOGO gene which encodes three different NOGO isoforms that are potent inhibitors of neurite outgrowth. Using oligonucleotide primers to amplify and clone overlapping regions of the open reading frame of NOGO cDNA, Phrinjha *et al.* (*supra*) identified three forms of cDNA clone corresponding to the three protein isoforms. The longest ORF of 1,192 amino acids corresponds to NOGO-A (Accession No. AJ251383). An intermediate-length splice variant that lacks residues 186-1,004 corresponds to NOGO-B (Accession No. AJ251384). The shortest splice variant, NOGO-C (Accession No. AJ251385), appears to be the previously described rat vp20 (Accession No. AF051335) and foocen-s (Accession No. AF132048), and also lacks residues 186-1,004. According to Prinjha *et al.* (*supra*), the NOGO amino-terminal region shows no significant homology to any known protein, while the carboxy-terminal tail shares homology with neuroendocrine-specific proteins and other members of the reticulon gene family. In addition, the carboxy-terminal tail contains a consensus sequence that may serve as an endoplasmic-reticulum retention region. Based on the NOGO protein sequence, Prinjha *et al.* (*supra*), postulate NOGO to be a membrane associated protein comprising a putative large extracellular domain of 1,024 residues with seven predicted N-linked glycosylation sites, two or three transmembrane domains, and a short carboxy-terminal region of 43 residues.

Grandpre et al. (Nature 403:439-44, 2000) also report the identification of NOGO as a potent inhibitor of axon regeneration. The 4.1 kilobase NOGO human cDNA clone identified by Grandpre *et al.* (*supra*), KIAA0886, is homologous to a cDNA derived from a previous effort to sequence random high molecular-weight brain derived cDNAs (Nagase et al., DNA Res., 31:355-364, 1998). This cDNA clone encodes a protein that matches all six of the peptide sequences derived from bovine NOGO. Grandpre *et al.* (*supra*) demonstrate that NOGO expression is predominantly associated with the CNS and not the peripheral nervous system (PNS). Cellular localization of NOGO protein appears to be predominantly reticular in origin, however, NOGO is found on the surface of some oligodendrocytes. An active domain of NOGO has been identified, defined as residues 31-55 of a hydrophilic 66-residue lumenal/extracellular domain. A synthetic fragment corresponding to this sequence exhibits growth-cone collapsing and outgrowth inhibiting activities (Grandpre *et al., supra*).

A receptor for the NOGO-A extracellular domain (NOGO-66) is described in Fournier et al. (Nature 409:341-346, 2001). Fournier *et al.*, have shown that isolated NOGO-66 inhibits axonal extension but does not alter non-neuronal cell morphology. The receptor identified has a high affinity for soluble NOGO-66, and is expressed as a glycophosphatidylinositol-linked protein on the surface of CNS neurons. Since Nogo-R lacks a cytoplasmic domain, it was predicted early on that it signals through the action of coreceptors (Fournier *et al.,* supra), one of which was recently identified as the neurotrophin receptor p75NTR (Wang, K.C., et al., Nature 2002, 420:74; Wong, S.T., et al., Nat. Neurosci. 2002, 5: 1302). Nogo-R forms together with p75 neurotrophin receptor (p75NTR), low affinity neurotrophin receptor (Lingo-1) and TROY (also known as TAJ) a functional complex that mediates the neurite growth inhibitory effects of its ligands (Mueller, K., et al., Nature Reviews 2005, 4:387). p75NTR provides the cytoplasmic effector domain which Nogo-R so conspicuously lacks.

The expression of the NOGO-66 receptor in neurons that are NOGO insensitive results in NOGO dependent inhibition of axonal growth in these cells. Cleavage of the NOGO-66 receptor and other glycophosphatidylinositol-linked proteins from axonal surfaces renders neurons insensitive to NOGO-66 inhibition. As such, disruption of the interaction between NOGO-66 and the NOGO-66 receptor provides the possibility of treating a wide variety of neurological diseases, injuries, and conditions.

Importantly, Nogo has been shown to be the primary component of CNS myelin responsible for inhibiting axonal elongation and regeneration. Nogo's selective expression by oligodendrocytes and not by Schwann cells (the cells that myelinate PNS axons) is consistent with the inhibitory effects of CNS myelin, in contrast to PNS myelin (GrandPre et al., Nature 403:434-439, 2000). In culture, Nogo inhibits axonal elongation and causes growth cone collapse (Spillmann et al., J. Biol. Chem. 272:19283-19293, 1998). Antibodies (*e.g.*, IN-1) against Nogo have been shown to block most of the inhibitory action of CNS myelin on neurite growth *in vitro* (Spillmann et al., J. Biol. Chem. 272:19283-19293, 1998). These experiments indicate that Nogo is one of the main components of CNS myelin responsible for inhibition of axonal elongation in culture. Furthermore, *in vivo,* the IN-1 antibody has been shown to enhance axonal regeneration after spinal cord injury, resulting in recovery of behaviors such as contact placing and stride length (Schnell and Schwab, Nature 343:269-272, 1990; Bregman et al., Nature 378:498-501, 1995). Thus, there is substantial evidence that Nogo is a disease-relevant molecular target.

Modulation of Nogo has been described as a means for treatment of regeneration for neurons damaged by trauma, infarction and degenerative disorders of the CNS (Schwab et al., WO94/17831; Tatagiba et al., Neurosurgery 40:541-546, 1997) as well as malignant tumors in the CNS such as glioblastoma (Schwab et al., U.S. Pat. No. 5,250,414; Schwab et al., U.S. Pat. No. 6,025,333).

Antibodies which recognize Nogo have been suggested to be useful in the diagnosis and treatment of nerve damage resulting from trauma, infarction and degenerative disorders of the CNS (Schnell and Schwab, Nature 343: 269-272, 1990; Schwab et al., U.S. Pat. No. 5,684,133, 1997). For CNS axons, there is a correlation between the presence of myelin and the inhibition of axon regeneration over long distances (Savio and Schwab, Proc. Natl. Acad. Sci. 87:4130-4133, 1990; Keirstead et al., Proc. Natl. Acad. Sci. 89:11664-11668, 1992). After Nogo is blocked by antibodies, neurons can again extend across lesions caused by nerve damage (Schnell and Schwab, Nature 343: 269-272, 1990).

In addition, oligonucleotide based therapeutic approaches using ribozymes have suggested that the Nogo-Receptor (Nogo-R) and Nogo-Ligands (Nogo-L) can serve as targets for such a therapeutic intervention strategy, for example see US 20030113891 and US 20030060611. As used herein, Nogo-Ligands can refer to NOGO-A, NOGO-B, or NOGO-C.

While both MAG and Nogo-66, as well as oligodendrocyte-myelin glycoprotein, act through the Nogo-receptor, there are additional inhibitory mechanisms that act through different receptors. However, both Nogo- R dependent and Nogo-R independent mechanisms signal to activate RhoA, a small intracellular GTPAse (Dubreuil, C.L, et al, J. Cell BioL 2003, 162:233).

Evidently, Nogo-L and Nogo-R are potential targets for therapeutic intervention strategies aimed at diseases and conditions involving, e.g., the destruction and/or impaired regeneration of cells of the CNS. The present invention advances the art by providing methods and medicaments encompassing short dsRNAs leading.to the dowaregulation of Nogo-L or Nogo-R mRNA and protein levels in cells expressing the Nogo-L or Nogo-R genes. These methods and medicaments may be used in the treatment of disorders or pathological processes mediated, at least in part, by Nogo-L or Nogo-R, *e.g.*, by preventing the Nogo-L or Nogo-R inhibition of axonal elongation and regeneration, and consequently stimulating nerve growth and proliferation.

## SUMMARY

The present invention is based, at least in part, on an investigation of the Nogo-L or Nogo-R gene using iRNA agents and further testing of the iRNA agents that target the Nogo-L or Nogo-R site. The present invention provides compositions and methods that are useful in reducing Nogo-L or Nogo-R mRNA levels, Nogo-L or Nogo-R protein levels and the treatment of pathological process mediated, at least in part, by Nogo-L or Nogo-R, *e.g.* preventing Nogo-L or Nogo-R inhibition of axonal elongation and regeneration, in a subject, *e.g.*, a mammal, such as a human. To more detail, the prevent invention is defined by the claims.

According to the invention, an iRNA agent comprises (i) a sense strand, wherein said sense strand consists of the sequence of SEQ ID NO: 1545, (ii) an antisense strand, wherein said antisense strand consists of the sequence of SEQ ID NO: 1546.

Described herein are iRNA agents comprising a sense strand, wherein the sense strand comprises at least 15 contiguous nucleotides that differ by no more than 1, 2, or 3 nucleotides from the sense strand sequences of any one agent selected from the group consisting of: agents number 6000 - 6476 and 6837 as given in Table 1 and Table 2 below, and an antisense strand, wherein the antisense strand comprises at least 15 contiguous nucleotides that differ by no more than 1, 2, or 3 nucleotides from the antisense sequences of any one agent selected from the group consisting of: agents number 6000 - 6476 and 6837.

Furthermore described herein are iRNA agents including a sense strand, wherein the sense strand comprises at least 15 contiguous nucleotides that differ by no more than 1, 2, or 3 nucleotides from the sense strand sequences of any one agent selected from the group consisting of: agents number 6000 to 6029, and an antisense strand wherein the antisense strand comprises at least 15 contiguous nucleotides of the antisense sequences of any one agent selected from the group consisting of agents number 6000 to 6029, and wherein the iRNA agent reduces the amount of Nogo-R or Nogo-A mRNA present in cultured human cells after incubation with these agents by more than 40 % compared to cells which have not been incubated with the agent

Also described herein are iRNA agents comprising a sense strand and an antisense strand each comprising a sequence of at least 16, 17 or 18 nucleotides which is essentially identical to one of the sequences of any one agent selected from the group consisting of: agents number 6000 to 6029, except that not more than 1, 2 or 3 nucleotides per strand, respectively, have been substituted by other nucleotides *(e.g.* adenosine replaced by uracil), while essentially retaining the ability to inhibit Nogo-R or Nogo-A expression. Preferably, such iRNA agents are chosen from the group consisting of: agent numbers 6000, 6017, 6021, 6027, and 6029. More preferably the iRNA agent is chosen from the group consisting of: AL-DP-5870, AL-DP-5871, AL-DP-5872, AL-DP-5873, AL-DP-5876, AL-DP-5883, AL-DP-5884.

Also described herein are iRNA agents including a sense strand, wherein the sense strand comprises at least 15 contiguous nucleotides that differ by no more than 1, 2, or 3 nucleotides from the sense strand sequences of any one agent selected from the group consisting of: agents number 6030 to 6476 and 6837, and an antisense strand wherein the antisense strand comprises at least 15 contiguous nucleotides of the antisense sequences of any one agent selected from the group consisting of: agents number 6030 to 6476 and 6837, and wherein the iRNA agent reduces the amount of Nogo-L mRNA present in cultured human cells after incubation with these agents by more than 40 % compared to cells which have not been incubated with the agent.

Also described herein are iRNA agents comprising a sense strand and an antisense strand each comprising a sequence of at least 16, 17 or 18 nucleotides which is essentially identical to one of the sequences of any one agent selected from the group consisting of: agents number 6030 to 6476 and 6837, except that not more than 1, 2 or 3 nucleotides per strand, respectively, have been substituted by other nucleotides (*e.g.* adenosine replaced by uracil), while essentially retaining the ability to inhibit Nogo-L expression. Preferably, such iRNA agents are chosen from the group consisting of: agent numbers 6142, 6160, 6151, 6152, 6156, 6158, 6162, 6170, 6173, 6174, 6187, 6188, 6189, 6191, 6195, 6197, 6199, 6201, 6203, 6209, 6256, 6306, 6308, 6327, 6329, 6344, 6366, 6368, 6378, 6380, 6382, 6408, 6410, 6418, 6419, and 6837. More preferably, they are chosen from the group consisting of: AL-DP-5920, AL-DP-5921, AL-DP-5922, AL-DP-5924, AL-DP-5925, AL-DP-5926, AL-DP-5927, AL-DP-5928, AL-DP-5929, AL-DP-5930, AL-DP-5931, AL-DP-5932, AL-DP-5933, AL-DP-5934, AL-DP-5935, AL-DP-5936, AL-DP-5938, AL-DP-5939, AL-DP-5942, AL-DP-5945, AL-DP-5946, AL-DP-5947, AL-DP-5948, AL-DP-5949, AL-DP-5950, AL-DP-5951, AL-DP-5953, AL-DP-5954, AL-DP-5995, AL-DP-5957, AL-DP-5959, AL-DP-5960, AL-DP-5961, AL-DP-5964, AL-DP-5965, AL-DP-5966. DP-5965, AL-DP-5966.

In the iRNA agents described herein, the antisense RNA strand may be 30 or fewer nucleotides in length, and the duplex region of the iRNA agent may be 15 - 30 nucleotide pairs in length. These iRNA agents may comprise a modification that causes the iRNA agent to have increased stability in a biological sample. For example, they may comprise a phosphorothioate or a 2'-modified nucleotide. A 2'-modified nucleotide according to the instant invention may comprise at least one 5'-uridine-adenine-3' (5'-ua-3') dinucleotide wherein the uridine is a 2'-modified nucleotide; at least one 5'-uridine-guanine-3' (5'-ug-3') dinucleotide, wherein the 5'-uridine is a 2'-modified nucleotide; at least one 5'-cytidine-adenine-3' (5'-ca-3') dinucleotide, wherein the 5'-cytidine is a 2'-modified nucleotide; or at least one 5'-uridine-uridine-3' (5'-uu-3') dinucleotide, wherein the 5'-uridine is a 2'-modified nucleotide.

The iRNA agents described herein may be designed such that

every 5'-nucleotide in 5'-ua-3', 5'-uu-3', 5'-ca-3', and 5'-ug-3' motifs is a 2'-modified in sense strand, and every 5'-nucleotide in 5'-ua-3' and 5'-ca-3' motifs is 2'-modified in antisense strand, or

every 5'-nucleotide in 5'-ua-3', 5'-uu-3', 5'-ca-3', and 5'-ug-3' motifs is 2'-modified in the sense and antisense strand, or

every pyrimidine nucleotide is 2'-modified in the sense strand, and every 5'-nucleotide in 5'-ua-3' and 5'-ca-3' motifs is 2'-modified in the antisense strand, or

every pyrimidine nucleotide is 2'-modified in sense strand, and every 5'-nucleotide in 5'-ua-3', 5'-uu-3', 5'-ca-3', and 5'-ug-3' motifs is 2'-modified in the antisense strand, or

every pyrimidine nucleotide in the sense strand is 2'-modified, and no nucleotide is 2'-modified in the antisense strand.

The 2'-modification in the iRNA agents described herein may be selected from the group consisting of: 2'-deoxy, 2'-deoxy-2'-fluoro, 2'-O-methyl, 2'-O-methoxyethyl (2'-O-MOE), 2'-O-aminopropyl (2'-O-AP), 2'-O-dimethylaminoethyl (2'-O-DMAOE), 2'-O-dimetlxylaminopropyl (2'-O-DMAP), 2'-O-dimethylaminoethyloxyethyl (2'-O-DMAEOE), and 2'-O-N-methylacetamido (2'-O-NMA).

The iRNA agents described herein may comprise a nucleotide overhang having 1 to 4 unpaired nucleotides, preferably 2 or 3 unpaired nucleotides. The nucleotide overhang may be at the 3'-end of the antisense strand of the iRNA agent. The iRNA agents may comprise a cholesterol moiety, which is preferably conjugated to the 3'-end of the sense strand of the iRNA agent. In a preferred embodiment, the iRNA agent is targeted for uptake by nerve cells or nerve sheath cells.

Further described herein are methods for reducing the level of Nogo-L or Nogo-R mRNA in a cell. The present methods utilize the cellular mechanisms involved in RNA interference to selectively degrade Nogo-L or Nogo-R mRNA in a cell and are comprised of the step of contacting a cell with one of the iRNA agents of the present invention. Such methods can be performed directly on a cell or can be performed on a mammalian subject by administering to a subject one of the iRNA agents described herein. Reduction of Nogo-L or Nogo-R mRNA in a cell results in a reduction in the amount of Nogo-L or Nogo-R protein produced, and in an organism, may result in a decrease in Nogo-L or Nogo-R specific pathological/disease effects, e.g. preventing Nogo-L or Nogo-R inhibition of axonal elongation and regeneration.

Also described herein is a method of treating a human subject having a pathological process mediated in part by Nogo-R is provided, comprising administering an iRNA agent, wherein the iRNA agent comprises a sense strand wherein the sense strand comprises at least 15 contiguous nucleotides that differ by no more than 1, 2, or 3 nucleotides from the sense strand sequences any one of the agents, agent numbers 6000 to 6029, and an antisense strand, wherein the antisense strand comprises at least 15 contiguous nucleotides that differ by no more than 1, 2, or 3 nucleotides from the antisense strand sequences of any one of the agents, agent numbers 6000 to 6029.

Also described herein is a method of treating a human subject having a pathological process mediated in part by Nogo-L is provided, comprising administering an iRNA agent, wherein the iRNA agent comprises a sense strand wherein the sense strand comprises at least 15 contiguous nucleotides that differ by no more than 1, 2, or 3 nucleotides from the sense strand sequences any one of the agents, agent numbers 6030 to 6476 and 6837, and an antisense strand, wherein the antisense strand comprises at least 15 contiguous nucleotides that differ by no more than 1, 2, or 3 nucleotides from the antisense strand sequences of any one of the agents, agent numbers 6030 to 6476 and 6837.

In one embodiments of the methods, the pathological process is the inhibition of nerve growth or elongation, preferably as a result of nerve injury or damage. The iRNA agent may be administered in an amount sufficient to reduce the expression of Nogo-R in a cell or tissue of the subject, or sufficient to reduce the expression of Nogo-L in a cell or tissue of the subject, as the case may be Preferably, the subject is a human.

In another aspect, the instant invention provides pharmaceutical compositions, comprising:

a.) an iRNA agent according to the invention as defined herein above and

b.) a pharmaceutically acceptable carrier

According by also described herein is a method of making a pharmaceutical composition, comprising formulating an iRNA agent described herein with a pharmaceutically acceptable carrier.

In yet another-apect, the invention provides a method of reducing the expression of a Nogo-L or Nogo-A gene in a cell comprising providing an iRNA agent to said cell, wherein said iRNA agent is the above defined iRNA agent according to the invention, wherein said iRNA agent is contacted with said cell which is outside an

Also described herein is a method of reducing the expression of a Nogo-R gene in a cell comprising providing an iRNA agent to said cell, wherein the iRNA agent comprises a sense strand wherein the sense strand comprises at least 15 contiguous nucleotides that differ by no more than 1, 2, or 3 nucleotides from the sense strand sequences any one of the agents, agent numbers 6000 to 6029, and an antisense strand, wherein the antisense strand comprises at least 15 contiguous nucleotides that differ by no more than 1,2, or 3 nucleotides from the antisense strand sequences of any one of the agents, agent numbers 6000 to 6029.

With regard to the above described methods of reducing the expression of a gene, said iRNA agent may be administered to an organism. Alternatively, the cell may be outside an organism. For example, said cell may be a cell of a cell line.

In yet another aspect, the invention provides a kit comprising an iRNA agent of the invention, as defined herein above a sterile container in which the iRNA agent is disclosed, and instructions for use.

The methods and compositions described herein *e.g.*, the methods and iRNA compositions can be used with any dosage and/or formulation described herein, as well as with any route of administration described herein.

The details of one or more methods and compositions described herein are set forth in the description below. Other features, objects, and advantages of the methods and compositions described herein will be apparent from this description and from the claims.

## BRIEF DESCRIPTION OF DRAWING

FIG. 1 is a schematic illustrating the synthesis and structure of cholesterol conjugated RNA strands. The sphere represents the solid phase (controlled pore glass, CPG).

## DETAILED DESCRIPTION

For ease of exposition the term "nucleotide" or "ribonucleotide" is sometimes used herein in reference to one or more monomeric subunits of an RNA agent. It will be understood that the usage of the term "ribonucleotide" or "nucleotide" herein can, in the case of a modified RNA or nucleotide surrogate, also refer to a modified nucleotide, or surrogate replacement moiety, as further described below, at one or more positions.

An "RNA agent" as used herein, is an unmodified RNA, modified RNA, or nucleoside surrogate, each of which is describe herein or is well known in the RNA synthetic art. While numerous modified RNAs and nucleoside surrogates are described, preferred examples include those which have greater resistance to nuclease degradation than do unmodified RNAs. Preferred examples include those that have a 2' sugar modification, a modification in a single strand overhang, preferably a 3' single strand overhang, or, particularly if single stranded, a 5'-modification which includes one or more phosphate groups or one or more analogs of a phosphate group.

An "iRNA agent" (abbreviation for "interfering RNA agent") as used herein, is an RNA agent, which can downregulate the expression of a target gene, *e.g.*, Nogo-L or Nogo-R. While not wishing to be bound by theory, an iRNA agent may act by one or more of a number of mechanisms, including post-transcriptional cleavage of a target mRNA sometimes referred to in the art as RNAi, or pre-transcriptional or pre-translational mechanisms. An iRNA agent can be a double stranded iRNA agent.

A "ds iRNA agent" (abbreviation for "double stranded iRNA agent"), as used herein, is an iRNA agent which includes more than one, and preferably two, strands in which interstrand hybridization can form a region of duplex structure. A "strand" herein refers to a contigouous sequence of nucleotides (including non-naturally occurring or modified nucleotides). The two or more strands may be, or each form a part of, separate molecules, or they may be covalently interconnected, *e.g.*, by a linker, *e.g.*, a polyethyleneglycol linker, to form one molecule. At least one strand can include a region which is sufficiently complementary to a target RNA. Such strand is termed the "antisense strand." A second strand of the dsRNA agent, which comprises a region complementary to the antisense strand, is termed the "sense strand." However, a ds iRNA agent can also be formed from a single RNA molecule which is at least partly self-complementary, forming, *e.g.*, a hairpin or panhandle structure, including a duplex region. In such case, the term "strand" refers to one of the regions of the RNA molecule that is complementary to another region of the same RNA molecule.

Although, in mammalian cells, long ds iRNA agents can induce the interferon response which is frequently deleterious, short ds iRNA agents do not trigger the interferon response, at least not to an extent that is deleterious to the cell and/or host (Manche et al., Mol. Cell. Biol. 12:5238, 1992; Lee et al., Virology 199:491, 1994; Castelli et al., J. Exp. Med. 186:967, 1997; Zheng et al., RNA 10:1934, 2004; Heidel et al., "lack of intension response in animals to naked siRNAs" Nature Biotechn. *advance online publication* doi:10.1038/nbt1038, Nov. 21, 2004). The iRNA agents described herein include molecules which are sufficiently short that they do not trigger a deleterious non-specific interferon response in normal mammalian cells. Thus, the administration of a composition including an iRNA agent (*e.g.*, formulated as described herein) to a subject can be used to decreased expression of the Nogo-L or Nogo-R genes in Nogo-L or Nogo-R expressing cells in the subject, while circumventing an interferon response. Molecules that are short enough that they do not trigger a deleterious interferon response are termed siRNA agents or siRNAs herein. "siRNA agent" or "siRNA" as used herein, refers to an iRNA agent, *e.g.*, a ds iRNA agent, that is sufficiently short that it does not induce a deleterious interferon response in a human cell, *e.g.*, it has a duplexed region of less than 60 but preferably less than 50, 40, or 30 nucleotide pairs.

The isolated iRNA agents described herein, including ds iRNA agents and siRNA agents, can mediate the decreased expression of a Nogo-L or Nogo-R nucleic acid, *e.g.*, by RNA degradation. For convenience, such RNA is also referred to herein as the RNA to be silenced. Such a nucleic acid is also referred to as a target gene. Preferably, the RNA to be silenced is a gene product of an endogenous Nogo-L or Nogo-R gene.

As used herein, the phrase "mediates RNAi" refers to the ability of an agent to silence, in a sequences specific manner, a target gene. "Silencing a target gene" means the process whereby a cell containing and/or expressing a certain product of the target gene when not in contact with the agent, will contain and/or express at least 10%, 15%,

20%, 25%, 30%, 35%, 40%, 45%, 50%, 55%, 60%, 65%, 70%, 75%, 80%, 85%, or 90% less of such gene product when contacted with the agent, as compared to a similar cell which has not been contacted with the agent. Such product of the target gene can, for example, be a messenger RNA (mRNA), a protein, or a regulatory element.

As used herein, the term "complementary" is used to indicate a sufficient degree of complementarity such that stable and specific binding occurs between a compound and described herein a target RNA molecule, *e.g.*, a Nogo-L or Nogo-R mRNA. Specific binding requires a sufficient degree of complementarity to avoid non-specific binding of the oligomeric compound to non-target sequences under conditions in which specific binding is desired, *i.e.*, under physiological conditions in the case of *in vivo* assays or therapeutic treatment, or in the case of *in vitro* assays, under conditions in which the assays are performed. The non-target sequences typically differ from the target sequences by at least 4 nucleotides.

As used herein, an iRNA agent is "sufficiently complementary" to a target RNA, *e.g.*, a target mRNA (*e.g.*, a target Nogo-L or Nogo-R mRNA) if the iRNA agent reduces the production of a protein encoded by the target RNA in a cell. The iRNA agent may also be "exactly complementary" to the target RNA, *e.g.*, the target RNA and the iRNA agent anneal, preferably to form a hybrid made exclusively of Watson-Crick basepairs in the region of exact complementarity. A "sufficiently complementary" iRNA agent can include an internal region (*e.g.*, of at least 10 nucleotides) that is exactly complementary to a target Nogo-L or Nogo-R RNA. Moreover, the iRNA agent may specifically discriminates a single-nucleotide difference. In this case, the iRNA agent only mediates RNAi if exact complementarity is found in the region (*e.g.*, within 7 nucleotides of) the single-nucleotide difference. Preferred iRNA agents will be based on or consist of or comprise the sense and antisense sequences provided in Table 1 or Table 2.

As used herein, "essentially identical" when used referring to a first nucleotide sequence in comparison to a second nucleotide sequence means that the first nucleotide sequence is identical to the second nucleotide sequence except for up to one, two or three nucleotide substitutions (*e.g.,* adenosine replaced by uracil). "Essentially retaining the ability to inhibit Nogo-L or Nogo-R expression in cultured human Nogo-L or Nogo-R expressing cells," as used herein referring to an iRNA agent not identical to but derived from one of the iRNA agents of Table 1 or Table 2 by deletion, addition or substitution of nucleotides, means that the derived iRNA agent possesses an inhibitory activity not more than 20% (in terms of remaining target mRNA) different from the inhibitory activity of the iRNA agent of Table 1 or Table 2 from which it was derived. For example, an iRNA agent derived from an iRNA agent of Table 1 which lowers the amount of Nogo-R mRNA present in cultured human Nogo-R expressing cells by 70% may itself lower the amount of Nogo-R mRNA present in cultured human Nogo-R expressing cells by at least 50% in order to be considered as essentially retaining the ability to inhibit Nogo-R expression in cultured human Nogo-R expressing cells. Likewise, an iRNA agent derived from an iRNA agent of Table 2 which lowers the amount of Nogo-L mRNA present in cultured human Nogo-L expressing cells by 70% may itself lower the amount of Nogo-L mRNA present in cultured human Nogo-L expressing cells by at least 50% in order to be considered as essentially retaining the ability to inhibit Nogp-L expression in cultured human Nogo-L expressing cells. Optionally, an iRNA agent described herein may lower the amount of Nogo-L or Nogo-R mRNA present in cultured human Nogo-L or Nogo-R expressing cells by at least 50%, or at least 40%.

As used herein, a "subject" refers to a mammalian organism undergoing treatment for a disorder mediated by Nogo-L or Nogo-R protein expression. The subject can be any mammal, such as a cow, horse, mouse, rat, dog, pig, goat, or a primate. The subject may be a human.

## Design and Selection of iRNA agents

As used herein, "disorders associated with Nogo-L or Nogo-R expression" refers to any biological or pathological state that (1) is mediated in part by the presence of Nogo-L or Nogo-R protein and (2) whose outcome can be affected by reducing the level of Nogo-L or Nogo-R protein present. Specific disorders associated with Nogo-L or Nogo-R expression are noted below and are primarily based on the responsibility of Nogo-R/L action in inhibiting axonal elongation and regeneration.

In general terms the present invention is based on the design, synthesis and generation of iRNA agents that target Nogo-R or Nogo-L and the demonstration of silencing of a Nogo-R or Nogo-L gene *in vitro* in cultured cells after incubation with an iRNA agent, and the resulting Nogo-R- or Nogo-L-specific effect. Not with standing, the invention is defined by the claims.

An iRNA agent can be rationally designed based on sequence information and desired characteristics. For example, an iRNA agent can be designed according to the relative melting temperature of the candidate duplex. Generally, the duplex should have a lower melting temperature at the 5' end of the antisense strand than at the 3' end of the antisense strand.

Candidate iRNA agents can also be designed by performing, for example, a gene walk analysis of the genes that will serve as the target gene. Overlapping, adjacent, or closely spaced candidate agents corresponding to all or some of the transcribed region can be generated and tested. Each of the iRNA agents can be tested and evaluated for the ability to down regulate the target gene expression (see below, "Evaluation of Candidate iRNA agents").

Herein, potential iRNA agents targeting Nogo-L or Nogo-R were designed using the known sequences of Nogo-L or Nogo-R for human, rat and mouse and other known Nogo sequences. The target sequences shown in Table 1 or Table 2 hereinabove were selected from those regions of the human Nogo-L or Nogo-R mRNA sequences that show complete homology with the corresponding sequences in rat and mouse. Therefore, the siRNA agents, agent numbers 6000 - 6476 and 6837 should show cross reactivity between these three species. Based on the results provided, the present invention provides iRNA agents that silence Nogo-L or Nogo-R in cultured human Nogo-L or Nogo-R expressing cells and in a subject.

Table 1 provides iRNA agents targeting Nogo-R.

Table 2 provides iRNA agents targeting Nogo-L.

Table 1: Exemplary iRNA agents for targeting Nogo-R mRNA

| | | | | | sense strand | | antisense strand | |
|---|---|---|---|---|---|---|---|---|
| **Agent number.** | **Start pos. in RNA[b]** | **SEQ ID NO.** | **target sequence (5'-3')** | **duplex design (over-hang)[a]** | **SEQ ID NO.** | **sequence (5'-3')** | **SEQ ID NO.** | **sequence (5'-3')** |
| 6000 | 296 | 1 | uaugcuacaaugagcccaaggug | double | 2 | ugcuacaaugagcccaaggTT | 3 | ccuugggcucauuguagcaTT |
| 6001 | 297 | 4 | augcuacaaugagcccaagguga | double | 5 | gcuacaaugagcccaagguTT | 6 | accuugggcucauuguagcTT |
| 6002 | 320 | 7 | cgacaagcugcccccagcagggc | double | 8 | acaagcugcccccagcaggTT | 9 | ccugcugggggcagcuuguTT |
| 6003 | 321 | 10 | gacaagcugcccccagcagggcc | double | 11 | caagcugcccccagcagggTT | 12 | cccugcugggggcagcuugTT |
| 6004 | 974 | 13 | acgacaaccccugggugugugac | double | 14 | gacaaccccugggugugugTT | 15 | cacacacccagggguugucTT |
| 6005 | 975 | 16 | cgacaaccccugggugugugacu | double | 17 | acaaccccugggugugugaTT | 18 | ucacacacccagggguuguTT |
| 6006 | 976 | 19 | gacaaccccugggugugugacug | double | 20 | caaccccugggugugugacTT | 21 | gucacacacccagggguugTT |
| 6007 | 976 | 22 | gacaaccccugggugugugacug | single | 23 | caaccccugggugugugacug | 24 | cagucacacacccagggguuguc |
| 6008 | 977 | 25 | acaaccccugggugugugacugc | double | 26 | aaccccugggugugugacuTT | 27 | a gucacacaccca gggguuTT |
| 6009 | 977 | 28 | acaaccccugggugugugacugc | single | 29 | aaccccugggugugugacugc | 30 | gcagucacacacccagggguugu |
| 6010 | 978 | 31 | caaccccugggugugugacugcc | double | 32 | accccugggugugugacugTT | 33 | cagucacacacccaggggguTT |
| 6011 | 979 | 34 | aaccccugggugugugacugccg | double | 35 | ccccugggugugugacugcTT | 36 | gcagucacacacccaggggTT |
| 6012 | 1010 | 37 | cacucugggccuggcugcagaag | double | 38 | cucugggccuggcugcagaTT | 39 | ucugcagccaggcccagag'1T |
| 6013 | 1011 | 40 | acucugggccuggcugcagaagu | double | 41 | ucugggccuggcugcagaaTT | 42 | uucugcagccaggcccagaTT |
| 6014 | 1012 | 43 | cucugggccuggcugcagaaguu | double | 44 | cugggccuggcugcagaagTT | 45 | cuucugcagccaggcccagTT |
| 6015 | 1012 | 46 | cucugggccuggcugcagaaguu | single | 47 | cugggccuggcugcagaaguu | 48 | aacuucugcagccaggcccagag |
| 6016 | 1013 | 49 | ucugggccuggcugcagaaguuc | double | 50 | ugggccuggcugcagaaguTT | 51 | acuucugcagccaggcccaTT |
| 6017 | 1013 | 52 | ucugggccuggcugcagaaguuc | single | 53 | ugggccuggcugcagaaguuc | 54 | gaacuucugcagccaggcccaga |
| 6018 | 1014 | 55 | cugggccuggcugcagaaguucc | double | 56 | gggccuggcugcagaaguuTT | 57 | aacuucugcagccaggcccTT |
| 6019 | 1014 | 58 | cugggccuggcugcagaaguucc | single | 59 | gggccuggcugcagaaguucc | 60 | ggaacuucugcagccaggcccag |
| 6020 | 1015 | 61 | ugggccuggcugcagaaguuccg | double | 62 | ggccuggcugcagaaguucTT | 63 | gaacuucugcagccaggccTT |
| 6021 | 1015 | 64 | ugggccuggcugcagaaguuccg | single | 65 | ggccuggcugcagaaguuccg | 66 | cggaacuucugcagccaggccca |
| 6022 | 1016 | 67 | gggccuggcugcagaaguuccgc | double | 68 | gccuggcugcagaaguuccTT | 69 | ggaacuucugcagccaggcTT |
| 6023 | 1017 | 70 | ggccuggcugcagaaguuccgcg | double | 71 | ccuggcugcagaaguuccgTT | 72 | cggaacuucugcagccaggTT |
| 6024 | 1478 | 73 | gcagccacugccgucugggccag | double | 74 | agccacugccgucugggccTT | 75 | ggcccagacggcaguggcuTT |
| 6025 | 1479 | 76 | cagccacugccgucugggccagg | double | 77 | gccacugccgucugggccaTT | 78 | uggcccagacggcaguggcTT |
| 6026 | 1480 | 79 | agccacugccgucugggccaggc | double | 80 | ccacugccgucugggccagTT | 81 | cuggcccagacggcaguggTT |
| 6027 | 1480 | 82 | agccacugccgucugggccaggc | single | 83 | ccacugccgucugggccaggc | 84 | gccuggcccagacggcaguggcu |
| 6028 | 1481 | 85 | gccacugccgucugggccaggca | double | 86 | cacugccgucugggccaggTT | 87 | ccuggcccagacggcagugTT |

(continued)

| Agent number. | Start pos. in RNA[b] | SEQ ID NO. | target sequence (5'-3') | duplex design (over-hang)[a] | sense strand SEQ ID NO. | sense strand sequence (5'-3') | antisense strand SEQ ID NO. | antisense strand sequence (5'-3') |
|---|---|---|---|---|---|---|---|---|
| 6029 | 1482 | 88 | ccacugccgucugggccaggcag | double | 89 | acugccgucugggccaggcTT | 90 | gccuggcccagacggcaguTT |

[a]Single: Single overhang design 21 mer sense, corresponding 23 mer antisense with 2 nucleotides overhang at 3' end; Double: double overhang design corresponding 19mer sense and antisense strand each with TT nucleotide overhang at 3' end

[b]"Start position" corresponds to the position within the human NOGO receptor mRNA: Genbank accession no. NM_023004, to which the 5'-most nucleotide of the sense strand corresponds for single overhang designs; for double overhang designs, the 5'-most ribonucleotide of the sense strand corresponds to (Start position +2)

Table 2: Exemplary iRNA agents for targeting Nogo-L mRNA

| Agent number. | Start pos. in RNA[b] | SEQ ID NO. | target sequence (5'-3') | duplex design (over-hang)[a] | sense strand SEQ ID NO. | sense strand sequence (5'-3') | antisense strand SEQ ID NO. | antisense strand sequence (5'-3') |
|---|---|---|---|---|---|---|---|---|
| 6030 | 363 | 91 | cguucaaguaccaguucgugagg | double | 92 | uucaaguaccaguucgugaTT | 93 | ucacgaacugguacuugaaTT |
| 6031 | 383 | 94 | agggagcccgaggacgaggagga | double | 95 | ggagcccgaggacgaggagTT | 96 | cuccucguccucgggcuccTT |
| 6032 | 384 | 97 | gggagcccgaggacgaggaggaa | double | 98 | gagcccgaggacgaggaggTT | 99 | ccuccucguccucgggcucTT |
| 6033 | 385 | 100 | ggagcccgaggacgaggaggaag | double | 101 | agcccgaggacgaggaggaTT | 102 | uccuccucguccucgggcuTT |
| 6034 | 451 | 103 | gcuggaggugcuggagaggaagc | double | 104 | uggaggugcuggagaggaaTT | 105 | uuccucuccagcaccuccaTT |
| 6035 | 452 | 106 | cuggaggugcuggagaggaagcc | double | 107 | ggaggugcuggagaggaagTT | 108 | cuuccucuccagcaccuccTT |
| 6036 | 453 | 109 | uggaggugcuggagaggaagccc | double | 110 | gaggugcuggagaggaagcTT | 111 | gcuuccucuccagcaccucTT |
| 6037 | 453 | 112 | uggaggugcuggagaggaagccc | single | 113 | gaggugcuggagaggaagccc | 114 | gggcuuccucuccagcaccucca |
| 6038 | 454 | 115 | ggaggugcuggagaggaagcccg | double | 116 | aggugcuggagaggaagccTT | 117 | ggcuuccucuccagcaccuTT |
| 6039 | 454 | 118 | ggaggugcuggagaggaagcccg | single | 119 | aggugcuggagaggaagcccg | 120 | cgggcuuccucuccagcaccucc |
| 6040 | 455 | 121 | gaggugcuggagaggaagcccgc | double | 122 | ggugcuggagaggaagcccTT | 123 | gggcuuccucuccagcaccTT |
| 6041 | 455 | 124 | gaggugcuggagaggaagcccgc | single | 125 | ggugcuggagaggaagcccgc | 126 | gcgggcuuccucuccagcaccuc |
| 6042 | 456 | 127 | aggugcuggagaggaagcccgcc | double | 128 | gugcuggagaggaagcccgTT | 129 | cgggcuuccucuccagcacTT |
| 6043 | 457 | 130 | ggugcuggagaggaagcccgccg | double | 131 | ugcuggagaggaagcccgcTT | 132 | gcgggcuuccucuccagcaTT |
| 6044 | 813 | 133 | ccccggccgcgcccaagcgcagg | double | 134 | ccggccgcgcccaagcgcaTT | 135 | ugcgcuugggcgcggccggTT |
| 6045 | 814 | 136 | cccggccgcgcccaagcgcaggg | double | 137 | cggccgcgcccaagcgcagTT | 138 | cugcgcuugggcgcggccgTT |
| 6046 | 815 | 139 | ccggccgcgcccaagcgcagggg | double | 140 | ggccgcgcccaagcgcaggTT | 141 | ccugcgcuugggcgcggccTT |
| 6047 | 815 | 142 | ccggccgcgcccaagcgcagggg | single | 143 | ggccgcgcccaagcgcagggg | 144 | ccccugcgcuugggcgcggccgg |
| 6048 | 816 | 145 | cggccgcgcccaagcgcaggggc | double | 146 | gccgcgcccaagcgcagggTT | 147 | cccugcgcuugggcgcggcTT |
| 6049 | 816 | 148 | cggccgcgcccaagcgcaggggc | single | 149 | gccgcgcccaagcgcaggggc | 150 | gccccugcgcuugggcgcggccg |
| 6050 | 817 | 151 | ggccgcgcccaagcgcaggggcu | double | 152 | ccgcgcccaagcgcaggggTT | 153 | ccccugcgcuugggcgcggTT |
| 6051 | 817 | 154 | ggccgcgcccaagcgcaggggcu | single | 155 | ccgcgcccaagcgcaggggcu | 156 | agccccugcgcuugggcgcggcc |
| 6052 | 818 | 157 | gccgcgcccaagcgcaggggcuc | double | 158 | cgcgcccaagcgcaggggcTT | 159 | gccccugcgcuugggcgcgTT |
| 6053 | 818 | 160 | gccgcgcccaagcgcaggggcuc | single | 161 | cgcgcccaagcgcaggggcuc | 162 | gagccccugcgcuugggcgcggc |
| 6054 | 819 | 163 | ccgcgcccaagcgcaggggcucc | double | 164 | gcgcccaagcgcaggggcuTT | 165 | agccccugcgcuugggcgcTT |
| 6055 | 820 | 166 | cgcgcccaagcgcaggggcuccu | double | 167 | cgcccaagcgcaggggcucTT | 168 | gagccccugcgcuugggcgTT |
| 6056 | 844 | 169 | gggcucaguggaugagacccuuu | double | 170 | gcucaguggaugagacccuTT | 171 | agggucucauccacugagcTT |
| 6057 | 845 | 172 | ggcucaguggaugagacccuuuu | double | 173 | cucaguggaugagacccuuTT | 174 | aagggucucauccacugagTT |
| 6058 | 846 | 175 | gcucaguggaugagacccuuuuu | double | 176 | ucaguggaugagacccuuuTT | 177 | aaagggucucauccacugaTT |

(continued)

| Agent number. | Start pos. in RNA[b] | SEQ ID NO. | target sequence (5'-3') | duplex design (overhang)[a] | sense strand SEQ ID NO. | sense strand sequence (5'-3') | antisense strand SEQ ID NO. | antisense strand sequence (5'-3') |
|---|---|---|---|---|---|---|---|---|
| 6059 | 846 | 178 | gcucaguggaugagacccuuuuu | single | 179 | ucaguggaugagacccuuuuu | 180 | aaaaagggucucauccacugagc |
| 6060 | 847 | 181 | cucaguggaugagacccuuuuug | double | 182 | caguggaugagacccuuuuTT | 183 | aaaagggucucauccacugTT |
| 6061 | 847 | 184 | cucaguggaugagacccuuuuug | single | 185 | caguggaugagacccuuuuug | 186 | caaaaagggucucauccacugag |
| 6062 | 848 | 187 | ucaguggaugagacccuuuuugc | double | 188 | aguggaugagacccuuuuuTT | 189 | aaaaagggucucauccacuTT |
| 6063 | 848 | 190 | ucaguggaugagacccuuuuugc | single | 191 | aguggaugagacccuuuuugc | 192 | gcaaaaagggucucauccacuga |
| 6064 | 849 | 193 | caguggaugagacccuuuuugcu | double | 194 | guggaugagacccuuuuugTT | 195 | caaaaagggucucauccacTT |
| 6065 | 849 | 196 | caguggaugagacccuuuuugcu | single | 197 | guggaugagacccuuuuugcu | 198 | agcaaaaagggucucauccacug |
| 6066 | 850 | 199 | aguggaugagacccuuuuugcuc | double | 200 | uggaugagacccuuuuugcTT | 201 | gcaaaaagggucucauccaTT |
| 6067 | 850 | 202 | aguggaugagacccuuuuugcuc | single | 203 | uggaugagacccuuuuugcuc | 204 | gagcaaaaagggucucauccacu |
| 6068 | 851 | 205 | guggaugagacccuuuuugcucu | double | 206 | ggaugagacccuuuuugcuTT | 207 | agcaaaaagggucucauccTT |
| 6069 | 851 | 208 | guggaugagacccuuuuugcucu | single | 209 | ggaugagacccuuuuugcucu | 210 | agagcaaaaagggucucauccac |
| 6070 | 852 | 211 | uggaugagacccuuuuugcucuu | double | 212 | gaugagacccuuuuugcucTT | 213 | gagcaaaaagggucucaucTT |
| 6071 | 852 | 214 | uggaugagacccuuuuugcucuu | single | 215 | gaugagacccuuuuugcucuu | 216 | aagagcaaaaagggucucaucca |
| 6072 | 853 | 217 | ggaugagacccuuuuugcucuuc | double | 218 | augagacccuuuuugcucuTT | 219 | agagcaaaaagggucucauTT |
| 6073 | 853 | 220 | ggaugagacccuuuuugcucuuc | single | 221 | augagacccuuuuugcucuuc | 222 | gaagagcaaaaagggucucaucc |
| 6074 | 854 | 223 | gaugagacccuuuuugcucuucc | double | 224 | ugagacccuuuuugcucuuTT | 225 | aagagcaaaaagggucucaTT |
| 6075 | 854 | 226 | gaugagacccuuuuugcucuucc | single | 227 | ugagacccuuuuugcucuucc | 228 | ggaagagcaaaaagggucucauc |
| 6076 | 855 | 229 | augagacccuuuuugcucuuccu | double | 230 | gagacccuuuuugcucuucTT | 231 | gaagagcaaaaagggucucTT |
| 6077 | 855 | 232 | augagacccuuuuugcucuuccu | single | 233 | gagacccuuuuugcucuuccu | 234 | aggaagagcaaaaagggucucau |
| 6078 | 856 | 235 | ugagacccuuuuugcucuuccug | double | 236 | agacccuuuuugcucuuccTT | 237 | ggaagagcaaaaagggucuTT |
| 6079 | 856 | 238 | ugagacccuuuuugcucuuccug | single | 239 | agacccuuuuugcucuuccug | 240 | caggaagagcaaaaagggucuca |
| 6080 | 857 | 241 | gagacccuuuuugcucuuccugc | double | 242 | gacccuuuuugcucuuccuTT | 243 | aggaagagcaaaaagggucTT |
| 6081 | 857 | 244 | gagacccuuuuugcucuuccugc | single | 245 | gacccuuuuugcucuuccugc | 246 | gcaggaagagcaaaaagggucuc |
| 6082 | 858 | 247 | agacccuuuuugcucuuccugcu | double | 248 | acccuuuuugcucuuccugTT | 249 | caggaagagcaaaaaggguTT |
| 6083 | 858 | 250 | agacccuuuuugcucuuccugcu | single | 251 | acccuuuuugcucuuccugcu | 252 | agcaggaagagcaaaaagggucu |
| 6084 | 859 | 253 | gacccuuuuugcucuuccugcug | double | 254 | cccuuuuugcucuuccugcTT | 255 | gcaggaagagcaaaaagggTT |
| 6085 | 859 | 256 | gacccuuuuugcucuuccugcug | single | 257 | cccuuuuugcucuuccugcug | 258 | cagcaggaagagcaaaaaggguc |
| 6086 | 860 | 259 | acccuuuuugcucuuccugcugc | double | 260 | ccuuuuugcucuuccugcuTT | 261 | agcaggaagagcaaaaaggTT |
| 6087 | 860 | 262 | acccuuuuugcucuuccugcugc | single | 263 | ccuuuuugcucuuccugcugc | 264 | gcagcaggaagagcaaaaagggu |
| 6088 | 861 | 265 | cccuuuuugcucuuccugcugca | double | 266 | cuuuuugcucuuccugcugTT | 267 | cagcaggaagagcaaaaagTT |

(continued)

| Agent number. | Start pos. in RNA[b] | SEQ ID NO. | target sequence (5'-3') | duplex design (over-hang)[a] | sense strand SEQ ID NO. | sense strand sequence (5'-3') | antisense strand SEQ ID NO. | antisense strand sequence (5'-3') |
|---|---|---|---|---|---|---|---|---|
| 6089 | 861 | 268 | cccuuuuugcucuuccugcugca | single | 269 | cuuuuugcucuuccugcugca | 270 | ugcagcaggaagagcaaaaaggg |
| 6090 | 862 | 271 | ccuuuuugcucuuccugcugcau | double | 272 | uuuuugcucuuccugcugcTT | 273 | gcagcaggaagagcaaaaaTT |
| 6091 | 862 | 274 | ccuuuuugcucuuccugcugcau | single | 275 | uuuuugcucuuccugcugcau | 276 | augcagcaggaagagcaaaaagg |
| 6092 | 863 | 277 | cuuuuugcucuuccugcugcauc | double | 278 | uuuugcucuuccugcugcaTT | 279 | ugcagcaggaagagcaaaaTT |
| 6093 | 663 | 280 | cuuuuugcucuuccugcugcauc | single | 281 | uuuugcucuuccugcugcauc | 282 | gaugcagcaggaagagcaaaaag |
| 6094 | 864 | 283 | uuuuugcucuuccugcugcaucu | double | 284 | uuugcucuuccugcugcauTT | 285 | augcagcaggaagagcaaaTT |
| 6095 | 864 | 286 | uuuuugcucuuccugcugcaucu | single | 287 | uuugcucuuccugcugcaucu | 288 | agaugcagcaggaagagcaaaaa |
| 6096 | 865 | 289 | uuuugcucuuccugcugcaucug | double | 290 | uugcucuuccugcugcaucTT | 291 | gaugcagcaggaagagcaaTT |
| 6097 | 865 | 292 | uuuugcucuuccugcugcaucug | single | 293 | uugcucuuccugcugcaucug | 294 | cagaugcagcaggaagagcaaaa |
| 6098 | 866 | 295 | uuugcucuuccugcugcaucuga | double | 296 | ugcucuuccugcugcaucuTT | 297 | agaugcagcaggaagagcaTT |
| 6099 | 866 | 298 | uuugcucuuccugcugcaucuga | single | 299 | ugcucuuccugcugcaucuga | 300 | ucagaugaagcaggaagagcaaa |
| 6100 | 867 | 301 | uugcucuuccugcugcaucugag | double | 302 | gcucuuccugcugcaucugTT | 303 | cagaugcagcaggaagagcTT |
| 6101 | 867 | 304 | uugcucuuccugcugcaucugag | single | 305 | gcucuuccugcugcaucugag | 306 | cucagaugcagcaggaagagcaa |
| 6102 | 868 | 307 | ugcucuuccugcugcaucugagc | double | 308 | cucuuccugcugcaucugaTT | 309 | ucagaugcagcaggaagagTT |
| 6103 | 868 | 310 | ugcucuuccugcugcaucugagc | single | 311 | cucuuccugcugcaucugagc | 312 | gcucagaugcagcaggaagagca |
| 6104 | 869 | 313 | gcucuuccugcugcaucugagcc | double | 314 | ucuuccugcugcaucugagTT | 315 | cucagaugcagcaggaagaTT |
| 6105 | 869 | 316 | gcucuuccugcugcaucugagcc | single | 317 | ucuuccugcugcaucugagcc | 318 | ggcucagaugcagcaggaagagc |
| 6106 | 870 | 319 | cucuuccugcugcaucugagccu | double | 320 | cuuccugcugcaucugagcTT | 321 | gcucagaugcagcaggaagTT |
| 6107 | 870 | 322 | cucuuccugcugcaucugagccu | single | 323 | cuuccugcugcaucugagccu | 324 | aggcucagaugcagcaggaagag |
| 6108 | 871 | 325 | ucuuccugcugcaucugagccug | double | 326 | uuccugcugcaucugagccTT | 327 | ggcucagaugcagcaggaaTT |
| 6109 | 871 | 328 | ucuuccugcugcaucugagccug | single | 329 | uuccugcugcaucugagccug | 330 | caggcucagaugcagcaggaaga |
| 6110 | 872 | 331 | cuuccugcugcaucugagccugu | double | 332 | uccugcugcaucugagccuTT | 333 | aggcucagaugcagcaggaTT |
| 6111 | 872 | 334 | cuuccugcugcaucugagccugu | single | 335 | uccugcugcaucugagccugu | 336 | acaggcucagaugcagcaggaag |
| 6112 | 873 | 337 | uuccugcugcaucugagccugug | double | 338 | ccugcugcaucugagccugTT | 339 | caggcucagaugcagcagTT |
| 6113 | 873 | 340 | uuccugcugcaucugagccugug | single | 341 | ccugcugcaucugagccugug | 342 | cacaggcucagaugcagcaggaa |
| 6114 | 874 | 343 | uccugcugcaucugagccuguga | double | 344 | cugcugcaucugagccuguTT | 345 | acaggcucagaugcagcagTT |
| 6115 | 874 | 346 | uccugcugcaucugagccuguga | single | 347 | cugcugcaucugagccuguga | 348 | ucacaggcucagaugcagcagga |
| 6116 | 875 | 349 | ccugcugcaucugagccugugau | double | 350 | ugcugcaucugagccugugTT | 351 | cacaggcucagaugcagcaTT |
| 6117 | 875 | 352 | ccugcugcaucugagccugugau | single | 353 | ugcugcaucugagccugugau | 354 | aucacaggcucagaugcagcagg |
| 6118 | 876 | 355 | cugcugcaucugagccugugaua | double | 356 | gcugcaucugagccugugaTT | 357 | ucacaggcucagaugcagcTT |

(continued)

| Agent number. | Start pos. in RNA[b] | SEQ ID NO. | target sequence (5'-3') | duplex design (overhang)[a] | sense strand SEQ ID NO. | sense strand sequence (5'-3') | antisense strand SEQ ID NO. | antisense strand sequence (5'-3') |
|---|---|---|---|---|---|---|---|---|
| 6119 | 876 | 358 | cugcugcaucugagccugugaua | single | 359 | gcugcaucugagccugugaua | 360 | uaucacaggcucagaugcagcag |
| 6120 | 877 | 361 | ugcugcaucugagccugugauac | double | 362 | cugcaucugagccugugauTT | 363 | aucacaggcucagaugcagTT |
| 6121 | 877 | 364 | ugcugcaucugagccugugauac | single | 365 | cugcaucugagccugugauac | 366 | guaucacaggcucagaugcagca |
| 6122 | 878 | 367 | gcugcaucugagccugugauacg | double | 368 | ugcaucugagccugugauaTT | 369 | uaucacaggcucagaugcaTT |
| 6123 | 879 | 370 | cugcaucugagccugugauacgc | double | 371 | gcaucugagccugugauacTT | 372 | guaucacaggcucagaugcTT |
| 6124 | 926 | 373 | aaggagcagccagguaacacuau | double | 374 | ggagcagccagguaacacuTT | 375 | aguguuaccuggcugcuccTT |
| 6125 | 952 | 376 | ggcuggucaagaggauuucccau | double | 377 | cuggucaagaggauuucccTT | 378 | gggaaauccucuugaccagTT |
| 6126 | 953 | 379 | gcuggucaagaggauuucccauc | double | 380 | uggucaagaggauuucccaTT | 381 | ugggaaauccucuugaccaTT |
| 6127 | 954 | 382 | cuggucaagaggauuucccaucu | double | 383 | ggucaagaggauuucccauTT | 384 | augggaaauccucuugaccTT |
| 6128 | 954 | 385 | cuggucaagaggauuucccaucu | single | 386 | ggucaagaggauuucccaucu | 387 | agaugggaaauccucuugaccag |
| 6129 | 955 | 388 | uggucaagaggauuucccaucug | double | 389 | gucaagaggauuucccaucTT | 390 | gaugggaaauccucuugacTT |
| 6130 | 955 | 391 | uggucaagaggauuucccaucug | single | 392 | gucaagaggauuucccaucug | 393 | cagaugggaaauccucuugacca |
| 6131 | 956 | 394 | ggucaagaggauuucccaucugu | double | 395 | ucaagaggauuucccaucuTT | 396 | agaugggaaauccucuugaTT |
| 6132 | 956 | 397 | ggucaagaggauuucccaucugu | single | 398 | ucaagaggauuucccaucugu | 399 | acagaugggaaauccucuugacc |
| 6133 | 957 | 400 | gucaagaggauuucccaucuguc | double | 401 | caagaggauuucccaucugTT | 402 | cagaugggaaauccucuugTT |
| 6134 | 957 | 403 | gucaagaggauuucccaucuguc | single | 404 | caagaggauuucccaucuguc | 405 | gacagaugggaaauccucuugac |
| 6135 | 958 | 406 | ucaagaggauuucccaucugucc | double | 407 | aagaggauuucccaucuguTT | 408 | acagaugggaaauccucuuTT |
| 6136 | 958 | 409 | ucaagaggauuucccaucugucc | single | 410 | aagaggauuucccaucugucc | 411 | ggacagaugggaaauccucuuga |
| 6137 | 959 | 412 | caagaggauuucccaucuguccu | double | 413 | agaggauuucccaucugucTT | 414 | gacagaugggaaauccucuTT |
| 6138 | 959 | 415 | caagaggauuucccaucuguccu | single | 416 | agaggauuucccaucuguccu | 417 | aggacagaugggaaauccucuug |
| 6139 | 960 | 418 | aagaggauuucccaucuguccug | double | 419 | gaggauuucccaucuguccTT | 420 | ggacagaugggaaauccucTT |
| 6140 | 960 | 421 | aagaggauuucccaucuguccug | single | 422 | gaggauuucccaucuguccug | 423 | caggacagaugggaaauccucuu |
| 6141 | 961 | 424 | agaggauuucccaucuguccugc | double | 425 | aggauuucccaucuguccuTT | 426 | aggacagaugggaaauccuTT |
| 6142 | 962 | 427 | gaggauuucccaucuguccugcu | double | 428 | ggauuucccaucuguccugTT | 429 | caggacagaugggaaauccTT |
| 6143 | 1773 | 430 | ccgaugaaaaaaaaauagaagaa | double | 431 | gaugaaaaaaaaauagaagTT | 432 | cuucuauuuuuuuuucaucTT |
| 6144 | 1774 | 433 | cgaugaaaaaaaaauagaagaaa | double | 434 | augaaaaaaaaauagaagaTT | 435 | ucuucuauuuuuuuuucauTT |
| 6145 | 1775 | 436 | gaugaaaaaaaaauagaagaaaa | double | 437 | ugaaaaaaaaauagaagaaTT | 438 | uucuucuauuuuuuuuucaTT |
| 6146 | 1776 | 439 | augaaaaaaaaauagaagaaaag | double | 440 | gaaaaaaaaauagaagaaaTT | 441 | uuucuucuauuuuuuuuucTT |
| 6147 | 1962 | 442 | uacaggaagcaugugaaagugaa | double | 443 | caggaagcaugugaaagugTT | 444 | cacuuucacaugcuuccugTT |
| 6148 | 1963 | 445 | acaggaagcaugugaaagugaau | double | 446 | aggaagcaugugaaagugaTT | 447 | ucacuuucacaugcuuccuTT |

(continued)

| Agent number. | Start pos. in RNA[b] | SEQ ID NO. | target sequence (5'-3') | duplex design (over-hang)[a] | sense strand SEQ ID NO. | sense strand sequence (5'-3') | antisense strand SEQ ID NO. | antisense strand sequence (5'-3') |
|---|---|---|---|---|---|---|---|---|
| 6149 | 1964 | 448 | caggaagcaugugaaagugaauu | double | 449 | ggaagcaugugaaagugaaTT | 450 | uucacuuucacaugcuuccTT |
| 6150 | 1998 | 451 | cugguacaaagauugcuuaugaa | double | 452 | gguacaaagauugcuuaugTT | 453 | cauaagcaaucuuuguaccTT |
| 6151 | 1999 | 454 | ugguacaaagauugcuuaugaaa | double | 455 | guacaaagauugcuuaugaTT | 456 | ucauaagcaaucuuuguacTT |
| 6152 | 2000 | 457 | gguacaaagauugcuuaugaaac | double | 458 | uacaaagauugcuuaugaaTT | 459 | uucauaagcaaucuuuguaTT |
| 6153 | 2000 | 460 | gguacaaagauugcuuaugaaac | single | 461 | uacaaagauugcuuaugaaac | 462 | guuucauaagcaaucuuuguacc |
| 6154 | 2001 | 463 | guacaaagauugcuuaugaaaca | double | 464 | acaaagauugcuuaugaaaTT | 465 | uuucauaagcaaucuuuguTT |
| 6155 | 2001 | 466 | guacaaagauugcuuaugaaaca | single | 467 | acaaagauugcuuaugaaaca | 468 | uguuucauaagcaaucuuuguac |
| 6156 | 2002 | 469 | uacaaagauugcuuaugaaacaa | double | 470 | caaagauugcuuaugaaacTT | 471 | guuucauaagcaaucuuugTT |
| 6157 | 2002 | 472 | uacaaagauugcuuaugaaacaa | single | 473 | caaagauugcuuaugaaacaa | 474 | uuguuucauaagcaaucuuugua |
| 6158 | 2003 | 475 | acaaagauugcuuaugaaacaaa | double | 476 | aaagauugcuuaugaaacaTT | 477 | uguuucauaagcaaucuuuTT |
| 6159 | 2003 | 478 | acaaagauugcuuaugaaacaaa | single | 479 | aaagauugcuuaugaaacaaa | 480 | uuuguuucauaagcaaucuuugu |
| 6160 | 2004 | 481 | caaagauugcuuaugaaacaaaa | double | 482 | aagauugcuuaugaaacaaTT | 483 | uuguuucauaagcaaucuuTT |
| 6161 | 2004 | 484 | caaagauugcuuaugaaacaaaa | single | 485 | aagauugcuuaugaaacaaaa | 486 | uuuuguuucauaagcaaucuuug |
| 6162 | 2005 | 487 | aaagauugcuuaugaaacaaaaa | double | 488 | agauugcuuaugaaacaaaTT | 489 | uuuguuucauaagcaaucuTT |
| 6163 | 2006 | 490 | aagauugcuuaugaaacaaaaau | double | 491 | gauugcuuaugaaacaaaaTT | 492 | uuuuguuucauaagcaaucTT |
| 6164 | 2074 | 493 | ugcagcacagcuuugcccaucau | double | 494 | cagcacagcuuugcccaucTT | 495 | gaugggcaaagcugugcugTT |
| 6165 | 2075 | 496 | gcagcacagcuuugcccaucauu | double | 497 | agcacagcuuugcccaucaTT | 498 | ugaugggcaaagcugugcuTT |
| 6166 | 2076 | 499 | cagcacagcuuugcccaucauuu | double | 500 | gcacagcuuugcccaucauTT | 501 | augaugggcaaagcugugcTT |
| 6167 | 2076 | 502 | cagcacagcuuugcccaucauuu | single | 503 | gcacagcuuugcccaucauuu | 504 | aaaugaugggcaaagcugugcug |
| 6168 | 2077 | 505 | agcacagcuuugcccaucauuug | double | 506 | cacagcuuugcccaucauuTT | 507 | aaugaugggcaaagcugugTT |
| 6169 | 2077 | 508 | agcacagcuuugcccaucauuug | single | 509 | cacagcuuugcccaucauuug | 510 | caaaugaugggcaaagcugugcu |
| 6170 | 2078 | 511 | gcacagcuuugcccaucauuuga | double | 512 | acagcuuugcccaucauuuTT | 513 | aaaugaugggcaaagcuguTT |
| 6171 | 2078 | 514 | gcacagcuuugcccaucauuuga | single | 515 | acagcuuugcccaucauuuga | 516 | ucaaaugaugggcaaagcugugc |
| 6172 | 2079 | 517 | cacagcuuugcccaucauuugaa | double | 518 | cagcuuugcccaucauuugTT | 519 | caaaugaugggcaaagcugTT |
| 6173 | 2080 | 520 | acagcuuugcccaucauuugaag | double | 521 | agcuuugcccaucauuugaTT | 522 | ucaaaugaugggcaaagcuTT |
| 6174 | 2365 | 523 | aacagaagcuccuuauauaucua | double | 524 | cagaagcuccuuauauaucTT | 525 | gauauauaaggagcuucugTT |
| 6175 | 2391 | 526 | caugugauuuaauuaaagaaaca | double | 527 | ugugauuuaauuaaagaaaTT | 528 | uuucuuuaauuaaaucacaTT |
| 6176 | 2392 | 529 | augugauuuaauuaaagaaacaa | double | 530 | gugauuuaauuaaagaaacTT | 531 | guuucuuuaauuaaaucacTT |
| 6177 | 2393 | 532 | ugugauuuaauuaaagaaacaaa | double | 533 | ugauuuaauuaaagaaacaTT | 534 | uguuucuuuaauuaaaucaTT |
| 6178 | 2393 | 535 | ugugauuuaauuaaagaaacaaa | single | 536 | ugauuuaauuaaagaaacaaa | 537 | uuguuucuuuaauuaaaucaca |

(continued)

| Agent number. | Start pos. in RNA[b] | SEQ ID NO. | target sequence (5'-3') | duplex design (over-hang)[a] | sense strand SEQ ID NO. | sense strand sequence (5'-3') | antisense strand SEQ ID NO. | antisense strand sequence (5'-3') |
|---|---|---|---|---|---|---|---|---|
| 6179 | 2394 | 538 | gugauuuaauuaaagaaacaaag | double | 539 | gauuuaauuaaagaaacaaTT | 540 | uuguuucuuuaauuaaaucTT |
| 6180 | 2394 | 541 | gugauuuaauuaaagaaacaaag | single | 542 | gauuuaauuaaagaaacaaag | 543 | cuuuguuucuuuaauuaaaucac |
| 6181 | 2395 | 544 | ugauuuaauuaaagaaacaaagc | double | 545 | auuuaauuaaagaaacaaaTT | 546 | uuuguuucuuuaauuaaauTT |
| 6182 | 2395 | 547 | ugauuuaauuaaagaaacaaagc | single | 548 | auuuaauuaaagaaacaaagc | 549 | gcuuuguuucuuuaauuaaauca |
| 6183 | 2396 | 550 | gauuuaauuaaagaaacaaagcu | double | 551 | uuuaauuaaagaaacaaagTT | 552 | cuuuguuucuuuaauuaaaTT |
| 6184 | 2396 | 553 | gauuuaauuaaagaaacaaagcu | single | 554 | uuuaauuaaagaaacaaagcu | 555 | agcuuuguuucuuuaauuaaauc |
| 6185 | 2397 | 556 | auuuaauuaaagaaacaaagcuu | double | 557 | uuaauuaaagaaacaaagcTT | 558 | gcuuuguuucuuuaauuaaTT |
| 6186 | 2398 | 559 | uuuaauuaaagaaacaaagcuuu | double | 560 | uaauuaaagaaacaaagcuTT | 561 | agcuuuguuucuuuaauuaTT |
| 6187 | 2520 | 562 | auucugaaccaguugacuuauuu | double | 563 | ucugaaccaguugacuuauTT | 564 | auaagucaacugguucagaTT |
| 6188 | 2521 | 565 | uucugaaccaguugacuuauuua | double | 566 | cugaaccaguugacuuauuTT | 567 | aauaagucaacugguucagTT |
| 6189 | 2522 | 568 | ucugaaccaguugacuuauuuag | double | 569 | ugaaccaguugacuuauuuTT | 570 | aaauaagucaacugguucaTT |
| 6190 | 2522 | 571 | ucugaaccaguugacuuauuuag | single | 572 | ugaaccaguugacuuauuuag | 573 | cuaaauaagucaacugguucaga |
| 6191 | 2523 | 574 | cugaaccaguugacuuauuuagu | double | 575 | gaaccaguugacuuauuuaTT | 576 | uaaauaagucaacugguucTT |
| 6192 | 2523 | 577 | cugaaccaguugacuuauuuagu | single | 578 | gaaccaguugacuuauuuagu | 579 | acuaaauaagucaacugguucag |
| 6193 | 2524 | 580 | ugaaccaguugacuuauuuagug | double | 581 | aaccaguugacuuauuuagTT | 582 | cuaaauaagucaacugguuTT |
| 6194 | 2524 | 583 | ugaaccaguugacuuauuuagug | single | 584 | aaccaguugacuuauuuagug | 585 | cacuaaauaagucaacugguuca |
| 6195 | 2525 | 586 | gaaccaguugacuuauuuaguga | double | 587 | accaguugacuuauuuaguTT | 588 | acuaaauaagucaacugguTT |
| 6196 | 2525 | 589 | gaaccaguugacuuauuuaguga | single | 590 | accaguugacuuauuuaguga | 591 | ucacuaaauaagucaacugguuc |
| 6197 | 2526 | 592 | aaccaguugacuuauuuagugau | double | 593 | ccaguugacuuauuuagugTT | 594 | cacuaaauaagucaacuggTT |
| 6198 | 2526 | 595 | aaccaguugacuuauuuagugau | single | 596 | ccaguugacuuauuuagugau | 597 | aucacuaaauaagucaacugguu |
| 6199 | 2527 | 598 | accaguugacuuauuuagugaug | double | 599 | caguugacuuauuuagugaTT | 600 | ucacuaaauaagucaacugTT |
| 6200 | 2527 | 601 | accaguugacuuauuuagugaug | single | 602 | caguugacuuauuuagugaug | 603 | caucacuaaauaagucaacuggu |
| 6201 | 2528 | 604 | ccaguugacuuauuuagugauga | double | 605 | aguugacuuauuuagugauTT | 606 | aucacuaaauaagucaacuTT |
| 6202 | 2528 | 607 | ccaguugacuuauuuagugauga | single | 608 | aguugacuuauuuagugauga | 609 | ucaucacuaaauaagucaacugg |
| 6203 | 2529 | 610 | caguugacuuauuuagugaugau | double | 611 | guugacuuauuuagugaugTT | 612 | caucacuaaauaagucaacTT |
| 6204 | 2529 | 613 | caguugacuuauuuagugaugau | single | 614 | guugacuuauuuagugaugau | 615 | aucaucacuaaauaagucaacug |
| 6205 | 2530 | 616 | aguugacuuauuuagugaugauu | double | 617 | uugacuuauuuagugaugaTT | 618 | ucaucacuaaauaagucaaTT |
| 6206 | 2530 | 619 | aguugacuuauuuagugaugauu | single | 620 | uugacuuauuuagugaugauu | 621 | aaucaucacuaaauaagucaacu |
| 6207 | 2531 | 622 | guugacuuauuuagugaugauuc | double | 623 | ugacuuauuuagugaugauTT | 624 | aucaucacuaaauaagucaTT |
| 6208 | 2531 | 625 | guugacuuauuuagugaugauuc | single | 626 | ugacuuauuuagugaugauuc | 627 | gaaucaucacuaaauaagucaac |

(continued)

| Agent number. | Start pos. in RNA[b] | SEQ ID NO. | target sequence (5'-3') | duplex design (overhang)[a] | sense strand SEQ ID NO. | sense strand sequence (5'-3') | antisense strand SEQ ID NO. | antisense strand sequence (5'-3') |
|---|---|---|---|---|---|---|---|---|
| 6209 | 2532 | 628 | uugacuuauuuagugaugauuca | double | 629 | gacuuauuuagugaugauuTT | 630 | aaucaucacuaaauaagucTT |
| 6210 | 2533 | 631 | ugacuuauuuagugaugauucaa | double | 632 | acuuauuuagugaugauucTT | 633 | gaaucaucacuaaauaaguTT |
| 6211 | 2982 | 634 | cccacaaaagugaaauugcuaau | double | 635 | cacaaaagugaaauugcuaTT | 636 | uagcaauuucacuuuugugTT |
| 6212 | 2983 | 637 | ccacaaaagugaaauugcuaaug | double | 638 | acaaaagugaaauugcuaaTT | 639 | uuagcaauuucacuuuuguTT |
| 6213 | 2984 | 640 | cacaaaagugaaauugcuaaugc | double | 641 | caaaagugaaauugcuaauTT | 642 | auuagcaauuucacuuuugTT |
| 6214 | 3299 | 643 | aguaaaacuucaguuguugaccu | double | 644 | uaaaacuucaguuguugacTT | 645 | gucaacaacugaaguuuuaTT |
| 6215 | 3300 | 646 | guaaaacuucaguuguugaccuc | double | 647 | aaaacuucaguuguugaccTT | 648 | ggucaacaacugaaguuuuTT |
| 6216 | 3301 | 649 | uaaaacuucaguuguugaccucc | doubly | 650 | aaacuucaguuguugaccuTT | 651 | aggucaacaacugaaguuuTT |
| 6217 | 3301 | 652 | uaaaacuucaguuguugaccucc | single | 653 | aaacuucaguuguugaccucc | 654 | ggaggucaacaacugaaguuuua |
| 6218 | 3302 | 655 | aaaacuucaguuguugaccuccu | double | 656 | aacuucaguuguugaccucTT | 657 | gaggucaacaacugaaguuTT |
| 6219 | 3302 | 658 | aaaacuucaguuguugaccuccu | single | 659 | aacuucaguuguugaccuccu | 660 | aggaggucaacaacugaaguuuu |
| 6220 | 3303 | 661 | aaacuucaguuguugaccuccug | double | 662 | acuucaguuguugaccuccTT | 663 | ggaggucaacaacugaaguTT |
| 6221 | 3304 | 664 | aacuucaguuguugaccuccugu | double | 665 | cuucaguuguugaccuccuTT | 666 | aggaggucaacaacugaagTT |
| 6222 | 3324 | 667 | uguacuggagagacauuaagaag | double | 668 | uacuggagagacauuaagaTT | 669 | ucuuaaugucucuccaguaTT |
| 6223 | 3325 | 670 | guacuggagagacauuaagaaga | double | 671 | acuggagagacauuaagaaTT | 672 | uucuuaaugucucuccaguTT |
| 6224 | 3326 | 673 | uacuggagagacauuaagaagac | double | 674 | cuggagagacauuaagaagTT | 675 | cuucuuaaugucucuccagTT |
| 6225 | 3326 | 676 | uacuggagagacauuaagaagac | single | 677 | cuggagagacauuaagaagac | 678 | gucuucuuaaugucucuccagua |
| 6226 | 3327 | 679 | acuggagagacauuaagaagacu | double | 680 | uggagagacauuaagaagaTT | 681 | ucuucuuaaugucucuccaTT |
| 6227 | 3327 | 682 | acuggagagacauuaagaagacu | single | 683 | uggagagacauuaagaagacu | 684 | agucuucuuaaugucucuccagu |
| 6228 | 3328 | 685 | cuggagagacauuaagaagacug | double | 686 | ggagagacauuaagaagacTT | 687 | gucuucuuaaugucucuccTT |
| 6229 | 3328 | 688 | cuggagagacauuaagaagacug | single | 689 | ggagagacauuaagaagacug | 690 | cagucuucuuaaugucucuccag |
| 6230 | 3329 | 691 | uggagagacauuaagaagacugg | double | 692 | gagagacauuaagaagacuTT | 693 | agucuucuuaaugucucucTT |
| 6231 | 3329 | 694 | uggagagacauuaagaagacugg | single | 695 | gagagacauuaagaagacugg | 696 | ccagucuucuuaaugucucucca |
| 6232 | 3330 | 697 | ggagagacauuaagaagacugga | double | 698 | agagacauuaagaagacugTT | 699 | cagucuucuuaaugucucuTT |
| 6233 | 3330 | 700 | ggagagacauuaagaagacugga | single | 701 | agagacauuaagaagacugga | 702 | uccagucuucuuaaugucucucc |
| 6234 | 3331 | 703 | gagagacauuaagaagacuggag | double | 704 | gagacauuaagaagacuggTT | 705 | ccagucuucuuaaugucucTT |
| 6235 | 3331 | 706 | gagagacauuaagaagacuggag | single | 707 | gagacauuaagaagacuggag | 708 | cuccagucuucuuaaugucucuc |
| 6236 | 3332 | 709 | agagacauuaagaagacuggagu | double | 710 | agacauuaagaagacuggaTT | 711 | uccagucuucuuaaugucuTT |
| 6237 | 3332 | 712 | agagacauuaagaagacuggagu | single | 713 | agacauuaagaagacuggagu | 714 | acuccagucuucuuaaugucucu |
| 6238 | 3333 | 715 | gagacauuaagaagacuggagug | double | 716 | gacauuaagaagacuggagTT | 717 | cuccagucuucuuaaugucTT |

(continued)

| Agent number. | Start pos. in RNA[b] | SEQ ID NO. | target sequence (5'-3') | duplex design (over-hang)[a] | sense strand SEQ ID NO. | sense strand sequence (5'-3') | antisense strand SEQ ID NO. | antisense strand sequence (5'-3') |
|---|---|---|---|---|---|---|---|---|
| 6239 | 3333 | 718 | gagacauuaagaagacuggagug | single | 719 | gacauuaagaagacuggagug | 720 | cacuccagucuucuuaaugucuc |
| 6240 | 3334 | 721 | agacauuaagaagacuggagugg | double | 722 | acauuaagaagacuggaguTT | 723 | acuccagucuucuuaauguTT |
| 6241 | 3334 | 724 | agacauuaagaagacuggagugg | single | 725 | acauuaagaagacuggagugg | 726 | ccacuccagucuucuuaaugucu |
| 6242 | 3335 | 727 | gacauuaagaagacuggaguggu | double | 728 | cauuaagaagacuggagugTT | 729 | cacuccagucuucuuaaugTT |
| 6243 | 3335 | 730 | gacauuaagaagacuggaguggu | single | 731 | cauuaagaagacuggaguggu | 732 | accacuccagucuucuuaauguc |
| 6244 | 3336 | 733 | acauuaagaagacuggaguggug | double | 734 | auuaagaagacuggaguggTT | 735 | ccacuccagucuucuuaauTT |
| 6245 | 3336 | 736 | acauuaagaagacuggaguggug | single | 737 | auuaagaagacuggaguggug | 738 | caccacuccagucuucuuaaugu |
| 6246 | 3337 | 739 | cauuaagaagacuggagugguguu | double | 740 | uuaagaagacuggagugguTT | 741 | accacuccagucuucuuaaTT |
| 6247 | 3337 | 742 | cauuaagaagacuggaguggugu | single | 743 | uuaagaagacuggaguggugu | 744 | acaccacuccagucuucuuaaug |
| 6248 | 3338 | 745 | auuaagaagacuggagugguguu | double | 746 | uaagaagacuggaguggugTT | 747 | caccacuccagucuucuuaTT |
| 6249 | 3338 | 748 | auuaagaagacuggagugguguu | single | 749 | uaagaagacuggagugguguu | 750 | aacaccacuccagucuucuuaau |
| 6250 | 3339 | 751 | uuaagaagacuggagugguguuu | double | 752 | aagaagacuggagugguguTT | 753 | acaccacuccagucuucuuTT |
| 6251 | 3339 | 754 | uuaagaagacuggagugguguuu | single | 755 | aagaagacuggagugguguuu | 756 | aaacaccacuccagucuucuuaa |
| 6252 | 3340 | 757 | uaagaagacuggagugguguuug | double | 758 | agaagacuggagugguguuTT | 759 | aacaccacuccagucuucuTT |
| 6253 | 3340 | 760 | uaagaagacuggagugguguuug | single | 761 | agaagacuggagugguguuug | 762 | caaacaccacuccagucuucuua |
| 6254 | 3341 | 763 | aagaagacuggagugguguuugg | double | 764 | gaagacuggagugguguuuTT | 765 | aaacaccacuccagucuucTT |
| 6255 | 3341 | 766 | aagaagacuggagugguguuugg | single | 767 | gaagacuggagugguguuugg | 768 | ccaaacaccacuccagucuucuu |
| 6256 | 3342 | 769 | agaagacuggagugguguuuggu | double | 770 | aagacuggagugguguuugTT | 771 | caaacaccacuccagucuuTT |
| 6257 | 3342 | 772 | agaagacuggagugguguuuggu | single | 773 | aagacuggagugguguuuggu | 774 | accaaacaccacuccagucuucu |
| 6258 | 3343 | 775 | gaagscuggagugguguuuggug | double | 776 | agacuggagugguguuuggTT | 777 | ccaaacaccacuccagucuTT |
| 6259 | 3343 | 778 | gaagacuggagugguguuuggug | single | 779 | agacuggagugguguuuggug | 780 | caccaaacaccacuccagucuuc |
| 6260 | 3344 | 781 | aagacuggagugguguuuggugc | double | 782 | gacuggagugguguuugguTT | 783 | accaaacaccacuccagucTT |
| 6261 | 3344 | 784 | aagacuggagugguguuuggugc | single | 785 | gacuggagugguguuuggugc | 786 | gcaccaaacaccacuccagucuu |
| 6262 | 3345 | 787 | agacuggagugguguuuggugcc | double | 788 | acuggagugguguuuggugTT | 789 | caccaaacaccacuccaguTT |
| 6263 | 3345 | 790 | agacuggagugguguuuggugcc | single | 791 | acuggagugguguuuggugcc | 792 | ggcaccaaacaccacuccagucu |
| 6264 | 3346 | 793 | gacuggagugguguuuggugcca | double | 794 | cuggagugguguuuggugcTT | 795 | gcaccaaacaccacuccagTT |
| 6265 | 3346 | 796 | gacuggagugguguuuggugcca | single | 797 | cuggagugguguuuggugcca | 798 | uggcaccaaacaccacuccaguc |
| 6266 | 3347 | 799 | acuggagugguguuuggugccag | double | 800 | uggagugguguuuggugccTT | 801 | ggcaccaaacaccacuccaTT |
| 6267 | 3347 | 802 | acuggagugguguuuggugccag | single | 803 | uggagugguguuuggugccag | 804 | cuggcaccaaacaccacuccagu |
| 6268 | 3348 | 805 | cuggagugguguuuggugccagc | double | 806 | ggagugguguuuggugccaTT | 807 | uggcaccaaacaccacuccTT |

(continued)

| Agent number. | Start pos. in RNA[b] | SEQ ID NO. | target sequence (5'-3') | duplex design (over-hang)[a] | sense strand SEQ ID NO. | sense strand sequence (5'-3') | antisense strand SEQ ID NO. | antisense strand sequence (5'-3') |
|---|---|---|---|---|---|---|---|---|
| 6269 | 3348 | 808 | cuggaguggugguuuggugccagc | single | 809 | ggaguggugguuuggugccagc | 810 | gcuggcaccaaacaccacuccag |
| 6270 | 3349 | 811 | uggaguggugguuuggugccagcc | double | 812 | 9aguggugguuuggugccagTT | 813 | cuggcaccaaacaccacucTT |
| 6271 | 3350 | 814 | ggaguggugguuuggugccagccu | double | 815 | aguggugguuuggugccagcTT | 816 | gcuggcaccaaacaccacuTT |
| 6272 | 3417 | 817 | cagccuacauugccuuggcccug | double | 818 | gccuacauugccuuggcccTT | 819 | gggccaaggcaauguaggcTT |
| 6273 | 3418 | 820 | agccuacauugccuuggcccugc | double | 821 | ccuacauugccuuggcccuTT | 822 | agggccaaggcaauguaggTT |
| 6274 | 3419 | 823 | gccuacauugccuuggcccugcu | double | 824 | cuacauugccuuggcccugTT | 825 | cagggccaaggcaauguagTT |
| 6275 | 3419 | 826 | gccuacauugccuuggcccugcu | single | 827 | cuacauugccuuggcccugcu | 828 | agcagggccaaggcaauguaggc |
| 6276 | 3420 | 829 | ccuacauugccuuggcccugcuc | double | 830 | uacauugccuuggcccugcTT | 831 | gcagggccaaggcaauguaTT |
| 6277 | 3420 | 832 | ccuacauugccuuggcccugcuc | single | 833 | uacauugccuuggcccugcuc | 834 | gagcagggccaaggcaauguagg |
| 6278 | 3421 | 835 | cuacauugccuuggcccugcucu | double | 836 | acauugccuuggcccugcuTT | 837 | agcagggccaaggcaauguTT |
| 6279 | 3421 | 838 | cuacauugccuuggcccugcucu | single | 839 | acauugccuuggcccugcucu | 840 | agagcagggccaaggcaauguag |
| 6280 | 3422 | 841 | uacauugccuuggcccugcucuc | double | 842 | cauugccuuggcccugcucTT | 843 | gagcagggccaaggcaaugTT |
| 6281 | 3422 | 844 | uacauugccuuggcccugcucuc | single | 845 | cauugccuuggcccugcucuc | 846 | gagagcagggccaaggcaaugua |
| 6282 | 3423 | 847 | acauugccuuggcccugcucucu | double | 848 | auugccuuggcccugcucuTT | 849 | agagcagggccaaggcaauTT |
| 6283 | 3424 | 850 | cauugccuuggcccugcucucug | double | 851 | uugccuuggcccugcucucTT | 852 | gagagcagggccaaggcaaTT |
| 6284 | 3483 | 853 | aagcuauccagaaaucagaugaa | double | 854 | gcuauccagaaaucagaugTT | 855 | caucugauuucuggauagcTT |
| 6285 | 3484 | 856 | agcuauccagaaaucagaugaag | double | 857 | cuauccagaaaucagaugaTT | 858 | ucaueugauuucuggauagTT |
| 6286 | 3485 | 859 | gcuauccagaaaucagaugaagg | double | 860 | uauccagaaaucagaugaaTT | 861 | uucaucugauuucuggauaTT |
| 6287 | 3485 | 862 | gcuauccagaaaucagaugaagg | single | 863 | uauccagaaaucagaugaagg | 864 | ccuucaucugauuucuggauagc |
| 6288 | 3486 | 865 | cuauccagaaaucagaugaaggc | double | 866 | auccagaaaucagaugaagTT | 867 | cuucaucugauuucuggauTT |
| 6289 | 3486 | 868 | cuauccagaaaucagaugaaggc | single | 869 | auccagaaaucagaugaaggc | 870 | gccuucaucugauuucuggauag |
| 6290 | 3487 | 871 | uauccagaaaucagaugaaggcc | double | 872 | uccagaaaucagaugaaggTT | 873 | ccuucaucugauuucuggaTT |
| 6291 | 3487 | 874 | uauccagaaaucagaugaaggcc | single | 875 | uccagaaaucagaugaaggcc | 876 | ggccuucaucugauuucuggaua |
| 6292 | 3488 | 877 | auccagaaaucagaugaaggcca | double | 878 | ccagaaaucagaugaaggcTT | 879 | gccuucaucugauuucuggTT |
| 6293 | 3488 | 880 | auccagaaaucagaugaaggcca | single | 881 | ccagaaaucagaugaaggcca | 882 | uggccuucaucugauuucuggau |
| 6294 | 3489 | 883 | uccagaaaucagaugaaggccac | double | 884 | cagaaaucagaugaaggccTT | 885 | ggccuucaucugauuucugTT |
| 6295 | 3489 | 886 | uccagaaaucagaugaaggccac | single | 887 | cagaaaucagaugaaggccac | 888 | guggccuucaucugauuucugga |
| 6296 | 3490 | 889 | ccagaaaucagaugaaggccacc | double | 890 | agaaaucagaugaaggccaTT | 891 | uggccuucaucugauuucuTT |
| 6297 | 3490 | 892 | ccagaaaucagaugaaggccacc | single | 893 | agaaaucagaugaaggccacc | 894 | gguggccuucaucugauuucugg |
| 6298 | 3491 | 895 | cagaaaucagaugaaggccaccc | double | 896 | gaaaucagaugaaggccacTT | 897 | guggccuucaucugauuucTT |

(continued)

| Agent number. | Start pos. in RNA[b] | SEQ ID NO. | target sequence (5'-3') | duplex design (over-hang)[a] | sense strand SEQ ID NO. | sense strand sequence (5'-3') | antisense strand SEQ ID NO. | antisense strand sequence (5'-3') |
|---|---|---|---|---|---|---|---|---|
| 6299 | 3491 | 898 | cagaaaucagaugaaggccaccc | single | 899 | gaaaucagaugaaggccaccc | 900 | ggguggccuucaucugauuucug |
| 6300 | 3492 | 901 | agaaaucagaugaaggccaccca | double | 902 | aaaucagaugaaggccaccTT | 903 | gguggccuucaucugauuuTT |
| 6301 | 3492 | 904 | agaaaucagaugaaggccaccca | single | 905 | aaaucagaugaaggccaccca | 906 | uggguggccuucaucugauuucu |
| 6302 | 3493 | 907 | gaaaucagaugaaggccacccau | double | 908 | aaucagaugaaggccacccTT | 909 | ggguggccuucaucugauuTT |
| 6303 | 3493 | 910 | gaaaucagaugaaggccacccau | single | 911 | aaucagaugaaggccacccau | 912 | auggguggccuucaucugauuuc |
| 6304 | 3494 | 913 | aaaucagaugaaggccacccauu | double | 914 | aucagaugaaggccacccaTT | 915 | uggguggccuucaucugauTT |
| 6305 | 3494 | 916 | aaaucagaugaaggccacccauu | single | 917 | aucagaugaaggccacccauu | 918 | aauggguggccuucaucugauuu |
| 6306 | 3495 | 919 | aaucagaugaaggccacccauuc | double | 920 | ucagaugaaggccacccauTT | 921 | auggguggccuucaucugaTT |
| 6307 | 3495 | 922 | aaucagaugaaggccacccauuc | single | 923 | ucagaugaaggccacccauuc | 924 | gaauggguggccuucaucugauu |
| 6308 | 3496 | 925 | aucagaugaaggccacccauuca | double | 926 | cagaugaaggccacccauuTT | 927 | aauggguggccuucaucugTT |
| 6309 | 3496 | 928 | aucagaugaaggccacccauuca | single | 929 | cagaugaaggacacccauuca | 930 | ugaauggguggccuucaucugau |
| 6310 | 3497 | 931 | ucagaugaaggccacccauucag | double | 932 | agaugaaggccacccauucTT | 933 | gaauggguggccuucaucuTT |
| 6311 | 3497 | 934 | ucagaugaaggccacccauucag | single | 935 | agaugaaggccacccauucag | 936 | cugaauggguggccuucaucuga |
| 6312 | 3498 | 937 | cagaugaaggccacccauucagg | double | 938 | gaugaaggccacccauucaTT | 939 | ugaauggguggccuucaucTT |
| 6313 | 3498 | 940 | cagaugaaggccacccauucagg | single | 941 | gaugaaggccacccauucagg | 942 | ccugaauggguggccuucaucug |
| 6314 | 3499 | 943 | agaugaaggccacccauucaggg | double | 944 | augaaggccacccauucagTT | 945 | cugaau ggguggccuucauTT |
| 6315 | 3499 | 946 | agaugaaggccacccauucaggg | single | 947 | augaaggccacccauucaggg | 948 | cccugaauggguggccuucaucu |
| 6316 | 3500 | 949 | gaugaaggccacccauucagggc | double | 950 | ugaaggccacccauucaggTT | 951 | ccugaauggguggccuucaTT |
| 6317 | 3500 | 952 | gaugaaggccacccauucagggc | single | 953 | ugaaggccacccauucagggc | 954 | gcccugaauggguggccuucauc |
| 6318 | 3501 | 955 | augaaggccacccauucagggca | double | 956 | gaaggccacccauucagggTT | 957 | cccugaauggguggccuucTT |
| 6319 | 3501 | 958 | augaaggccacccauucagggca | single | 959 | gaaggccacccauucagggca | 960 | ugcccugaauggguggccuucau |
| 6320 | 3502 | 961 | ugaaggccacccauucagggcau | double | 962 | aaggccacccauucagggcTT | 963 | gcccugaauggguggccuuTT |
| 6321 | 3502 | 964 | ugaaggccacccauucagggcau | single | 965 | aaggccacccauucagggcau | 966 | augcccugaauggguggccuuca |
| 6322 | 3503 | 967 | gaaggccacccauucagggcaua | double | 968 | aggccacccauucagggcaTT | 969 | ugcccugaauggguggccuTT |
| 6323 | 3503 | 970 | gaaggccacccauucagggcaua | single | 971 | aggccacccauucagggcaua | 972 | uaugcccugaauggguggccuuc |
| 6324 | 3504 | 973 | aaggccacccauucagggcauau | double | 974 | ggccacccauucagggcauTT | 975 | augcccugaauggguggccTT |
| 6325 | 3504 | 976 | aaggccacccauucagggcauau | single | 977 | ggccacccauucagggcauau | 978 | auaugcccugaauggguggccuu |
| 6326 | 3505 | 979 | aggccacccauucagggcauauc | double | 980 | gccacccauucagggcauaTT | 981 | uaugcccugaauggguggcTT |
| 6327 | 3506 | 982 | ggccacccauucagggcauaucu | double | 983 | ccacccauucagggcauauTT | 984 | auaugcccugaauggguggTT |
| 6328 | 3570 | 985 | acaguaauucugcucuuggucau | double | 986 | aguaauucugcucuuggucTT | 987 | gaccaagagcagaauuacuTT |

(continued)

| Agent number. | Start pos. in RNA[b] | SEQ ID NO. | target sequence (5'-3') | duplex design (over-hang)[a] | sense strand SEQ ID NO. | sense strand sequence (5'-3') | antisense strand SEQ ID NO. | antisense strand sequence (5'-3') |
|---|---|---|---|---|---|---|---|---|
| 6329 | 3571 | 988 | caguaauucugcucuuggucaug | double | 989 | guaauucugcucuuggucaTT | 990 | ugaccaagagcagaauuacTT |
| 6330 | 3572 | 991 | aguaauucugcucuuggucaugu | double | 992 | uaauucugcucuuggucauTT | 993 | augaccaagagcagaauuaTT |
| 6331 | 3572 | 994 | aguaauucugcucuuggucaugu | single | 995 | uaauucugcucuuggucaugu | 996 | acaugaccaagagcagaauuacu |
| 6332 | 3573 | 997 | guaauucugcucuuggucaugug | double | 998 | aauucugcucuuggucaugTT | 999 | caugaccaagagcagaauuTT |
| 6333 | 3573 | 1000 | guaauucugcucuuggucaugug | single | 1001 | aauucugcucuuggucaugug | 1002 | cacaugaccaagagcagaauuac |
| 6334 | 3574 | 1003 | uaauucugcucuuggucauguga | double | 1004 | auucugcucuuggucauguTT | 1005 | acaugaccaagagcagaauTT |
| 6335 | 3574 | 1006 | uaauucugcucuuggucauguga | single | 1007 | auucugcucuuggucauguga | 1008 | ucacaugaccaagagcagaauua |
| 6336 | 3575 | 1009 | aauucugcucuuggucaugugaa | double | 1010 | uucugcucuuggucaugugTT | 1011 | cacaugaccaagagcagaaTT |
| 6337 | 3575 | 1012 | aauucugcucuuggucaugugaa | single | 1013 | uucugcucuuggucaugugaa | 1014 | uucacaugaccaagagcagaauu |
| 6338 | 3576 | 1015 | auucugcucuuggucaugugaac | double | 1016 | ucugcucuuggugaugugaTT | 1017 | ucacaugaccaagagcagaTT |
| 6339 | 3576 | 1018 | auucugcucuuggucaugugaac | single | 1019 | ucugcucuuggucaugugaac | 1020 | guucacaugaccaagagcagaau |
| 6340 | 3577 | 1021 | uucugcucuuggucaugugaacu | double | 1022 | cugcucuuggucaugugaaTT | 1023 | uucacaugaccaagagcagTT |
| 6341 | 3578 | 1024 | ucugcucuuggucaugugaacug | double | 1025 | ugcucuuggucaugugaacTT | 1026 | guucacaugaccaagagcaTT |
| 6342 | 3624 | 1027 | ucuucuuaguugaugauuuaguu | double | 1028 | uucuuaguugaugauuuagTT | 1029 | cuaaaucaucaacuaagaaTT |
| 6343 | 3625 | 1030 | cuucuuaguugaugauuuaguug | double | 1031 | ucuuaguugaugauuuaguTT | 1032 | acuaaaucaucaacuaagaTT |
| 6344 | 3626 | 1033 | uucuuaguugaugauuuaguuga | double | 1034 | cuuaguugaugauuuaguuTT | 1035 | aacuaaaucaucaacuaagTT |
| 6345 | 3626 | 1036 | uucuuaguugaugauuuaguuga | single | 1037 | cuuaguugaugauuuaguuga | 1038 | ucaacuaaaucaucaacuaagaa |
| 6346 | 3627 | 1039 | ucuuaguugaugauuuaguugau | double | 1040 | uuaguugaugauuuaguugTT | 1041 | caacuaaaucaucaacuaaTT |
| 6347 | 3627 | 1042 | ucuuaguugaugauuuaguugau | single | 1043 | uuaguugaugauuuaguugau | 1044 | aucaacuaaaucaucaacuaaga |
| 6348 | 3628 | 1045 | cuuaguugaugauuuaguugauu | double | 1046 | uaguugaugauuuaguugaTT | 1047 | ucaacuaaaucaucaacuaTT |
| 6349 | 3628 | 1048 | cuuaguugaugauuuaguugauu | single | 1049 | uaguugaugauuuaguugauu | 1050 | aaucaacuaaaucaucaacuaag |
| 6350 | 3629 | 1051 | uuaguugaugauuuaguugauuc | double | 1052 | aguugaugauuuaguugauTT | 1053 | aucaacuaaaucaucaacuTT |
| 6351 | 3629 | 1054 | uuaguugaugauuuaguugauuc | single | 1055 | aguugaugauuuaguugauuc | 1056 | gaaucaacuaaaucaucaacuaa |
| 6352 | 3630 | 1057 | uaguugaugauuuaguugauucu | double | 1058 | guugaugauuuaguugauuTT | 1059 | aaucaacuaaaucaucaacTT |
| 6353 | 3631 | 1060 | aguugaugauuuaguugauucuc | double | 1061 | uugaugauuuaguugauucTT | 1062 | gaaucaacuaaaucaucaaTT |
| 6354 | 3651 | 1063 | cucugaaguuugcaguguugaug | double | 1064 | cugaaguuugcaguguugaTT | 1065 | ucaacacugcaaacuucagTT |
| 6355 | 3652 | 1066 | ucugaaguuugcaguguugaugu | double | 1067 | ugaaguuugcaguguugauTT | 1068 | aucaacacugcaaacuucaTT |
| 6356 | 3653 | 1069 | cugaaguuugcaguguugaugug | double | 1070 | gaaguuugcaguguugaugTT | 1071 | caucaacacugcaaacuucTT |
| 6357 | 3653 | 1072 | cugaaguuugcaguguugaugug | single | 1073 | gaaguuugcaguguugaugug | 1074 | cacaucaacacugcaaacuucag |
| 6358 | 3654 | 1075 | ugaaguuugcaguguugaugugg | double | 1076 | aaguuugcaguguugauguTT | 1077 | acaucaacacugcaaacuuTT |

(continued)

| Agent number. | Start pos. in RNA[b] | SEQ ID NO. | target sequence (5'-3') | duplex design (over-hang)[a] | sense strand SEQ ID NO. | sense strand sequence (5'-3') | antisense strand SEQ ID NO. | antisense strand sequence (5'-3') |
|---|---|---|---|---|---|---|---|---|
| 6359 | 3654 | 1078 | ugaaguuugcaguguugaugugg | single | 1079 | aaguuugcaguguugaugugg | 1080 | ccacaucaacacugcaaacuuca |
| 6360 | 3655 | 1081 | gaaguuugcaguguugaugugg | double | 1082 | aguuugcaguguugaugugTT | 1083 | cacaucaacacugcaaacuTT |
| 6361 | 3655 | 1084 | gaaguuugcaguguugaugugg | single | 1085 | aguuugcaguguugaugugg | 1086 | cccacaucaacacugcaaacuuc |
| 6362 | 3656 | 1087 | aaguuugcaguguugauguggu | double | 1088 | guuugcaguguugauguggTT | 1089 | ccacaucaacacugcaaacTT |
| 6363 | 3656 | 1090 | aaguuugcaguguugauguggu | single | 1091 | guuugcaguguugauguggu | 1092 | acccacaucaacacugcaaacuu |
| 6364 | 3657 | 1093 | aguuugcaguguugauguggua | double | 1094 | uuugcaguguugaugugggTT | 1095 | cccacaucaacacugcaaaTT |
| 6365 | 3658 | 1096 | guuugcaguguugaugugguau | double | 1097 | uugcaguguugaugugguTT | 1098 | acccacaucaacacugcaaTT |
| 6366 | 3777 | 1099 | cacagauagaucauuaucuagga | double | 1100 | cagauagaucauuaucuagTT | 1101 | cuagauaaugaucuaucugTT |
| 6367 | 3778 | 1102 | acagauagaucauuaucuaggac | double | 1103 | agauagaucauuaucuaggTT | 1104 | ccuagauaaugaucuaucuTT |
| 6368 | 3779 | 1105 | cagauagaucauuaucuaggacu | double | 1106 | gauagaucauuaucuaggaTT | 1107 | uccuagauaaugaucuaucTT |
| 6369 | 3779 | 1108 | cagauagaucauuaucuaggacu | single | 1109 | gauagaucauuaucuaggacu | 1110 | aguccuagauaaugaucuaucug |
| 6370 | 3780 | 1111 | agauagaucauuaucuaggacuu | double | 1112 | auagaucauuaucuaggacTT | 1113 | guccuagauaaugaucuauTT |
| 6371 | 3780 | 1114 | agauagaucauuaucuaggacuu | single | 1115 | auagaucauuaucuaggacuu | 1116 | aaguccuagauaaugaucuaucu |
| 6372 | 3781 | 1117 | gauagaucauuaucuaggacuug | double | 1118 | uagaucauuaucuaggacuTT | 1119 | aguccuagauaaugaucuaTT |
| 6373 | 3781 | 1120 | gauagaucauuaucuaggacuug | single | 1121 | uagaucauuaucuaggacuug | 1122 | caaguccuagauaaugaucuauc |
| 6374 | 3782 | 1123 | auagaucauuaucuaggacuugc | double | 1124 | agaucauuaucuaggacuuTT | 1125 | aaguccuagauaaugaucuTT |
| 6375 | 3782 | 1126 | auagaucauuaucuaggacuugc | single | 1127 | agaucauuaucuaggacuugc | 1128 | gcaaguccuagauaaugaucuau |
| 6376 | 3783 | 1129 | uagaucauuaucuaggacuugca | double | 1130 | gaucauuaucuaggacuugTT | 1131 | caaguccuagauaaugaucTT |
| 6377 | 3783 | 1132 | uagaucauuaucuaggacuugca | single | 1133 | gaucauuaucuaggacuugca | 1134 | ugcaaguccuagauaaugaucua |
| 6378 | 3784 | 1135 | agaucauuaucuaggacuugcaa | double | 1136 | aucauuaucuaggacuugcTT | 1137 | gcaaguccuagauaaugauTT |
| 6379 | 3784 | 1138 | agaucauuaucuaggacuugcaa | single | 1139 | aucauuaucuaggacuugcaa | 1140 | uugcaaguccuagauaaugaucu |
| 6380 | 3785 | 1141 | gaucauuaucuaggacuugcaaa | double | 1142 | ucauuaucuaggacuugcaTT | 1143 | ugcaaguccuagauaaugaTT |
| 6381 | 3785 | 1144 | gaucauuaucuaggacuugcaaa | single | 1145 | ucauuaucuaggacuugcaaa | 1146 | uuugcaaguccuagauaaugauc |
| 6382 | 3786 | 1147 | aucauuaucuaggacuugcaaau | double | 1148 | cauuaucuaggacuugcaaTT | 1149 | uugcaaguccuagauaaugTT |
| 6383 | 3787 | 1150 | ucauuaucuaggacuugcaaaua | double | 1151 | auuaucuaggacuugcaaaTT | 1152 | uuugcaaguccuagauaauTT |
| 6384 | 3831 | 1153 | cuaaaauccaagcaaaaaucccu | double | 1154 | aaaauccaagcaaaaauccTT | 1155 | ggauuuuugcuuggauuuuTT |
| 6385 | 3832 | 1156 | uaaaauccaagcaaaaaucccug | double | 1157 | aaauccaagcaaaaaucccTT | 1158 | gggauuuuugcuuggauuuTT |
| 6386 | 3833 | 1159 | aaaauccaagcaaaaaucccugg | double | 1160 | aauccaagcaaaaaucccuTT | 1161 | agggauuuuugcuuggauuTT |
| 6387 | 3833 | 1162 | aaaauccaagcaaaaaucccugg | single | 1163 | aauccaagcaaaaaucccugg | 1164 | ccagggauuuuugcuuggauuuu |
| 6388 | 3834 | 1165 | aaauccaagcaaaaaucccugga | double | 1166 | auccaagcaaaaaucccugTT | 1167 | cagggauuuuugcuuggauTT |

(continued)

| Agent number. | Start pos. in RNA[b] | SEQ ID NO. | target sequence (5'-3') | duplex design (over-hang)[a] | sense strand SEQ ID NO. | sense strand sequence (5'-3') | antisense strand SEQ ID NO. | antisense strand sequence (5'-3') |
|---|---|---|---|---|---|---|---|---|
| 6389 | 3834 | 1168 | aaauccaagcaaaaaucccugga | single | 1169 | auccaagcaaaaaucccugga | 1170 | uccagggauuuuugcuuggauuu |
| 6390 | 3835 | 1171 | aauccaagcaaaaaucccuggau | double | 1172 | uccaagcaaaaaucccuggTT | 1173 | ccagggauuuuugcuuggaTT |
| 6391 | 3835 | 1174 | aauccaagcaaaaaucccuggau | single | 1175 | uccaagcaaaaaucccuggau | 1176 | auccagggauuuuugcuuggauu |
| 6392 | 3836 | 1177 | auccaagcaaaaaucccuggauu | double | 1178 | ccaagcaaaaaucccuggaTT | 1179 | uccagggauuuuugcuuggTT |
| 6393 | 3836 | 1180 | auccaagcaaaaaucccuggauu | single | 1181 | ccaagcaaaaaucccuggauu | 1182 | aauccagggauuuuugcuuggau |
| 6394 | 3837 | 1183 | uccaagcaaaaaucccuggauug | double | 1184 | caagcaaaaaucccuggauTT | 1185 | auccagggauuuuugcuugTT |
| 6395 | 3837 | 1186 | uccaagcaaaaaucccuggauug | single | 1187 | caagcaaaaaucccuggauug | 1188 | caauccagggauuuuugcuugga |
| 6396 | 3838 | 1189 | ccaagcaaaaaucccuggauuga | double | 1190 | aagcaaaaaucccuggauuTT | 1191 | aauccagggauuuuugcuuTT |
| 6397 | 3838 | 1192 | ccaagcaaaaaucccuggauuga | single | 1193 | aagcaaaaaucccuggauuga | 1194 | ucaauccagggauuuuugcuugg |
| 6398 | 3839 | 1195 | caagcaaaaaucccuggauugaa | double | 1196 | agcaaaaaucccuggauugTT | 1197 | caauccagggauuuuugcuTT |
| 6399 | 3839 | 1198 | caagcaaaaaucccuggauugaa | single | 1199 | agcaaaaaucccuggauugaa | 1200 | uucaauccagggauuuuugcuug |
| 6400 | 3840 | 1201 | aagcaaaaaucccuggauugaag | double | 1202 | gcaaaaaucccuggauugaTT | 1203 | ucaauccagggauuuuugcTT |
| 6401 | 3840 | 1204 | aagcaaaaaucccuggauugaag | single | 1205 | gcaaaaaucccuggauugaag | 1206 | cuucaauccagggauuuuugcuu |
| 6402 | 3841 | 1207 | agcaaaaaucccuggauugaagc | double | 1208 | caaaaaucccuggauugaaTT | 1209 | uucaauccagggauuuuugTT |
| 6403 | 3841 | 1210 | agcaaaaaucccuggauugaagc | single | 1211 | caaaaaucccuggauugaagc | 1212 | gcuucaauccagggauuuuugcu |
| 6404 | 3842 | 1213 | gcaaaaaucccuggauugaagcg | double | 1214 | aaaaaucccuggauugaagTT | 1215 | cuucaauccagggauuuuuTT |
| 6405 | 3842 | 1216 | gcaaaaaucccuggauugaagcg | single | 1217 | aaaaaucccuggauugaagcg | 1218 | cgcuucaauccagggauuuuugc |
| 6406 | 3843 | 1219 | caaaaaucccuggauugaagcgc | double | 1220 | aaaaucccuggauugaagcTT | 1221 | gcuucaauccagggauuuuTT |
| 6407 | 3843 | 1222 | caaaaaucccuggauugaagcgc | single | 1223 | aaaaucccuggauugaagcgc | 1224 | gcgcuucaauccagggauuuuug |
| 6408 | 3844 | 1225 | aaaaaucccuggauugaagcgca | double | 1226 | aaaucccuggauugaagcgTT | 1227 | cgcuucaauccagggauuuTT |
| 6409 | 3844 | 1228 | aaaaaucccuggauugaagcgca | single | 1229 | aaaucccuggauugaagcgca | 1230 | ugcgcuucaauccagggauuuuu |
| 6410 | 3845 | 1231 | aaaaucccuggauugaagcgcaa | double | 1232 | aaucccuggauugaagcgcTT | 1233 | gcgcuucaauccagggauuTT |
| 6411 | 3845 | 1234 | aaaaucccuggauugaagcgcaa | single | 1235 | aaucccuggauugaagcgcaa | 1236 | uugcgcuucaauccagggauuuu |
| 6412 | 3846 | 1237 | aaaucccuggauugaagcgcaaa | double | 1238 | aucccuggauugaagcgcaTT | 1239 | ugcgcuucaauccagggauTT |
| 6413 | 3846 | 1240 | aaaucccuggauugaagcgcaaa | single | 1241 | aucccuggauugaagcgcaaa | 1242 | uuugcgcuucaauccagggauuu |
| 6414 | 3847 | 1243 | aaucccuggauugaagcgcaaag | double | 1244 | ucccuggauugaagcgcaaTT | 1245 | uugcgcuucaauccagggaTT |
| 6415 | 3847 | 1246 | aaucccuggauugaagcgcaaag | single | 1247 | ucccuggauugaagcgcaaag | 1248 | cuuugcgcuucaauccagggauu |
| 6416 | 3848 | 1249 | aucccuggauugaagcgcaaagc | double | 1250 | cccuggauugaagcgcaaaTT | 1251 | uuugcgcuucaauccagggTT |
| 6417 | 3848 | 1252 | aucccuggauugaagcgcaaagc | single | 1253 | cccuggauugaagcgcaaagc | 1254 | gcuuugcgcuucaauccagggau |
| 6418 | 3849 | 1255 | ucccuggauugaagcgcaaagcu | double | 1256 | ccuggauugaagcgcaaagTT | 1257 | cuuugcgcuucaauccaggTT |

(continued)

| Agent number. | Start pos. in RNA[b] | SEQ ID NO. | target sequence (5'-3') | duplex design (over-hang)[a] | sense strand SEQ ID NO. | sense strand sequence (5'-3') | antisense strand SEQ ID NO. | antisense strand sequence (5'-3') |
|---|---|---|---|---|---|---|---|---|
| 6419 | 3850 | 1258 | cccuggauugaagcgcaaagcug | double | 1259 | cuggauugaagcgcaaagcTT | 1260 | gcuuugcgcuucaauccagTT |
| 6420 | 4024 | 1261 | ugugaggaaaaauuaccugucuu | double | 1262 | ugaggaaaaauuaccugucTT | 1263 | gacagguaauuuuuccucaTT |
| 6421 | 4025 | 1264 | gugaggaaaaauuaccugucuug | double | 1265 | gaggaaaaauuaccugucuTT | 1266 | agacagguaauuuuuccucTT |
| 6422 | 4026 | 1267 | ugaggaaaaauuaccugucuuga | double | 1268 | aggaaaaauuaccugucuuTT | 1269 | aagacagguaauuuuuccuTT |
| 6423 | 4026 | 1270 | ugaggaaaaauuaccugucuuga | single | 1271 | aggaaaaauuaccugucuuga | 1272 | ucaagacagguaauuuuuccuca |
| 6424 | 4027 | 1273 | gaggaaaaauuaccugucuugac | double | 1274 | ggaaaaauuaccugucuugTT | 1275 | caagacagguaauuuuuccTT |
| 6425 | 4027 | 1276 | gaggaaaaauuaccugucuugac | single | 1277 | ggaaaaauuaccugucuugac | 1278 | gucaagacagguaauuuuuccuc |
| 6426 | 4028 | 1279 | aggaaaaauuaccugucuugacu | double | 1280 | gaaaaauuaccugucuugaTT | 1281 | ucaagacagguaauuuuucTT |
| 6427 | 4028 | 1282 | aggaaaaauuaccugucuugacu | single | 1283 | gaaaaauuaccugucuugacu | 1284 | agucaagacagguaauuuuuccu |
| 6428 | 4029 | 1285 | ggaaaaauuaccugucuugacug | double | 1286 | aaaaauuaccugucuugacTT | 1287 | gucaagacagguaauuuuuTT |
| 6429 | 4030 | 1288 | gaaaaauuaccugucuugacugc | double | 1289 | aaaauuaccugucuugacuTT | 1290 | agucaagacagguaauuuuTT |
| 6430 | 4060 | 1291 | ucaucaucuuaaguauuguaagc | double | 1292 | aucaucuuaaguauuguaaTT | 1293 | uuacaauacuuaagaugauTT |
| 6431 | 4061 | 1294 | caucaucuuaaguauuguaagcu | double | 1295 | ucaucuuaaguauuguaagTT | 1296 | cuuacaauacuuaagaugaTT |
| 6432 | 4062 | 1297 | aucaucuuaaguauuguaagcug | double | 1298 | caucuuaaguauuguaagcTT | 1299 | gcuuacaauacuuaagaugTT |
| 6433 | 4062 | 1300 | aucaucuuaaguauuguaagcug | single | 1301 | caucuuaaguauuguaagcug | 1302 | cagcuuacaauacuuaagaugau |
| 6434 | 4063 | 1303 | ucaucuuaaguauuguaagcugc | double | 1304 | aucuuaaguauuguaagcuTT | 1305 | agcuuacaauacuuaagauTT |
| 6435 | 4063 | 1306 | ucaucuuaaguauuguaagcugc | single | 1307 | aucuuaaguauuguaagcugc | 1308 | gcagcuuacaauacuuaagauga |
| 6436 | 4064 | 1309 | caucuuaaguauuguaagcugcu | double | 1310 | ucuuaaguauuguaagcugTT | 1311 | cagcuuacaauacuuaagaTT |
| 6437 | 4064 | 1312 | caucuuaaguauuguaagcugcu | single | 1313 | ucuuaaguauuguaagcugcu | 1314 | agcagcuuacaauacuuaagaug |
| 6438 | 4065 | 1315 | aucuuaaguauuguaagcugcua | double | 1316 | cuuaaguauuguaagcugcTT | 1317 | gcagcuuacaauacuuaagTT |
| 6439 | 4066 | 1318 | ucuuaaguauuguaagcugcuau | double | 1319 | uuaaguauuguaagcugcuTT | 1320 | agcagcuuacaauacuuaaTT |
| 6440 | 4360 | 1321 | uauguucuagguguauugugacu | double | 1322 | uguucuagguguauugugaTT | 1323 | ucacaauacaccuagaacaTT |
| 6441 | 4398 | 1324 | auugccaauauaaguaaauauag | double | 1325 | ugccaauauaaguaaauauTT | 1326 | auauuuacuuauauuggcaTT |
| 6442 | 4399 | 1327 | uugccaauauaaguaaauauaga | double | 1328 | gccaauauaaguaaauauaTT | 1329 | uauauuuacuuauauuggcTT |
| 6443 | 4400 | 1330 | ugccaauauaaguaaauauagau | double | 1331 | ccaauauaaguaaauauagTT | 1332 | cuauauuuacuuauauuggTT |
| 6444 | 4401 | 1333 | gccaauauaaguaaauauagauu | double | 1334 | caauauaaguaaauauagaTT | 1335 | ucuauauuuacuuauauugTT |
| 6445 | 4431 | 1336 | uauaguguuucacaaagcuuaga | double | 1337 | uaguguuucacaaagcuuaTT | 1338 | uaagcuuugugaaacacuaTT |
| 6446 | 4432 | 1339 | auaguguuucacaaagcuuagac | double | 1340 | aguguuucacaaagcuuagTT | 1341 | cuaagcuuugugaaacacuTT |
| 6447 | 4433 | 1342 | uaguguuucacaaagcuuagacc | double | 1343 | guguuucacaaagcuuagaTT | 1344 | ucuaagcuuugugaaacacTT |
| 6448 | 4434 | 1345 | aguguuucacaaagcuuagaccu | double | 1346 | uguuucacaaagcuuagacTT | 1347 | gucuaagcuuugugaaacaTT |

(continued)

| Agent number. | Start pos. in RNA[b] | SEQ ID NO. | target sequence (5'-3') | duplex design (over-hang)[a] | sense strand SEQ ID NO. | sense strand sequence (5'-3') | antisense strand SEQ ID NO. | antisense strand sequence (5'-3') |
|---|---|---|---|---|---|---|---|---|
| 6449 | 4705 | 1348 | aucaaacauuguuaugcaagaaa | double | 1349 | caaacauuguuaugcaagaTT | 1350 | ucuugcauaacaauguuugTT |
| 6450 | 4706 | 1351 | ucaaacauuguuaugcaagaaau | double | 1352 | aaacauuguuaugcaagaaTT | 1353 | uucuugcauaacaauguuuTT |
| 6451 | 4707 | 1354 | caaacauuguuaugcaagaaauu | double | 1355 | aacauuguuaugcaagaaaTT | 1356 | uuucuugcauaacaauguuTT |
| 6452 | 4708 | 1357 | aaacauuguuaugcaagaaauua | double | 1358 | acauuguuaugcaagaaauTT | 1359 | auuucuugcauaacaauguTT |
| 6453 | 4783 | 1360 | aaucuguggaaugcauugugaac | double | 1361 | ucuguggaaugcauugugaTT | 1362 | ucacaaugcauuccacagaTT |
| 6454 | 4784 | 1363 | aucuguggaaugcauugugaacu | double | 1364 | cuguggaaugcauugugaaTT | 1365 | uucacaaugcauuccacagTT |
| 6455 | 4785 | 1366 | ucuguggaaugcauugugaacug | double | 1367 | uguggaaugcauugugaacTT | 1368 | guucacaaugcauuccacaTT |
| 6456 | 4785 | 1369 | ucuguggaaugcauugugaacug | single | 1370 | uguggaaugcauugugaacug | 1371 | caguucacaaugcauuccacaga |
| 6457 | 4786 | 1372 | cuguggaaugcauugugaacugu | double | 1373 | guggaaugcauugugaacuTT | 1374 | aguucacaaugcauuccacTT |
| 6458 | 4786 | 1375 | cuguggaaugcauugugaacugu | single | 1376 | guggaaugcauugugaacugu | 1377 | acaguucacaaugcauuccacag |
| 6459 | 4787 | 1378 | uguggaaugcauugugaacugua | double | 1379 | uggaaugcauugugaacugTT | 1380 | caguucacaaugcauuccaTT |
| 6460 | 4787 | 1381 | uguggaaugcauugugaacugua | single | 1382 | uggaaugcauugugaacugua | 1383 | uacaguucacaaugcauuccaca |
| 6461 | 4788 | 1384 | guggaaugcauugugaacuguaa | double | 1385 | ggaaugcauugugaacuguTT | 1386 | acaguucacaaugcauuccTT |
| 6462 | 4788 | 1387 | guggaaugcauugugaacuguaa | single | 1388 | ggaaugcauugugaacuguaa | 1389 | uuacaguucacaaugcauuccac |
| 6463 | 4789 | 1390 | uggaaugcauugugaacuguaaa | double | 1391 | gaaugcauugugaacuguaTT | 1392 | uacaguucacaaugcauucTT |
| 6464 | 4789 | 1393 | uggaaugcauugugaacuguaaa | single | 1394 | gaaugcauugugaacuguaaa | 1395 | uuuacaguucacaaugcauucca |
| 6465 | 4790 | 1396 | ggaaugcauugugaacuguaaaa | double | 1397 | aaugcauugugaacuguaaTT | 1398 | uuacaguucacaaugeauuTT |
| 6466 | 4791 | 1399 | gaaugcauugugaacuguaaaag | double | 1400 | augcauugugaacuguaaaTT | 1401 | uuuacaguucacaaugcauTT |
| 6467 | 4811 | 1402 | aagcaaaguaucaauaaagcuua | double | 1403 | gcaaaguaucaauaaagcuTT | 1404 | agcuuuauugauacuuugcTT |
| 6468 | 4812 | 1405 | agcaaaguaucaauaaagcuuau | double | 1406 | caaaguaucaauaaagcuuTT | 1407 | aagcuuuauugauacuuugTT |
| 6469 | 4813 | 1408 | gcaaaguaucaauaaagcuuaua | double | 1409 | aaaguaucaauaaagcuuaTT | 1410 | uaagcuuuauugauacuuuTT |
| 6470 | 4813 | 1411 | gcaaaguaucaauaaagcuuaua | single | 1412 | aaaguaucaauaaagcuuaua | 1413 | uauaagcuuuauugauacuuugc |
| 6471 | 4814 | 1414 | caaaguaucaauaaagcuuauag | double | 1415 | aaguaucaauaaagcuuauTT | 1416 | auaagcuuuauugauacuuTT |
| 6472 | 4814 | 1417 | caaaguaucaauaaagcuuauag | single | 1418 | aaguaucaauaaagcuuauag | 1419 | cuauaagcuuuauugauacuuug |
| 6473 | 4815 | 1420 | aaaguaucaauaaagcuuauaga | double | 1421 | aguaucaauaaagcuuauaTT | 1422 | uauaagcuuuauugauacuTT |
| 6474 | 4815 | 1423 | aaaguaucaauaaagcuuauaga | single | 1424 | aguaucaauaaagcuuauaga | 1425 | ucuauaagcuuuauugauacuuu |
| 6475 | 4816 | 1426 | aaguaucaauaaagcuuauagac | double | 1427 | guaucaauaaagcuuauagTT | 1428 | cuauaagcuuuauugauacTT |
| 6476 | 4817 | 1429 | aguaucaauaaagcuuauagacu | double | 1430 | uaucaauaaagcuuauagaTT | 1431 | ucuauaagcuuuauugauaTT |

(continued)

| Agent number. | Start pos. in RNA[b] | SEQ ID NO. | target sequence (5'-3') | duplex design (over-hang)[a] | sense strand SEQ ID NO. | sense strand sequence (5'-3') | antisense strand SEQ ID NO. | antisense strand sequence (5'-3') |
|---|---|---|---|---|---|---|---|---|
| 6837 | 2534 | 1432 | gacuuauuuagugaugauucaau | double | 1433 | cuuauuuagugaugauucaTT | 1434 | ugaaucaucacuaaauaagTT |

[a]Single: Single overhang design 21mer sense, corresponding 23mer antisense with 2 nucleotides overhang at 3' end; Double: double overhang design corresponding 19mer sense and antisense strand each with 2 dT nucleotide overhang at 3' end

[b]"Start position" corresponds to the position within the sequence of human NOGO (Genbank accession no. NM_020532) mRNA, to which the 5'-most nucleotide of the sense strand corresponds for single overhang designs; for double overhang designs, the 5'-most ribonucleotide of the sense strand corresponds to (Start position +2)

Based on these results, specifically described herein is an iRNA agent that includes a sense strand having at least 15 contiguous nucleotides of the sense strand sequences of the agents provided in Table 1 or Table 2 under agent numbers 6000 - 6476 and 6837, and an antisense stand having at least 15 contiguous nucleotides of the antisense sequences of the agents provided in Table 1 or Table 2 under agent numbers 6000 - 6476 and 6837.

The iRNA agents shown in Table 1 and Table 2 are composed either of two strands of 19 nucleotides in length which are complementary or identical to the target sequence, plus a 3'-TT overhang, or of an antisense strand 23 nucleotides in length which is complementary to the target sequence and a sense strand of 21 nucleotides in length which is complementary to positions 1 to 21 of the antisense strand. Described herein are agents that comprise 15 contiguous nucleotides from these agents. However, while these lengths may potentially be optimal, the iRNA agents are not meant to be limited to these lengths. The skilled person is well aware that shorter or longer iRNA agents may be similarly effective, since, within certain length ranges, the efficacy is rather a function of the nucleotide sequence than strand length. For example, Yang, at al., PNAS 99:9942-9947 (2002), demonstrated similar efficacies for iRNA agents of lengths between 21 and 30 base pairs. Others have shown effective silencing of genes by iRNA agents down to a length of approx. 15 base pairs (Byrom, et al., "Inducing RNAi with siRNA Cocktails Generated by RNase III" Tech Notes 10(1), Ambion, Inc., Austin, TX).

Therefore, it is possible to select from the sequences provided in Table 1 and Table 2 under agent numbers 6000 - 6476 and 6837 a partial sequence of between 15 to 22 nucleotides for the generation of an iRNA agent derived from one of the sequences provided in Table 1 and Table 2 under agent numbers 6000 - 6476 and 6837. Alternatively, one may add one or several nucleotides to one of the sequences provided in Table 1 and Table 2 under agent numbers 6000 - 6476 and 6837, or an agent comprising 15 contiguous nucleotides from one of these agents, preferably, but not necessarily, in such a fashion that the added nucleotides are complementary to the respective sequence of the target gene, *e.g.*, Nogo-L or Nogo-R. For example, the first 15 nucleotides from one of the agents can be combined with the 8 nucleotides found 5' to these sequence in the Nogo-L or Nogo-R mRNA to obtain an agent with 23 nucleotides in the sense and antisense strands. All such derived iRNA agents are included in the iRNA agents also described herein, provided they essentially retain the ability to inhibit Nogo-L or Nogo-R expression in cultured human Nogo-L or Nogo-R expressing cells.

The antisense strand of an iRNA agent should be equal to or at least, 14,15,16 17, 18, 19, 25, 29, 40, or 50 nucleotides in length. It should be equal to or less than 60, 50,40, or 30, nucleotides in length. Preferred ranges are 15-30, 17 to 25, 19 to 23, and 19 to 21 nucleotides in length.

The sense strand of an iRNA agent should be equal to or at least 14, 15, 16 17, 18, 19, 25, 29, 40, or 50 nucleotides in length. It should be equal to or less than 60, 50, 40, or 30 nucleotides in length. Preferred ranges are 15-30, 17 to 25, 19 to 23, and 19 to 21 nucleotides in length.

The double stranded portion of an iRNA agent should be equal to or at least, 15, 16 17, 18, 19, 20, 21, 22, 23, 24, 25, 29, 40, or 50 nucleotide pairs in length. It should be equal to or less than 60, 50, 40, or 30 nucleotides pairs in length. Preferred ranges are 15-30, 17 to 25, 19 to 23, and 19 to 21 nucleotides pairs in length.

Generally, the iRNA agents described herein include a region of sufficient complementarity to the respective Nogo-L or Nogo-R gene, and are of sufficient length in terms of nucleotides, that the iRNA agent, or a fragment thereof, can mediate down regulation of the Nogo-L or Nogo-R gene. The ribonucleotide portions of the antisense strands of the iRNA agents of Table 1 and Table 2 under agent numbers 6000 - 6476 and 6837 are fully complementary to the mRNA sequences of the Nogo-L or Nogo-R gene, respectively, and ribonucleotide portion of their sense strands are fully complementary to the ribonucleotide portions of the respective antisense strands, except for the two 3'-terminal nucleotides on the antisense strand in single overhang design iRNA agents. However, it is not necessary that there be perfect complementarity between the iRNA agent and the target, but the correspondence must be sufficient to enable the iRNA agent, or a cleavage product thereof, to direct sequence specific silencing, *e.g.*, by RNAi cleavage of a Nogo-L or Nogo-R mRNA.

Therefore, the iRNA agents described herein include agents comprising a sense strand and antisense strand each comprising a sequence of at least 16, 17 or 18 nucleotides which is essentially identical, as defined below, to one of the sequences of Table 1 and Table 2 under agent numbers 6000 - 6476 and 6837, except that not more than 1, 2 or 3 nucleotides per strand, respectively, have been substituted by other nucleotides (*e.g.* adenosine replaced by uracil), while essentially retaining the ability to inhibit Nogo-L or Nogo-R expression in cultured human Nogo-L or Nogo-R expressing cells, respectively. These agents will therefore possess at least 15 nucleotides identical to one of the sequences of Table 1 and Table 2 under agent numbers 6000-6476 and 6837, but 1, 2 or 3 base mismatches with respect to either the target Nogo-L or Nogo-R mRNA sequence or between the sense and antisense strand are introduced. Mismatches to the target Nogo-L or Nogo-R mRNA sequence, particularly in the antisense strand, are most tolerated in the terminal regions and if present are preferably in a terminal region or regions, *e.g.*, within 6, 5, 4, or 3 nucleotides of a 5' and/or 3' terminus, most preferably within 6, 5, 4, or 3 nucleotides of the 5'-terminus of the sense strand or the 3'-terminus of the antisense strand. The sense strand need only be sufficiently complementary with the antisense strand to maintain the overall double stranded character of the molecule.

It is preferred that the sense and antisense strands be chosen such that the iRNA agent includes a single strand or unpaired region at one or both ends of the molecule. Thus, an iRNA agent contains sense and antisense strands, preferably paired to contain an overhang, *e.g.*, one or two 5' or 3' overhangs but preferably a 3' overhang of 2-3 nucleotides. Most embodiments will have a 3' overhang. Preferred siRNA agents will have single-stranded overhangs, preferably 3' overhangs, of 1 to 4, or preferably 2 or 3 nucleotides, in length, at one or both ends of the iRNA agent. The overhangs can be the result of one strand being longer than the other, or the result of two strands of the same length being staggered. The unpaired nucleotides forming the overhang can be ribonucleotides, or they can be deoxyribonucleotides, preferably thymidine. 5'-ends are preferably phosphorylated.

Preferred lengths for the duplexed region are between 15 and 30, most preferably 18, 19, 20, 21, 22, and 23 nucleotides in length, *e.g.*, in the siRNA agent range discussed above. siRNA agents can resemble in length and structure the natural Dicer processed products from long dsRNAs. The two strands of the siRNA agent may be linked, *e.g.*, covalently linked. Hairpin, or other single strand structures which provide the required double stranded region, and preferably a 3' overhang are also described herein.

## Evaluation of Candidate iRNA. Agents

A candidate iRNA agent can be evaluated for its ability to downregulate target gene expression. For example, a candidate iRNA agent can be provided, and contacted with a cell, that expresses the target gene, *e.g.*, the Nogo-L or Nogo-R gene, either endogenously or because it has been transected with a construct from which a Nogo-L or Nogo-R protein can be expressed. The level of target gene expression prior to and following contact with the candidate iRNA agent can be compared, *e.g.*, on an mRNA or protein level. If it is determined that the amount of RNA or protein expressed from the target gene is lower following contact with the iRNA agent, then it can be concluded that the iRNA agent downregulates target gene expression. The level of target Nogo-L or Nogo-R RNA or Nogo-L or Nogo-R protein in the cell can be determined by any method desired. For example, the level of target RNA can be determined by Northern blot analysis, reverse transcription coupled with polymerase chain reaction (RT-PCR), or RNAse protection assay. The level of protein can be determined, for example, by Western blot analysis or immuno-fluorescence. Preferably, the assay also tests the ability of the iRNA agent to inhibit Nogo-L or Nogo-R expression on a functional level, e.g. by assessing the ability of the iRNA agent to trigger neuronal growth.

### Stability testing modification and retesting of iRNA agents

A candidate iRNA agent can be evaluated with respect to stability, *e.g.*, its susceptibility to cleavage by an endonuclease or exonuclease, such as when the iRNA agent is introduced into the body of a subject. Methods can be employed to identify sites that are susceptible to modification, particularly cleavage, *e.g.*, cleavage by a component found in the body of a subject.

When sites susceptible to cleavage are identified, a further iRNA agent can be designed and/or synthesized wherein the potential cleavage site is made resistant to cleavage, e.g. by introduction of a 2'-modification on the site of cleavage, *e.g.* a 2'-O-methyl group. This further iRNA agent can be retested for stability, and this process may be iterated until an iRNA agent is found exhibiting the desired stability.

### *In Vivo* Testing

An iRNA agent identified as being capable of inhibiting Nogo-L or Nogo-R gene expression can be tested for functionality *in vivo* in an animal model (*e.g.*, in a mammal, such as in mouse or rat). For example, the iRNA agent can be administered to an animal, and the iRNA agent evaluated with respect to its biodistribution, stability, and its ability to inhibit Nogo-L or Nogo-R gene expression or reduce a biological or pathological process mediated at least in part by Nogo-L or Nogo-R.

The iRNA agent can be administered directly to the target tissue, such as by injection, or the iRNA agent can be administered to the animal model in the same manner that it would be administered to a human.

The iRNA agent can also be evaluated for its intracellular distribution. The evaluation can include determining whether the iRNA agent was taken up into the cell. The evaluation can also include determining the stability (*e.g.*, the half-life) of the iRNA agent. Evaluation of an iRNA agent *in vivo* can be facilitated by use of an iRNA agent conjugated to a traceable marker (*e.g.*, a fluorescent marker such as fluorescein; a radioactive label, such as $^{35}S$, $^{32}P$, $^{33}P$, or $^{3}H$; gold particles; or antigen particles for immunohistochemistry).

The iRNA agent can be evaluated with respect to its ability to down regulate Nogo-L or Nogo-R gene expression. Levels of Nogo-L or Nogo-R gene expression *in vivo* can be measured, for example, by *in situ* hybridization, or by the isolation of RNA from tissue prior to and following exposure to the iRNA agent. Where the animal needs to be sacrificed in order to harvest the tissue, an untreated control animal will serve for comparison. Nogo-L or Nogo-R mRNA can be

detected by any desired method, including but not limited to RT-PCR, Northern blot, branched-DNA assay, or RNAase protection assay. Alternatively, or additionally, Nogo-L or Nogo-R gene expression can be monitored by performing Western blot analysis on tissue extracts treated with the iRNA agent.

Animal models may be used to establish the concentration necessary to achieve a certain desired effect (*e.g.*, EC50). Such animal models may include transgenic animal that express a human gene, *e.g.*, a gene that produces a target human Nogo-L or Nogo-R RNA. The composition for testing may include an iRNA agent that is complementary, at least in an internal region, to a sequence that is conserved between the target Nogo-L or Nogo-R RNA in the animal model and the target Nogo-L or Nogo-R RNA in a human.

## iRNA Chemistry

Described herein are isolated iRNA agents, *e.g.*, ds RNA agents that mediate RNAi to inhibit expression of a Nogo-L or Nogo-R gene.

RNA agents discussed herein include otherwise unmodified RNA as well as RNA which has been modified, *e.g.*, to improve efficacy, and polymers of nucleoside surrogates. Unmodified RNA refers to a molecule in which the components of the nucleic acid, namely sugars, bases, and phosphate moieties, are the same or essentially the same as that which occur in nature, preferably as occur naturally in the human body. The art has referred to rare or unusual, but naturally occurring, RNAs as modified RNAs, see, *e.g.*, Limbach et al. Nucleic Acids Res. 22: 2183-2196, 1994. Such rare or unusual RNAs, often termed modified RNAs (apparently because they are typically the result of a post-transcriptional modification) are within the term unmodified RNA, as used herein. Modified RNA as used herein refers to a molecule in which one or more of the components of the nucleic acid, namely sugars, bases, and phosphate moieties, are different from that which occurs in nature, preferably different from that which occurs in the human body. While they are referred to as modified "RNAs," they will of course, because of the modification, include molecules which are not RNAs. Nucleoside surrogates are molecules in which the ribophosphate backbone is replaced with a non-ribophosphate construct that allows the bases to the presented in the correct spatial relationship such that hybridization is substantially similar to what is seen with a ribophosphate backbone, *e.g.*, non-charged mimics of the ribophosphate backbone. Examples of the above are discussed herein.

Modifications described herein can be incorporated into any double-stranded RNA and RNA-like molecule described herein, *e.g.*, an iRNA agent. It may be desirable to modify one or both of the antisense and sense strands of an iRNA agent. As nucleic acids are polymers of subunits or monomers, many of the modifications described below occur at a position which is repeated within a nucleic acid, *e.g.*, a modification of a base, or a phosphate moiety, or the non-linking O of a phosphate moiety. In some cases the modification will occur at all of the subject positions in the nucleic acid but in many, and in fact in most, cases it will not. By way of example, a modification may only occur at a 3' or 5' terminal position, may only occur in a terminal region, *e.g.* at a position on a terminal nucleotide or in the last 2, 3, 4, 5, or 10 nucleotides of a strand. A modification may occur in a double strand region, a single strand region, or in both. *E.g.*, a phosphorothioate modification at a non-linking O position may only occur at one or both termini, may only occur in a terminal regions, *e.g.*, at a position on a terminal nucleotide or in the last 2, 3, 4, 5, or 10 nucleotides of a strand, or may occur in double strand and single strand regions, particularly at termini. Similarly, a modification may occur on the sense strand, antisense strand, or both. In some cases, the sense and antisense strand will have the same modifications or the same class of modifications, but in other cases the sense and antisense strand will have different modifications, *e.g.*, in some cases it may be desirable to modify only one strand, *e.g.* the sense strand.

Two prime objectives for the introduction of modifications into iRNA agents is their stabilization towards degradation in biological environments and the improvement of pharmacological properties, *e.g.* pharmacodynamic properties, which are further discussed below. Other suitable modifications to a sugar, base, or backbone of an iRNA agent are described in co-owned PCT Application No. WO 2004/064737, filed January 16, 2004. An iRNA agent can include a non-naturally occurring base, such as the bases described in co-owned PCT Application No. WO 2004/094345, filed April 16, 2004. An iRNA agent can include a non-naturally occurring sugar, such as a non-carbohydrate cyclic carrier molecule. Exemplary features of non-naturally occurring sugars for use in iRNA agents are described in co-owned PCT Application No. WO 2004/094345, filed April 16, 2003.

An iRNA agent can include an intemucleotide linkage (*e.g.*, the chiral phosphorothioate linkage) useful for increasing nuclease resistance. In addition, or in the alternative, an iRNA agent can include a ribose mimic for increased nuclease resistance. Exemplary intemucleotide linkages and ribose mimics for increased nuclease resistance are described in co-owned PCT Application No. WO 2004/080406, filed on March 8, 2004.

An iRNA agent can include ligand-conjugated monomer subunits and monomers for oligonucleotide synthesis. Exemplary monomers are described in co-owned U.S. Application 2005/107325, filed on August 10, 2004.

An iRNA agent can have a ZXY structure, such as is described in co-owned PCT Application No. WO 2004/080406, filed on March 8, 2004.

An iRNA agent can be complexed with an amphipathic moiety. Exemplary amphipathic moieties for use with

iRNA agents are described in co-owned PCT Application WO 2004/080406, filed on March 8, 2004.

In another embodiment, the iRNA agent can be complexed to a delivery agent that features a modular complex. The complex can include a carrier agent linked to one or more of (preferably two or more, more preferably all three of): (a) a condensing agent (*e.g.*, an agent capable of attracting, *e.g.*, binding, a nucleic acid, *e.g.*, through ionic or electrostatic interactions); (b) a fusogenic agent (*e.g.*, an agent capable of fusing and/or being transported through a cell membrane); and (c) a targeting group, *e.g.*, a cell or tissue targeting agent, *e.g.*, a lectin, glycoprotein, lipid or protein, *e.g.*, an antibody, that binds to a specified cell type. iRNA agents complexed to a delivery agent are described in co-owned PCT Application No. WO 2004/080406, filed on March 8, 2004.

An iRNA agent can have non-canonical pairings, such as between the sense and antisense sequences of the iRNA duplex. Exemplary features of non-canonical iRNA agents are described in co-owned PCT Application No. WO 2004/080406, filed on March 8, 2004.

Enhanced nuclease resistance

An iRNA agent, *e.g.*, an iRNA agent that targets Nogo-L or Nogo-R, can have enhanced resistance to nucleases. Naked RNA is often an easy prey for nucleolytic enzymes, such as exonucleases and endonucleases, which are omnipresent in biological media, such as the cellular cytoplasm, blood, or cerebrospinal fluid (CSF). Quick degradation can severly hamper the ability of an siRNA to inhibit the expression of a target gene. The vulnerability towards nucleolytic degradation can be greatly reduced by chemically modifying certain nucleotides of an siRNA. However, adding modifications in order to stabilize an siRNA sometimes represents a trade-off with its activity, and stabilizing modifications may even introduce toxic effects. It is therefore desirable to introduce the minimum number of modifications that still imparts the desired level of stability. Modifications in the sense strand usually have less impact on the activity of an siRNA.

In order to increase the stability of an siRNA towards nucleolytic degradation by endonucleases, it is therefore advantageous to modify only a limited number of nucleotides in particularly degradation prone positions, as described in co-owned U.S. Application No. 60/559,917, filed on May 4, 2004, co-owned U.S. Application No. 60/574,744, filed on May 27, 2004, and co-owned international application PCT/US2005/018931, filed May 27, 2005. We have determined that pyrimidine nucleotides, and specifically the 5' nucleotide in a 5'-ua-3' sequence context, a 5'-ug-3' sequence context, a 5'-ca-3' sequence context, a 5'-uu-3' sequence context, or a 5'-cc-3' sequence context are particularly prone to degradative attack, in that approximate order. Sufficiently stable and highly active siRNAs have been obtained by our laboratory when the 5'-most pyrimidines in all occurrences of the sequence contexts 5'-ua-3' and 5'-ca-3', or in all occurrences of 5'-ua-3', 5'-ca-3', and 5'-uu-3', or in all occurrences of 5'-ua-3', 5'-ca-3', 5'-uu-3', and 5'-ug-3' were replaced by 2'-modified nucleotides, such as 2'-*O*-methyl nucleotides, in both strands. Alternatively, 2'-modifying all pyrimidine nucleotides in the sense strand and the 5'-most pyrimidines in all occurrences of the sequence contexts 5'-a-3' and 5'-ca-3' in the antisense strand has given good results in terms of activity and stability. Sometimes, it has been necessary to 2'-modify all pyrimidine nucleotides in the sense strand and the 5'-most pyrimidines in all occurrences of the sequence contexts 5'-ua-3', 5'-ca-3', 5'-uu-3', and 5'-ug-3' in the antisense strand. The iRNA agent can include at least 2, at least 3, at least 4 or at least 5 of such dinucleotides.

Preferably, the 2'-modified nucleotides include, for example, a 2'-modified ribose unit, *e.g.*, the 2'-hydroxyl group (OH) can be modified or replaced with a number of different "oxy" or "deoxy" substituents.

Examples of "oxy"-2' hydroxyl group modifications include alkoxy or aryloxy (OR, *e.g.*, R = H, alkyl, cycloalkyl, aryl, aralkyl, heteroaryl or sugar); polyethyleneglycols (PEG), $O(CH_2CH_2O)_nCH_2CH_2OR$; "locked" nucleic acids (LNA) in which the 2' hydroxyl is connected, *e.g.*, by a methylene bridge, to the 4' carbon of the same ribose sugar; O-AMINE and aminoalkoxy, $O(CH_2)_n$AMINE, (*e.g.*, AMINE = $NH_2$; alkylamino, dialkylamino, heterocyclyl amino, arylamino, diaryl amino, heteroaryl amino, or diheteroaryl amino, ethylene diamine, polyamino). It is noteworthy that oligonucleotides containing only the methoxyethyl group (MOE), ($OCH_2CH_2OCH_3$, a PEG derivative), exhibit nuclease stabilities comparable to those modified with the robust phosphorothioate modification.

"Deoxy" modifications include hydrogen (*i.e.* deoxyribose sugars, which are of particular relevance to the overhang portions of partially ds RNA); halo (*e.g.*, fluoro); amino (*e.g.* $NH_2$; alkylamino, dialkylamino, heterocyclyl, arylamino, diaryl amino, heteroaryl amino, diheteroaryl amino, or amino acid); $NH(CH_2CH_2NH)_nCH_2CH_2$-AMINE (AMINE = $NH_2$; alkylamino, dialkylamino, heterocyclyl amino, arylamino, diaryl amino, heteroaryl amino, or diheteroaryl amino), -NHC(O)R (R = alkyl, cycloalkyl, aryl, aralkyl, heteroaryl or sugar), cyano; mercapto; alkyl-thio-alkyl; thioalkoxy; and alkyl, cycloalkyl, aryl, alkenyl and alkynyl, which may be optionally substituted with *e.g.*, an amino functionality.

Preferred substitutents are 2'-methoxyethyl, 2'-$OCH_3$, 2'-O-allyl, 2'-C- allyl, and 2'-fluoro.

The inclusion of furanose sugars in the oligonucleotide backbone can also decrease endonucleolytic cleavage. An iRNA agent can be further modified by including a 3' cationic group, or by inverting the nucleoside at the 3'-terminus with a 3'-3' linkage. In another alternative, the 3'-terminus can be blocked with an aminoalkyl group, *e.g.*, a 3' C5-aminoalkyl dT. Other 3' conjugates can inhibit 3'-5' exonucleolytic cleavage. While not being bound by theory, a 3' conjugate, such as naproxen or ibuprofen, may inhibit exonucleolytic cleavage by sterically blocking the exonuclease

from binding to the 3'-end of oligonucleotide. Even small alkyl chains, aryl groups, or heterocyclic conjugates or modified sugars (D-ribose, deoxyribose, glucose etc.) can block 3'-5'-exonucleases.

Nucleolytic cleavage can also be inhibited by the introduction of phosphate linker modifications, *e.g.*, phosphorothioate linkages. Thus, preferred iRNA agents include nucleotide dimers enriched or pure for a particular chiral form of a modified phosphate group containing a heteroatom at a nonbridging position normally occupied by oxygen. The heteroatom can be S, Se, $Nr_2$, or $Br_3$. When the heteroatom is S, enriched or chirally pure Sp linkage is preferred. Enriched means at least 70, 80, 90, 95, or 99% of the preferred form. Modified phosphate linkages are particularly efficient in inhibiting exonucleolytic cleavage when introduced near the 5'- or 3'-terminal positions, and preferably the 5'-terminal positions, of an iRNA agent.

5' conjugates can also inhibit 5'-3' exonucleolytic cleavage. While not being bound by theory, a 5' conjugate, such as naproxen or ibuprofen, may inhibit exonucleolytic cleavage by sterically blocking the exonuclease from binding to the 5'-end of oligonucleotide. Even small alkyl chains, aryl groups, or heterocyclic conjugates or modified sugars (D-ribose, deoxyribose, glucose etc.) can block 3'-5'-exonucleases.

An iRNA agent can have increased resistance to nucleases when a duplexed iRNA agent includes a single-stranded nucleotide overhang on at least one end. The nucleotide overhang may include 1 to 4, preferably 2 to 3, unpaired nucleotides. In a preferred embodiment, the unpaired nucleotide of the single-stranded overhang that is directly adjacent to the terminal nucleotide pair contains a purine base, and the terminal nucleotide pair is a G-C pair, or at least two of the last four complementary nucleotide pairs are G-C pairs. Further , the nucleotide overhang may have 1 or 2 unpaired nucleotides, and, for example, nucleotide overhang is 5'-GC-3'. The nucleotide overhang may be on the 3'-end of the antisense strand. The iRNA agent may include the motif 5'-CGC-3' on the 3'-end of the antisense strand, such that a 2-nt overhang 5'-GC-3' is formed.

Thus, an iRNA agent can include modifications so as to inhibit degradation, *e.g.*, by nucleases, *e.g.*, endonucleases or exonucleases, found in the body of a subject These monomers are referred to herein as NRMs, or Nuclease Resistance promoting Monomers, the corresponding modifications as NRM modifications. In many cases these modifications will modulate other properties of the iRNA agent as well, *e.g.*, the ability to interact with a protein, *e.g.*, a transport protein, *e.g.*, serum albumin, or a member of the RISC, or the ability of the first and second sequences to form a duplex with one another or to form a duplex with another sequence, e-g., a target molecule.

One or more different NRM modifications can be introduced into an iRNA agent or into a sequence of an iRNA agent. An NRMmodification can be used more than once in a sequence or in an iRNA agent

NRM modifications include some which can be placed only at the terminus and others which can go at any position. Some NRM modifications can inhibit hybridization so it is preferable to use them only in terminal regions, and preferable to not use them at the cleavage site or in the cleavage region of a sequence which targets a subject sequence or gene, particularly on the antisense strand. They can be used anywhere in a sense strand, provided that sufficient hybridization between the two strands of the ds iRNA agent is maintained. It may be desirable to put the NRM at the cleavage site or in the cleavage region of a sense strand, as it can minimize off-target silencing.

In most cases, NRM modifications will be distributed differently depending on whether they are comprised on a sense or antisense strand. If on an antisense strand, modifications which interfere with or inhibit endonuclease cleavage should not be inserted in the region which is subject to RISC mediated cleavage, *eg.,* the cleavage site or the cleavage region (As described in Elbashir et al., 2001, Genes and Dev. 15: 188, hereby incorporated by reference). Cleavage of the target occurs about in the middle of a 20 or 21 nt antisense strand, or about 10 or 11 nucleotides upstream of the first nucleotide on the target mRNA which is complementary to the antisense strand. As used herein cleavage site refers to the nucleotides on either side of the cleavage site, on the target or on the iRNA agent strand which hybridizes to it. Cleavage region means the nucleotides within 1, 2, or 3 nucleotides of the cleavagee site, in either direction.

Such modification can be introduced into the terminal regions, *e.g.,* at the terminal position or with 2, 3, 4, or 5 positions of the terminus, of a sense or antisense strand.

## Tethered Ligands

The properties of an iRNA agent, including its pharmacological properties, can be influenced and tailored, for example, by the introduction of ligands, *e.g,* tethered ligands.

A wide variety of entities, *e.g.,* ligands, can be tethered to an iRNA agent, *e.g.,* to the carrier of a ligand-conjugated monomer subunit. Examples are described below in the context of a ligand-conjugated monomer subunit but that is only preferred, entities can be coupled at other points to an iRNA agent.

Preferred moieties are ligands, which are coupled, preferably covalently, either directly or indirectly via an intervening tether, to the carrier. The may be ligand attached to the carrier *via* an intervening tether. The ligand or tethered ligand may be present on the ligand-conjugate monomer when the ligand-conjugated monomer is incorporated into the growing strand. Further, the ligand may be incorporated into a "precursor" ligand-conjugated monomer subunit after a "precursor" ligand-conjugated monomer subunit has been incorporated into the growing strand. For example, a monomer

having, *e.g.,* an amino-terminated tether, *e.g.,* TAP-$(CH_2)_nNH_2$ may be incorporated into a growing sense or antisense strand. In a subsequent operation, i.e., after incorporation of the precursor monomer subunit into the strand, a ligand having an electrophilic group, *e.g.,* a pentafluorophenyl ester or aldehyde group, can subsequently be attached to the precursor ligand-conjugated monomer by coupling the electrophilic group of the ligand with the terminal nucleophilic group of the precursor ligand-conjugated monomer subunit tether.

Furthermore, a ligand may alter the distribution, targeting or lifetime of an iRNA agent into which it is incorporated. A ligand may provide an enhanced affinity for a selected target, *e.g.,* molecule, cell or cell type, compartment, *e.g.,* a cellular or organ compartment, tissue, organ or region of the body, as, *e.g.,* compared to a species absent such a ligand.

Preferred ligands can improve transport, hybridization, and specificity properties and may also improve nuclease resistance of the resultant natural or modified oligoribonucleotide, or a polymeric molecule comprising any combination of monomers described herein and/or natural or modified ribonucleotides.

Ligands in general can include therapeutic modifiers, *e.g.,* for enhancing uptake; diagnostic compounds or reporter groups *e.g.,* for monitoring distribution; cross-linking agents; nuclease-resistance conferring moieties; and natural or unusual nucleobases. General examples include lipophilic molecules, lipids, lectins, steroids (*e.g.,* uvaol, hecigenin, diosgenin), terpenes (*e.g.,* triterpenes, *e.g.,* sarsasapogenin, Friedelin, epifriedelanol derivatized lithocholic acid), vitamins, carbohydrates(*e.g.,* a dextran, pullulan, chitin, chitosan, inulin, cyclodextrin or hyaluronic acid), proteins, protein binding agents, integrin targeting molecules, polycationics, peptides, polyamines, and peptide mimics.

The ligand may be a naturally occurring or recombinant or synthetic molecule, such as a synthetic polymer, *e.g.,* a synthetic polyamino acid. Examples of polyamino acids include polylysine (PLL), poly L-aspartic acid, poly L-glutamic acid, styrene-maleic acid anhydride copolymer, poly(L-lactide-co-glycolied) copolymer, divinyl ether-maleic anhydride copolymer, N-(2-hydroxypropyl)methacrylamide copolymer (HMPA), polyethylene glycol (PEG), polyvinyl alcohol (PVA), polyurethane, poly(2-ethylacrylic acid), N-isopropylacrylamide polymers, or polyphosphazine. Example of polyamines include: polyethylenimine, polylysine (PLL), spermine, spermidine, polyamine, pseudopeptide-polyamine, peptidomimetic polyamine, dendrimer polyamine, arginine, amidine, protamine, cationic moieties, *e.g.,* cationic lipid, cationic porphyrin, quaternary salt of a polyamine, or an alpha helical peptide.

Ligands can also include targeting groups, *e.g.,* a cell or tissue targeting agent, *e.g.,* a thyrotropin, melanotropin, surfactant protein A, Mucin carbohydrate, a glycosylated polyaminoacid, transferrin, bisphosphonate, polyglutamate, polyaspartate, or an RGD peptide or RGD peptide mimetic.

Ligands can be proteins, *e.g.,* glycoproteins, lipoproteins, *e.g.* low density lipoprotein (LDL), or albumins, *e.g.* human serum albumin (HSA), or peptides, *e.g.,* molecules having a specific affinity for a co-ligand, or antibodies *e.g.,* an antibody, that binds to a specified cell type such as a cancer cell, endothelial cell, or bone cell. Ligands may also include hormones and hormone receptors. They can also include non-peptidic species, such as cofactors, multivalent lactose, multivalent galactose, N-acetyl-galactosamine, N-acetyl-glucosamine, multivalent mannose, or multivalent fucose. The ligand can be, for example, a lipopolysaccharide, an activator of p38 MAP kinase, or an activator of NF-κB.

The ligand can be a substance, *e.g,* a drug, which can increase the uptake of the iRNA agent into the cell, for example, by disrupting the cell's cytoskeleton, *e.g.,* by disrupting the cell's microtubules, microflaments, and/or intermediate filaments. The drug can be, for example, taxon, vincristine, vinblastine, cytochalasin, nocodazole, japlakinolide, latrunculin A, phalloidin, swinholide A, indanocine, or myoservin.

In one aspect, the ligand is a lipid or lipid-based molecule. Such a lipid or lipid based molecule preferably binds a serum protein, *e.g.,* human serum albumin (HSA). An HSA binding ligand allows for distribution of the conjugate to a target tissue, *e.g.,* liver tissue, including parenchymal cells of the liver. Other molecules that can bind HSA can also be used as ligands. For example, neproxin or aspirin can be used. A lipid or lipid-based ligand can (a) increase resistance to degradation of the conjugate, (b) increase targeting or transport into a target cell or cell membrane, and/or (c) can be used to adjust binding to a serum protein, *e.g.,* HSA.

A lipid based ligand can be used to modulate, *e.g.,* control the binding of the conjugate to a target tissue. For example, a lipid or lipid-based ligand that binds to HSA more strongly will be less likely to be targeted to the kidney and therefore less likely to be cleared from the body. A lipid or lipid-based ligand that binds to HSA less strongly can be used to target the conjugate to the kidney.

The lipid based ligand may bind HSA. Preferably, it binds HSA with a sufficient affinity such that the conjugate will be preferably distributed to non-kidney tissue. However, it is preferred that the affinity not be so strong that the HSA-ligand binding cannot be reversed.

In another aspect, the ligand is a moiety, *e.g.,* a vitamin or nutrient, which is taken up by a target cell, *e.g.,* a proliferating cell. These are particularly useful for treating disorders characterized by unwanted cell proliferation, *e.g.,* of the malignant or non-malignant type, *e.g.,* cancer cells. Exemplary vitamins include vitamin A, E, and K. Other exemplary vitamins include the B vitamins, *e.g.,* folic acid, B12, riboflavin, biotin, pyridoxal or other vitamins or nutrients taken up by cancer cells.

In another aspect, the ligands is a cell-permeation agent, preferably a helical cell-permeation agent Preferably, the agent is amphipathic. An exemplary agent is a peptide such as tat or antennapedia. If the agent is a peptide, it can

be modified, including a peptidylmimetic, Invertomers, non-peptide or pseudo-peptide linkages, and use of D-amino acids. The helical agent is preferably an alpha-helical agent, which preferably has a lipophilic and a lipophobic phase.

5'-Phosphate modifications

iRNA agents may be 5' phosphorylated or include a phosphoryl analog at the 5' prime terminus. 5'-phosphate modifications of the antisense strand include those which are compatible with RISC mediated gene silencing. Suitable modifications include: 5'-monophosphate ((HO)2(O)P-O-5'); 5'-diphosphate ((HO)2(O)P-O-P(HO)(O)-O-5');-5'-triphosphate ((HO)2(O)P-O-(HO)(O)P-O-P(HO)(O)-O-5'); 5'-guanosine cap (7-methylated or non-methylated) (7m-G-O-5'-(HO)(O)P-O-(HO)(O)P-O-P(HO)(O)-O-5'; 5'-adenosine cap (Appp), and any modified or unmodified nucleotide cap structure (N-O-5'-(HO)(O)P-O-(HO)(O)P-O-P(HO)(O)-U-5'; 5'-monothiophosphate (phosphorothioate; (HO)2(S)P-O-5'); 5'-monodithiophosphate (phosphorodithioate; (HO)(HS)(S)P-O-5'), 5'-phosphorothiolate ((HO)2(O)P-S-5'); any additional combination of oxygen/sulfur replaced monophosphate, diphosphate and triphosphates (*e.g.* 5.-alpha-thiotriphosphate, 5'-gamma-thiotriphosphate, etc.), 5'-phosphoramidates (CHO)2(O)P-NH-5', (HO)(NH2)(O)P-O-5'), 5'-alkylphosphonates (R=alkyhethyl, methyl isopropyl, propyl etc., *e.g.* RP(OH)(O)-O-5'-, (OH)2(O)P-5'-CH2-). 5'-alkyletherphosphonates C=alkylether=methoxymethyl (MeOCH2-), ethoxymethy) etc., *e.g.* RP(OH)(O)-O-5'-).

The sense strand can be modified in order to inactivate the sense strand and prevent formation of an active RISC, thereby potentially reducing off-target effects. This can be accomplished by a modification which prevents 5'-phosphorylation of the sense strand, *e.g.,* by modifications with a 5'-O-methyl ribonucleotide (see Nykänen et al., (2001) ATP requirements and small interfering DNA structure in the RNA interference pathway. Cell 107, 309-321.) Other modifications which prevent phosphorylation can also be used, *e.g.,* simply substituting the 5'-OH by H rather than O-Me. Alternatively, a large bulky group may be added to the 5'-phosphate turning it into a phosphodiester linkage.

Transport of iRNA agents into cells

Not wishing to be bound by any theory, the chemical similarity between cholesterol-conjugated iRNA agents and certain constituents of lipoproteins (*e.g.* cholesterol, cholesteryl esters, phospholipids) may lead to the association of iRNA agents with lipoproteins (*e.g.* LDL, HDL) in blood and/or the interaction of the iRNA agent with cellular components having an affinity for cholesterol, *e.g.* components of the cholesterol transport pathway. Lipoproteins as well as their constituents are taken up and processed by cells by various active and passive transport mechanisms, for example, without limitation, endocytosis of LDL-receptor bound LDL, endocytosis of oxidized or otherwise modified LDLs through interaction with Scavenger receptor A, Scavenger receptor B 1-mediated uptake of HDL cholesterol in the liver, pinocytosis, or transport of cholesterol across membranes by ABC (ATP-binding cassette) transporter proteins, *e.g.* ABC-A1, AHC-G1 or ABC-G4. Hence, cholesterol-conjugated iRNA agents could enjoy facilitated uptake by cells possessing such transport mechanisms, *e.g.* cells of the liver. As such, provided herein is evidence and general methods for targeting iRNA agents to cells expressing certain cell surface components, *egg.* receptors, by conjugating a natural ligand for such component (*e.g.* cholesterol) to the iRNA agent, or by conjugating a chemical moiety (*e.g.* cholesterol) to the iRNA agent which associates with or binds to a natural ligand for the component (*e.g.* LDL, HDL).

**Other Embodiments**

An RNA, *e.g.,* an iRNA agent, can be produced in a cell in *vivo, eg.,* from exogenous DNA templates that are delivered into the cell. For example, the DNA templates can be inserted into vectors and used as gene therapy vectors. Gene therapy vectors can be delivered to a subject by, for example, intravenous injection, local administration (U.S. Pat No. 5.328,470), or by stereotactic injection (see, *e.g.,* Chen et al. Proc. Natl. Acad Sci. USA 91:3054-3057, 1994). The pharmaceutical preparation of the gene therapy vector can include the gene therapy vector in an acceptable diluent, or can comprise a slow release matrix in which the gene delivery vehicle is imbedded. The DNA templates, for example, can include two transcription units, one that produces a transcript that includes the top strand of an iRNA agent and one that produces a transcript that includes the bottom strand of an iRNA agent When the templates are transcribed, the iRNA agent is produced, and processed into siRNA agent fragments that mediate gene silencing.

**Formulation**

The iRNA agents described herein can be formulated for administration to a subject

For ease of exposition, the formulations, compositions, and methods in this section are discussed largely with regard to unmodified iRNA agents. It should be understood, however, that these formulations, compositions, and methods can be practiced with other iRNA agents, *e.g.,* modified iRNA agents, and such practice is within the invention.

A formulated iRNA agent composition can assume a variety of states. In some examples, the composition is

at least partially crystalline, uniformly crystalline, and/or anhydrous (*e.g.,* less than 80, 50, 30, 20, or 10% water). In another example, the iRNA agent is in an aqueous phase, *e.g.,* in a solution that includes water.

The aqueous phase or the crystalline compositions can, *e.g.,* be incorporated into a delivery vehicle, *e.g.,* a liposome (particularly for the aqueous phase) or a particle (*e.g.,* a microparticle as can be appropriate for a crystalline composition). Generally, the iRN A agent composition is formulated in a manner that is compatible with the intended method of administration.

An iRNA agent preparation can be formulated in combination with another agent, *eg.,* another therapeutic agent or an agent that stabilizes an iRNA agent, *e.g.,* a protein that complexes with the iRNA agent to form an iRNP. Still other agents include chelators, *e.g.,* EDTA (*e.g.,* to remove divalent cations such as $Mg^{2+}$), salts, RNAse inhibitors (*e.g.,* a broad specificity RNAse inhibitor such as RNAsin) and so forth.

The iRNA agent preparation may include two or more iRNA agent(s), *e.g.,* two or more iRNA agents that can mediate RNAi with respect to the same gene, or different alleles of the gene, or with respect to different genes. Such preparations can include at least three, five, ten, twenty, fifty, or a hundred or more different iRNA agent species. Such iRNA agents can mediate RNAi with respect to a similar number of different genes.

Where the two or more iRNA agents in such preparation target the same gene, they can have target sequences that are non-overlapping and non-adjacent, or the target sequences may be overlapping or adjacent

## Disorders associated with Nogo-L or Nogo-R expression

An iRNA agent that targets Nogo-L or Nogo-R, *e.g.,* an iRNA agent described herein, can be used to treat a subject, *e.g.,* a human having or at risk for developing a disease or disorder associated with Nogo-L or Nogo-R gene expression or treating a subject where a biological process mediated by Nogo-L or Nogo-R is unwanted. Since Nogo-L and Nogo-R participate in inhibiting axonal growth and elongations, the iRNA agents of the present invention are used to reverse this inhibition leading to nerve/axonal growth and elongation. Such a treatment is useful in treating injuries to the nervous system such as spinal cord injury or peripheral nerve death (caused by, e.g., Metastatic cancers of the CNS, *e.g.,* gliomas (such as glioblastomas, astrocytomas, oligodendrogliomas, ependymomas), meningiomas, medulloblastomas, neuroblastomas, choroid plexus papillomas, sarcomas can also be treated by the iRNA agents described herein. Other indications include diseases of the central nervous system, including but not limited to encephalomyelitis, ischemic stroke, Alzheimer's Disease, spongiform encephalopathy, Amyotrophic lateral sclerosis (ALS), spinal muscular atrophy (SMA), multiple sclerosis, transverse myelitis, motor neuron disease, Guillan Barre, Anterior Spinal Artery Syndrome, and schizophrenia.

For example, an iRNA agent that targets Nogo-L or Nogo-R mRNA can be used to treat a subject with a spinal cord injury or a subject having another pathological state which can be ameliorated, at least in part, by nerve growth and elongation. In such a use, an iRNA agent of the present invention is administered preferably locally at the site of nerve damage or the site at which the inhibitory effects of Nogo-L or Nogo-R is desired to be reversed. Administration of the iRNA agent leads to decrease in Nogo-L or Nogo-R protein resulting in reversing Nogo mediated inhibition of axonal elongation and growth.

## Treatment Methods and Routes of Delivery

A composition that includes an iRNA agent, *eg.,* an iRNA agent that targets Nogo-L or Nogo-R, can be delivered to a subject by a variety of routes to achieve either local delivery to the site of action of systemic delivery to the subject Exemplary routes include direct injection to the site of treatment, intrathecal, parenchymal, intravenous, nasal, oral, and ocular delivery. The preferred means of administering the iRNA agents of the present invention is through direct injection or infusion to the site of treatment

An iRNA agent can be incorporated into pharmaceutical compositions suitable for administration: For example, compositions can include one or more species of an iRNA agent and a pharmaceutically acceptable farrier. As used herein the language "pharmaceutically acceptable carrier" is intended to include any and all solvents, dispersion media, coatings, antibacterial and antifungal agents, isotonic and absorption delaying agents, and the like, compatible with pharmaceutical administration. The use of such media and agents for pharmaceutically active substances is well known in the are Except insofar as any conventional media or agent is incompatible with the active compound, use thereof in the compositions is contemplated. Supplementary active compounds can also be incorporated into the compositions.

The pharmaceutical compositions of the present may be administered in a number of ways depending upon whether local or systemic treatment is desired and upon the area to be treated. Administration may be topical (including ophthalmic, intranasal, transdermal), oral or parenteral. Parenteral administration includes intravenous drip, subcutaneous, intraperitoneal or intramuscular injection, or intrathecal or intraventricular administration.

The route of delivery can be dependent on the disorder of the patient. In general, the delivery of the iRNA agents of the present is done to achieve systemic delivery into the subject One preferred means of achieving this is

through enteral administration. The application may be achieved by direct application of the pharmaceutical composition to the site of nerve injury, such as the the site of spinal cord injury. Formulations for parenteral administration may include sterile aqueous solutions which may also contain buffers, diluents and other suitable additives. For intravenous use, the total concentrations of solutes should be controlled to render the preparation isotonic.

Using the small interfering RNA vectors previously described, the description also provides devices, systems, and methods for delivery of small interfering RNA to target locations in the nervous system and or/the brain. The envisioned route of delivery is through the use of implanted, indwelling, intrathecal or intraparenchymal catheters that provide de a means for injecting small volumes of fluid containing the dsRNA described herein directly into local nerves or local brain tissue. The proximal end of these catheters may be connected to an implanted, intrathecal or intracerebral access port surgically affixed to the patient's body or cranium, or to an implanted drug pump located in the patient's torso.

Alternatively, implantable delivery devices, such as an implantable pump may be employed. Examples of the delivery devices within the scope of the invention include the Model 8506 investigational device (by Medtronic, Inc. of Minneapolis, Ninn), which can be implanted subcutaneously in the body or on the cranium, and provides an access port through which therapeutic agents may be delivered to the nerves or brain. Delivery occurs through a stereotactically implanted polyurethane catheter. Two models of catheters that can function with the Model 8506 access port include the Model 8770 ventricular catheter by Medtronic, Inc., for delivery to the intracerebral ventricles, which is disclosed in U.S. Pat. No. 6,093,180, incorporated herein by reference, and the IPA1 catheter by Medtronic, Inc., for delivery to the brain tissue itself (i.e., intraparenchymal delivery), disclosed in U.S. Ser. Nos. 09/540,444 and 09/625,751, which are incorporated herein by reference. The latter catheter has multiple outlets on its distal end to deliver the therapeutic agent to multiple sites along the catheter path. In addition to the aforementioned device, the delivery of the small interfering RNA vectors in accordance with the invention can be accomplished with a wide variety of devices, including but not limited to U.S. Pat Nos. 5,735,814, 5,814,014. and 6,042,579 . Using the teachings provided herein those of skill in the art will recognize that these and other devices and systems may be suitable for delivery of small interfering RNA vectors for the treatment of pain is described herein

the method may further comprise the steps of implanting a pump outside the body or brain, the pump coupled to a proximal end of the catheter, and operating the pump to deliver the predetermined dosage of the at least one small interfering RNA or small interfering RNA vector through the discharge portion of the catheter. Furthermore, the further step of periodically refreshing a supply of the at least one small interfering RNA or small interfering RNA vector to the pump outside said body or brain may be comprised.

Thus also described herein is the delivery of small interfering RNA vectors using an implantable pump and catheter, like that taught in U.S. Pat No. 5,735,814 and 6,042,579, and further using a sensor as part of the infusion system to regulate the amount of small interfering RNA vectors deliverer to the nerves or brain, like that taught in U.S. Pat No. 5,814,014. Other devices and systems can be used in accordance with the method of the invention, for example, the devices and systems disclosed in U.S. Ser. Nos. 09/872,698 (filed Jun 1, 2001) and 09/864,646 (filed May 23, 2001)

Preferably, the outlet of the pump or catheter is placed in close proximity of the desired site of action of the pharmaceutical composition, such as near the site of spinal cord, or other nerve, injury.

Administration can be provided by the subject or by another person, eg., a caregiver. A caregiver can be any entity involved with providing care to the human: for example, a hospital, hospice, doctor's office, outpatient clinic; a healthcare worker such as a doctor, nurse, or other practitioner, or a spouse or guardian, such as a parent. The medication can be provided in measured doses or in a dispenser which delivers a metered dose.

The term "therapeutically effective amount" is the amount present in the composition that is needed to provide the desired level of drug in the subject to be treated to give the anticipated physiological response.

The term "physiologically effective amount" is that amount delivered to a subject to give the desired palliative or curative effect

The term "pharmaceutically acceptable carrier" means that the carrier can be taken into the lungs with no significant adverse toxicological effects on the lungs.

The term "co-admmistration" refers to administering to a subject two or more agents, and in particular two or more iRNA agents. The agents can be contained in a single pharmaceutical composition and be administered at the same time, or the agents can be contained in separate formulation and administered serially to a subject So long as the two agents can be detected in the subject at the same time, the two agents are said to be co-administered. Both Nogo-L and Nogo-R iRNA agents may be co-administered.

The types of pharmaceutical excipients that are useful as carrier include stabilizers such as human serum albumin (HSA), bulking agents such as carbohydrates, amino acids and polypeptides; pH adjusters or buffers; salts such as sodium chloride; and the like. These carriers may be in a crystalline or amorphous form or may be a mixture of the two.

Bulking agents that are particularly valuable include compatible carbohydrates, polypeptides, amino acids or combinations thereof. Suitable carbohydrates include monosaccharides such as galactose, D-mannose, sorbose, and the like; disaccharides, such as lactose, trehalose, and the like; cyclodextrins, such as 2-hydroxypropyl-beta-cyclodextrin;

and polysaccharides, such as raffinose, maltodextrins, dextrans, and the like; alditols, such as mannitol, xylitol, and the like. A preferred group of carbohydrates includes lactose, threhalose, raffinose maltodextrins, and mannitol. Suitable polypeptides include aspartame. Amino acids include alanine and glycine, with glycine being referred.

Suitable pH adjusters or buffers include organic salts prepared from organic acids and bases, such as sodium citrate, sodium ascorbate, and the like; sodium citrate is preferred.

*Dosage* An iRNA agent can be administered at a unit dose less than about 75 mg per kg of bodyweight, or less than about 70, 60, 50, 40, 30, 20, 10, 5, 2, 1, 0.5, 0.1, 0.05, 0.01, 0.005, 0.001, or 0.0005 mg per kg of bodyweight, and less than 200 nmol of iRNA agent (e.g., about 4.4 x 1016 copies) per kg of bodyweight, or less than 1500, 750, 300, 150, 75, 15, 7.5, 1.5, 0.75, 0.15, 0.075, 0.015, 0.0075, 0.0015, 0.000.75, 0.00015 mmol of iRNA agent per kg of bodyweight The unit dose, for example, can be administered by injection (*e.g.*, intravenous or intramuscular, intrathecally, or directly into an organ), an inhaled dose, or a topical application.

Delivery of an iRNA agent directly to an organ (e.g., directly to the liver) can be at a dosage on the order of about 0.00001 mg to about 3 mg per organ, or preferably about 0.0001-0.001 mg per organ, about 0.03- 3.0 mg per organ, about 0.1-3.0 mg per eye or about 0.3-3.0 mg per organ.

The dosage can be an amount effective to treat or prevent a disease or disorder.

The unit dose may be administered less frequently than once a day. *e.g.*, less than every 2, 4, 8 or 30 days. The unit dose may not be administered with a frequency (*e.g.*, not a regular frequency). For example, the unit dose may be administered a single time. Because iRNA agent mediated silencing can persist for several days after administering the iRNA agent composition, in many instances, it is possible to administer the composition with a frequency of less than once per day, or, for some instances, only once for the entire therapeutic regimen.

For example a subject is <u>administered</u> an initial dose, and one or more maintenance doses of an iRNA agent, *e.g.,* a double-stranded iRNA agent, or siRNA agent, (e.g., a precursor, *e.g.*, a larger iRNA agent which can be processed into an siRNA agent, or a DNA which encodes an iRNA agent, *e.g.,* a double-stranded iRNA agent, or siRNA agent, or precursor thereof). The maintenance dose or doses are generally lower than the initial dose, e.g., one-half less of the initial dose. A maintenance regimen can include treating the subject with a dose or doses ranging from 0.01 to 75 mg/kg of body weight per day, e.g., 70, 60, 50, 40, 30, 20, 10, 5, 2, 1, 0.5, 0.1, 0.05, 0.01, 0.005, 0.001, or 0.0005 mg per kg of body weight per day. The maintenance doses are preferably administered no more than once every 5, 10, or 30 days. Further, the treatment, regimen may last for a period of time which will vary depending upon the nature of the particular disease, its severity and the overall condition of the patient The dosage may also be delivered no more than once per day, *e.g.*, no more than once per 24, 36, 48, or more hours, *e.g.*, no more than once every 5 or 8 days. Following treatment, the patient can be monitored for changes in his condition and for alleviation of the symptoms of the disease state. The dosage of the compound may either be increased in the event the patient does not respond significantly to current dosage levels, or the dose may be decreased if an alleviation of the symptoms of the disease state is observed, if the disease state has been ablated, or if undesired side-effects are observed.

The effective dose can be administered in a single dose or in two or more doses, as desired or considered appropriate under the specific circumstances. If desired to facilitate repeated or frequent infusions, implantation of a delivery device, *e.g.*, a pump, semi-permanent stent *(e.g.*, intravenous, intraperitoneal, intracistemal or intracapsular), or reservoir may be advisable.

Following successful treatment, it may be desirable to have the patient undergo maintenance therapy to prevent the recurrence of the disease state, wherein the compound of the invention is administered in maintenance doses, ranging from 0.001 g to 100 g per kg of body weight (see US 6,107,094).

The concentration of the iRNA agent composition is an amount sufficient to be effective in treating or preventing a disorder or to regulate a physiological condition in humans. The concentration or amount of iRNA agent administered will depend on the parameters determined for the agent and the method of administration, *e.g.* nasal, buccal, pulmonary, or topical, such as intrathecal or at the site of nerve injury. For example, topical formulations tend to require much lower concentrations of some ingredients in order to avoid irritation or burning.

Certain factors may influence the dosage required to effectively treat a subject, including but not limited to the severity of the disease or disorder, previous treatments, the general health and/or age of the subject, and other diseases present. It will also be appreciated that the effective dosage of an iRNA agent such as an siRNA used for treatment may increase or decrease over the course of a particular treatment. Changes in dosage may result and become apparent from the results of diagnostic assays. For example, the subject can be monitored after administering an iRNA agent composition. Based on information from the monitoring, an additional amount of the iRNA agent composition can be administered.

Dosing is dependent on severity and responsiveness of the disease condition to be treated, with the course of treatment lasting from several days to several months, or until a cure is effected or a diminution of disease state is achieved. Optimal dosing schedules can be calculated from measurements of drug accumulation in the body of the patient, or of drug accumulation at the site of application when delivering locally, e.g. at the site of nerve inj usry, e.g. at the site of spinal cord injury. Persons of ordinary skill can easily determine optimum dosages, dosing methodologies

and repetition rates. Optimum dosages may vary depending on the relative potency of individual compounds, and can generally be estimated based on EC50s found to be effective in *in vitro* and *in vivo* animal models as described above.

The invention is further illustrated by the following examples, which should not be construed as further limiting.

## EXAMPLES

Nucleic acid sequences are represented below using standard nomenclature, and specifically the abbreviations of Table 3.

Table 3: Abbreviations of nucleotide monomers used in nucleic acid sequence representation. It will be understood that these monomers, when present in an oligonucleotide, are mutually linked by 5'-3'-phosphodiester bonds.

| **Abbreviation[a]** | **Nucleotide(s)** |
|---|---|
| A, a | 2'-deoxy-adenosine-5'-phosphate, adenosine-5'-phosphate |
| C, c | 2'-deoxy-cytidine-5'-phosphate, cytidine-5'-phosphate |
| G, g | 2'-deoxy-guanosine-5'-phosphate, guanosine-5'-phosphate |
| T,t | 2'-deoxy-thymidine-5'-phosphate, thymidine-5'-phosphate |
| U, u | 2'-deoxy-uridine-5'-phosphate, uridine-5'-phosphate |
| N, n | any 2'-deoxy-nucleotide/nucleotide (G, A, C, or T, g, a, c or u) |
| am | 2'-*O*-methyladenosine-5'-phosphate |
| cm | 2'-*O*-methylcytidine-5'-phosphate |
| gm | 2'-*O*-methylguanosine-5'-phosphate |
| tm | 2'-*O*-methyl-thymidine-5'-phosphate |
| um | 2'-*O*-methyhmdine-5'-phosphate |
| ***A, C, G, T, U, a, c, g, t, u*** | bold italic: corresponding nucleoside (i.e. lacking a 5'-monophosphate group) |
| <u>A</u>, <u>C</u>, <u>G</u>, <u>T</u>, <u>U</u>, <u>a</u>, <u>c</u>, <u>g</u>, <u>t</u>, <u>u</u> | underlined: corresponding nucleoside-5'-thiophosphate |
| <u>-Chol</u> | 1-{6-[cholester-3-yloxycarbonylamino]-hexanoyl}-4-hydroxypyrrolidin-3-phosphorothioate diester |
| [a]capital letters represent 2'-deoxyribonucleotides (DNA), lower case letters represent ribonucleotides (RNA) | |

Source of reagents

Where the source of a reagent is not specifically given herein, such reagent may be obtained from any supplier of reagents for molecular biology at a quality/purity standard for application in molecular biology.

Example 1: Selection of sequences

Sequence alignment was performed to identify regions within the sequence of human NOGO (Genbank accession no. NM_020532) mRNA with full homology to the respective sequences in both mouse NOGO (Genbank accession no NM_194054) and rat NOGO (Genbank accession no. NM_031831) mRNA. To the same end, sequence alignment was carried out for human and mouse NOGO receptor mRNA (human NOGO receptor mRNA: Genbank accession no. NM_023004; mouse NOGO Receptor mRNA: Genbank accession no. NM_022982). Within the regions of homology thus identified, all possible contiguous sequences of 19 or 23 nucleotides were examined by further BLAST comparison for potential cross-reactivity of an siRNA comprising such sequence to other mRNA sequences present in humans. Only sequences with 3 or more mismatches to any other human mRNA or genomic sequence were chosen. The resulting set of 19 nt sequences is represented in the sense strand ribonucleotide sequences of the double-overhang

iRNA agents given in Table 1 and Table 2. The resulting set of 23 nt sequences are those sequences fully complementary to the antisense strands of the single-overhang iRNA agents given in Table 1 and Table 2.

In order to maximise the stability of the siRNAs for testing in biological media, particularly towards nucleolytic attack by endo- and exonucleases, the siRNAs were synthesized such that in the sense strands, all cytidine and uridine nucleotides comprise a 2'-*O*-methyl group, and in the antisense strand, all cytidines and uridines appearing in a sequence context of 5'-ca-3' or 5'-ua-3' comprise a 2'-*O*-methyl group, except for AL-DP-5883, AL-DP-5871, AL-DP-5870, AL-DP-5876, and AL-DP-5872, which do not contain any 2'-O-methyl groups. The latter were synthesized to compare the activities of unstabilized to stabilized siRNAs. As is evident from, the underlying base sequences are the same for AL-DP-5883 and AL-DP-5891, for AL-DP-5871 and AL-DP-5874, for AL-DP-5870 and AL-DP-5873, for AL-DP-5862, AL-DP-5876 and AL-DP-5884, and for AL-DP-5872 and AL-DP-5875.

To the same end, phosphorothioate linkages were introduced between 3'-terminal 5'-TT-3'-group thymidines. It has been our experience that the most active exonucleases in serum and other biological media relevant for the *in vivo* activity of siRNAs act by degrading siRNA strands 3'-5'. It has proven advantageous, and often sufficient, to replace the 2 penultimate nucleotides in the antisense strand by 2'-*O*-methyl-5'-phosphorothioate-modified nucleotides (e.g. the nucleotides in positions 21 and 22, counting 5' to 3', of a 23-nucleotide antisense strand); sometimes it is sufficient to modify only the penultimate nucleotide, or to use only 5'-phosphorothioate-modified nucleotides, or both. The sense strand may be protected in a similar fashion, and/or it may be 3'-conjugated to a tethered ligand via a phosphodiester or a phosphorothioate diester.

Example 2: siRNA synthesis

Single-stranded RNAs were produced by solid phase synthesis on a scale of 1 μmole using an Expedite 8909 synthesizer (Applied Biosystems, Applera Deutschland GmbH, Darmstadt, Germany) and controlled pore glass (CPG, 500Å, Glen Research, Sterling VA) as solid support. RNA and RNA containing 2'-O-methyl nucleotides were generated by solid phase synthesis employing the corresponding phosphoramidites and 2'-O-methyl phosphoramidites, respectively (Proligo Biochemie GmbH, Hamburg, Germany). These building blocks were incorporated at selected sites within the sequence of the oligoribonucleotide chain using standard nucleoside phosphoramidite chemistry such as described in Current protocols in nucleic acid chemistry, Beaucage, S.L. et al. (Edrs.), John Wiley & Sons, Inc., New York, NY, USA. Phosphorothioate linkages were introduced by replacement of the iodine oxidizer solution with a solution of the Beaucage reagent (Chruachem Ltd, Glasgow, UK) in acetonitrile (1%). Further ancillary reagents were obtained from Mallinckrodt Baker (Griesheim, Germany).

Deprotection and purification by anion exchange HPLC of the crude oligoribonucleotides were carried out according to established procedures. Yields and concentrations were determined by UV absorption of a solution of the respective RNA at a wavelength of 260 nm using a spectral photometer (DU 640B, Beckman Coulter GmbH, Unterschleißheim, Germany). Double stranded RNA was generated by mixing an equimolar solution of complementary strands in annealing buffer (20 mM sodium phosphate, pH 6.8; 100 mM sodium chloride), heated in a water bath at 85 - 90°C for 3 minutes and cooled to room temperature over a period of 3 - 4 hours. The purified RNA solution was stored at -20 °C until use.

As a result of the synthesis strategy described above, all oligonucleotides synthesized as described above do not comprise a phosphate group on their 5'-most nucleotide. It will be clear to the skilled person that this is an optional embodiment of the present invention, and that oligonucleotides derived from the nucleotide sequences shown in Table 1 and Table 2 can be made and used for purposes of the present invention with or without this feature.

Cholesterol was 3'-conjugated to siRNA as illustrated in FIG. 1. For the synthesis of these 3'-cholesterol-conjugated siRNAs, an appropriately modified solid support was used for RNA synthesis. The modified solid support was prepared as follows:

Diethyl-2-azabutane-1,4-dicarboxylate AA

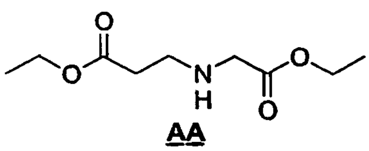

**AA**

A 4.7 M aqueous solution of sodium hydroxide (50 mL) was added into a stirred, ice-cooled solution of ethyl glycinate hydrochloride (32.19 g, 0.23 mole) in water (50 mL). Then, ethyl acrylate (23.1 g, 0.23 mole) was added and

the mixture was stirred at room temperature until the completion of reaction was ascertained by TLC (19 h). After 19 h which it was partitioned with dichloromethane (3 x 100 mL). The organic layer was dried with anhydrous sodium sulfate, filtered and evaporated. The residue was distilled to afford AA (28.8 g, 61%).

3-{Ethoxycarbonylmethyl-[6-(9H-fluoren-9-ylmethoxycarbonyl-amino)-hexanoyl]-amino}-propionic acid ethyl ester AB

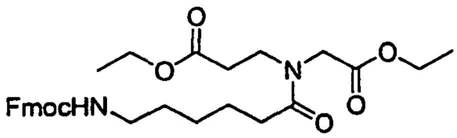

**AB**

Fmoc-6-amino-hexanoic acid (9.12 g, 25.83 mmol) was dissolved in dichloromethane (50 mL) and cooled with ice. Diisopropylcarbodiimde (3.25 g, 3.99 mL, 25.83 mmol) was added to the solution at 0°C. It was then followed by the addition of Diethyl-azabutane-1,4-dicarboxylate (5 g, 24.6 mmol) and dimethylamino pyridine (0.305 g, 2.5 mmol). The solution was brought to room temperature and stirred further for 6 h. the completion of the reaction was ascertained by TLC. The reaction mixture was concentrated in vacuum and to the ethylacetate was added to precipitate diisopropyl urea. The suspension was filtered. The filtrate was washed with 5% aqueous hydrochloric acid, 5% sodium bicarbonate and water. The combined organic layer was dried over sodium sulfate and concentrated to give the crude product which was purified by column chromatography (50 % EtOAC/Hexanes) to yield 11.87 g (88%) of AB.

3-[(6-Amino-hexanoyl)-ethoxycarbonylmethyl-aminol-propionic acid ethyl ester AC

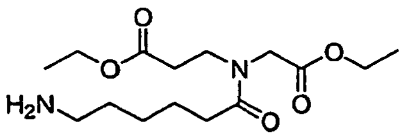

**AC**

3-{Ethoxycarbonylmethyl-[6-(9H-fluoren-9-ylmethoxycarbonylamino)-hexanoyl]-amino}-propionic acid ethyl ester AB (11.5 g, 21.3 mmol) was dissolved in 20% piperidine in dimethylformamide at 0oC. The solution was continued stirring for 1 h. The reaction mixture was concentrated in vacuum and the residue water was added and the product was extracted with ethyl acetate. The crude product was purified by converting into hydrochloride salt.

3-({6-[17-1,5-Dimethyl-hexyl-10,13-dimethyl-2,3,4,7,8,9,10,11,12,13,14,15,16,17-tetradecahydro-1H-cyclopenta[a] phenanthren-3-yloxycarbonylamino]-hexanoyl} ethoxycarbonylmethyl-amino)-propionic acid ethyl ester AD

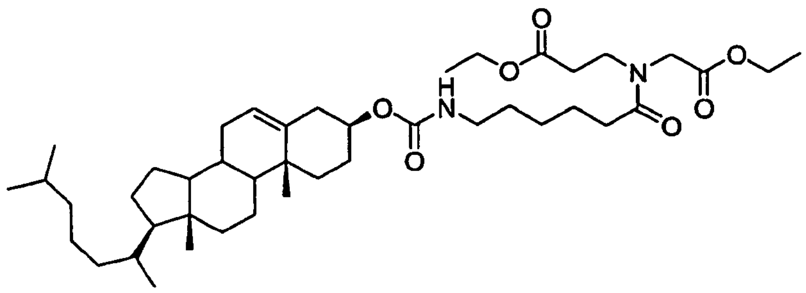

**AD**

The hydrochloride salt of 3-[(6-Amino-hexanoyl)-ethoxycarbonylmethyl-amino]-propionic acid ethyl ester AC (4.7 g, 14.8 mmol) was taken up in dichloromethane. The suspension was cooled to 0°C on ice. To the suspension diisopropylethylamine (3.87 g, 5.2 mL, 30 mmol) was added. To the resulting solution cholesteryl chloroformate (6.675 g, 14.8 mmol) was added. The reaction mixture was stirred overnight. The reaction mixture was diluted with dichloromethane and washed with 10% hydrochloric acid. The product was purified by flash chromatography (10.3 g, 92%).

1-{6-[17-(1,5-Dimethyl-hexyl)-10,13-dimethyl-2,3,4,7,8,9,10,11,12,13,14,15,16,17-tetradecahydro-1H-cyclopenta[a] phenanthren-3-yloxycarbonylamino]-hexanoyl}-4-oxo-pyrrolidine-3-carboxylic acid ethyl ester AE

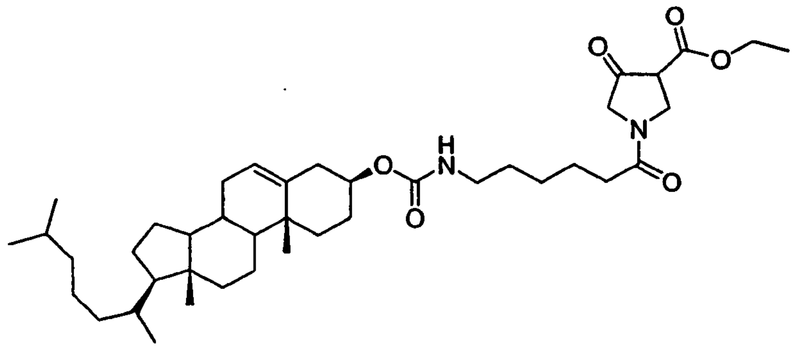

Potassium t-butoxide (1.1 g, 9.8 mmol) was slurried in 30 mL of dry toluene. The mixture was cooled to 0°C on ice and 5 g (6.6 mmol) of diester AD was added slowly with stirring within 20 mins. The temperature was kept below 5°C during the addition. The stirring was continued for 30 mins at 0°C and 1 mL of glacial acetic acid was added, immediately followed by 4 g of $NaH_2PO_4 \cdot H_2O$ in 40 mL of water The resultant mixture was extracted twice with 100 mL of dichloromethane each and the combined organic extracts were washed twice with 10 mL of phosphate buffer each, dried, and evaporated to dryness. The residue was dissolved in 60 mL of toluene, cooled to 0°C and extracted with three 50 mL portions of cold pH 9.5 carbonate buffer. The aqueous extracts were adjusted to pH 3 with phosphoric acid, and extracted with five 40 mL portions of chloroform which were combined, dried and evaporated to a residue. The residue was purified by column chromatography using 25% ethylacetate/hexane to afford 1.9 g of b-ketoester (39%).

[6-(3-Hydroxy-4-hydroxymethyl-pyrrolidin-1-yl)-6-oxo-hexyl]-carbamic acid 17-(1,5-dimethylhexyl)-10,13-dimethyl-2,3,4,7,8,9,10,11,12,13,14,15,16,17-tetradecahydro-1H-cyclopenta[a]phenanthren-3-yl ester AF

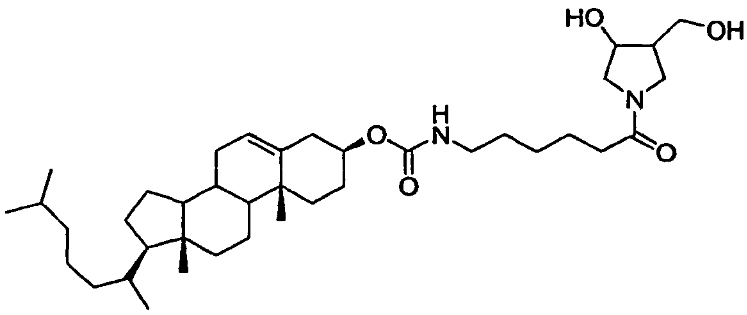

AF

Methanol (2 mL) was added dropwise over a period of 1 h to a refluxing mixture of b-ketoester AE (1.5 g, 2.2 mmol) and sodium borohydride (0.226 g, 6 mmol) in tetrahydrofuran (10 mL). Stirring was continued at reflux temperature for 1 h. After cooling to room temperature, 1 N HCl (12.5 mL) was added, the mixture was extracted with ethylacetate (3 x 40 mL). The combined ethylacetate layer was dried over anhydrous sodium sulfate and concentrated in vacuum to yield the product which was purified by column chromatography (10% MeOH/$CHCl_3$) (89%).

(6-{3-[Bis-(4-methoxy-phenyl)-phenyl-methoxymethyl]-4-hydroxy-pyrrolidin-1-yl}-6-oxohexyl)-carbamic acid 17-(1,5-dimethyl-hexyl)-10,13-dimethyl-2,3,4,7,8,9,10,11,12,13,14,15,16,17-tetradecahydro-1H-cyclopenta[a]phenanthren-3-yl ester AG

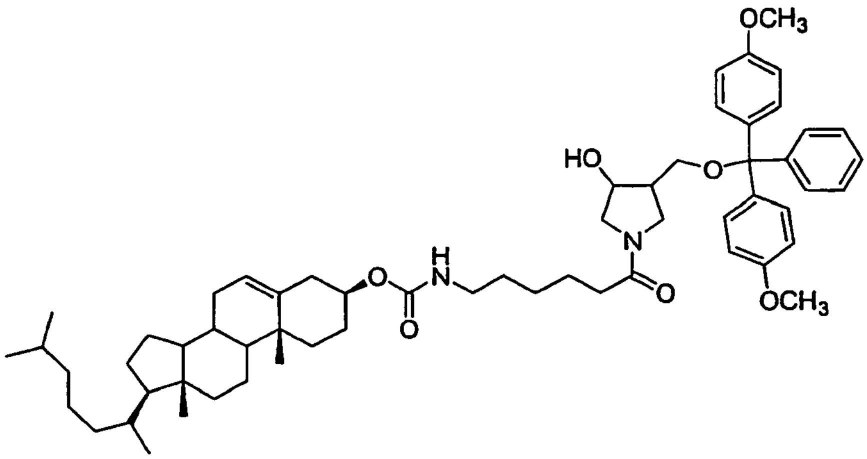

AG

Diol AF (1.25 gm 1.994 mmol) was dried by evaporating with pyridine (2 x 5 mL) in *vacuo.* Anhydrous pyridine (10 mL) and 4,4'-dimethoxytritylchloride (0.724 g, 2.13 mmol) were added with stirring. The reaction was carried out at room temperature overnight. The reaction was quenched by the addition of methanol. The reaction mixture was concentrated in vacuum and to the residue dichloromethane (50 mL) was added. The organic layer was washed with 1M aqueous sodium bicarbonate. The organic layer was dried over anhydrous sodium sulfate, filtered and concentrated. The residual pyridine was removed by evaporating with toluene. The crude product was purified by column chromatography (2% MeOH/Chloroform, Rf = 0.5 in 5% MeOH/$CHCl_3$) (1.75 g, 95%).

Succinic acid mono-(4-[bis-(4-methoxy-phenyl)-phenyl-methoxymethyl]-1- {6-[17-(1,5-dimethyl-hexyl)-10,13-dimethyl 2,3,4,7,8,9,10,11,12,13,14,15,16,17-tetradecahydro-1H cyclopenta[a]phenanthren-3-yloxycarbonylamino]-hexanoyl}-pyrrolidin-3-yl) ester AH

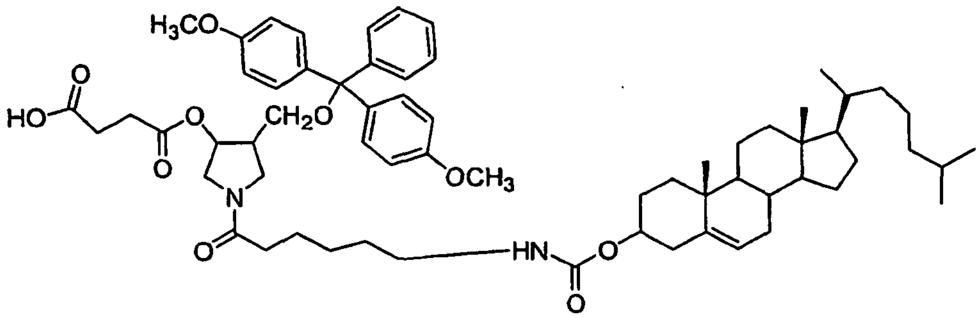

AH

Compound AG (1.0 g, 1.05 mmol) was mixed with succinic anhydride (0.150 g, 1.5 mmol) and DMAP (0.073 g, 0.6 mmol) and dried in a vacuum at 40°C overnight. The mixture was dissolved in anhydrous dichloroethane (3 mL), triethylamine (0.318 g, 0.440 mL, 3.15 mmol) was added and the solution was stirred at room temperature under argon atmosphere for 16 h. It was then diluted with dichloromethane (40 mL) and washed with ice cold aqueous citric acid (5 wt%, 30 mL) and water (2 X 20 mL). The organic phase was dried over anhydrous sodium sulfate and concentrated to dryness. The residue was used as such for the next step.

Cholesterol derivatised CPG AI

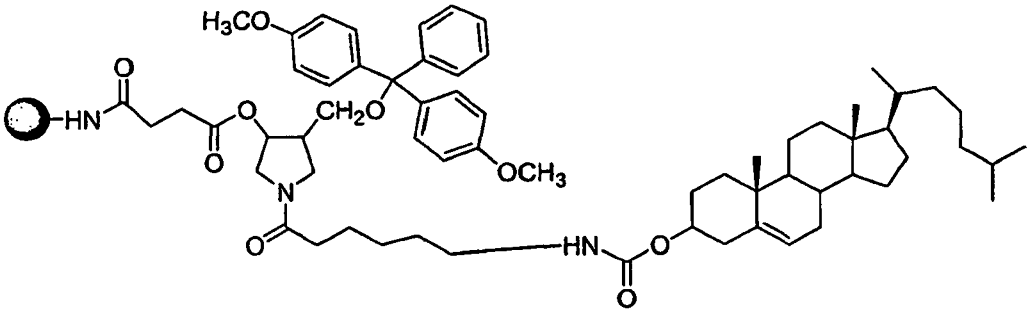

AI

Succinate AH (0.254 g, 0.242 mmol) was dissolved in a mixture of dichloromethane/acetonitrile (3:2, 3 mL). To that solution DMAP (0.0296 g, 0.242 mmol) in acetonitrile (1.25 mL), 2,2'-Dithio-bis(5-nitropyridine) (0.075 g, 0.242 mmol) in acetonitrile/dichloroethane (3:1, 1.25 mL) were added successively. To the resulting solution triphenylphosphine (0.064 g, 0.242 mmol) in acetonitrile (0.6 ml) was added. The reaction mixture turned bright orange in color. The solution was agitated briefly using wrist-action shaker (5 mins). Long chain alkyl amine-CPG (LCAA-CPG) (1.5 g, 61 mm/g) was added The suspension was agitated for 2 h. The CPG was filtered through a sintered funnel and washed with acetonitrile, dichloromethane and ether successively. Unreacted amino groups were masked using acetic anhydride/pyridine. The loading capacity of the CPG was measured by taking UV measurement (37 mM/g).

The synthesis and structure of cholesterol conjugated RNA strands is illustrated in FIG 1.

The siRNAs listed in Table 4 and Table 5 were synthesized for activity screening.

**Table 4: siRNAs specific for Nogo-R**

| Agent number | Sense strand | SEQ ID NO. | Antisense strand | SEQ ID NO. | C. a. #[1] |
|---|---|---|---|---|---|
| AL-DP-5883 | acugccgucugggccaggcTT | 1435 | gccuggcccagacggcaguTT | 1436 | 6029 |
| AL-DP-5891 | acmumgcmcmgumcmumgggcmcmaggcmTT | 1437 | gccuggcccmagacggcmaguTT | 1438 | 6029 |
| AL-DP-5871 | ccacugccgucugggccaggc | 1439 | gccuggcccagacggcaguggcu | 1440 | 6027 |
| AL-DP-5874 | cmcmacmumgcmcmgumcmumgggcmcmaggc | 1441 | gccumggcccmagacggcmagumggcmu | 1442 | 6027 |
| AL-DP-5870 | ggccuggcugcagaaguuccg | 1443 | cggaacuucugcagccaggccca | 1444 | 6021 |
| AL-DP-5873 | ggcmcmumggcmumgcmagaagumumcmcmg | 1445 | cggaacumucumgcmagccmaggccmcma | 1446 | 6021 |
| AL-DP-5862 | umgcmumacmaaumgagcmcmcmaaggTT | 1447 | ccumumgggcucmaumumgumagcmaTT | 1448 | 6000 |
| AL-DP-5876 | ugcuacaaugagcccaaggTT | 1449 | ccuugggcucauuguagcaTT | 1450 | 6000 |
| AL-DP-5884 | umgcmumacmaaumgagcmcmcmaaggTT | 1451 | ccuugggcucmauugumagcmaTT | 1452 | 6000 |
| AL-DP-5872 | ugggccuggcugcagaaguuc | 1453 | gaacuucugcagccaggcccaga | 1454 | 6017 |
| AL-DP-5875 | umgggcmcmumggcmumgcmagaagumumc | 1455 | gaacumucumgcmagccmaggcccaga | 1456 | 6017 |

[1]C. a. # = corresponding agent # in Table 2. The agent given under this agent number in Table 2 possesses the same core nucleotide sequence when nucleotide modifications, e.g. 2'-O-methyl modifications and phosphorothioate linkages, are disregarded

**Table 5: siRNAs specific for Nogo-L**

| Agent number | Sense strand | SEQ ID NO. | Antisense strand | SEQ ID NO. | C. a. #[1] |
|---|---|---|---|---|---|
| AL-DP-5919 | umacmaumumgcmcmumumggcmcmcmumgcmTT | 1457 | gcmagggccmaaggcmaaugumaTT | 1458 | 6276 |
| AL-DP-5920 | cmcmumggaumumgaagcmgcmaaagTT | 1459 | cuuugcgcuucmaauccmaggTT | 1460 | 6418 |
| AL-DP-5921 | aumcmaumumaumcmumaggacmumumgcmTT | 1461 | gcmaaguccumagaumaaugauTT | 1462 | 6378 |
| AL-DP-5922 | aaaumcmcmcmumggaumumgaagcmgTT | 1463 | cgcuucmaauccmagggauuuTT | 1464 | 6408 |
| AL-DP-5923 | umumcmaagumacmcmagumumcmgumgaTT | 1465 | ucmacgaacuggumacuugaaTT | 1466 | 6030 |
| AL-DP-5924 | acmagcmumumumgcmcmcmaumcmaumumumTT | 1467 | aaaugaugggcmaaagcuguTT | 1468 | 6170 |
| AL-DP-5925 | agcmumumumgcmcmcmaumcmaumumumgaTT | 1469 | ucmaaaugaugggcmaaagcuTT | 1470 | 6173 |
| AL-DP-5926 | cmagaagcmumcmcmumumaumaumaumcmTT | 1471 | gaumaumaumaaggagcuucugTT | 1472 | 6174 |
| AL-DP-5927 | umcmaumumaumcmumaggacmumumgcmaTT | 1473 | ugcmaaguccumagaumaaugaTT | 1474 | 6380 |
| AL-DP-5928 | cmumggaumumgaagcmgcmaaagcmTT | 1475 | gcuuugcgcuucmaauccmagTT | 1476 | 6419 |
| AL-DP-5929 | cmumumaumumumagumgaumgaumumcmaTT | 1477 | ugaaucmaucmacumaaaumaagTT | 1478 | 6837 |
| AL-DP-5930 | gumacmaaagaumumgcmumumaumgaTT | 1479 | ucmaumaagcmaaucuuugumacTT | 1480 | 6151 |
| AL-DP-5931 | umacmaaagaumumgcmumumaumgaaTT | 1481 | uucmaumaagcmaaucuuugumaTT | 1482 | 6152 |
| AL-DP-5932 | umcmumgaacmcmagumumgacmumumaumTT | 1483 | aumaagucmaacugguucmagaTT | 1484 | 6187 |
| AL-DP-5933 | acmcmagumumgacmumumaumumumagumTT | 1485 | acumaaaumaagucmaacugguTT | 1486 | 6195 |
| AL-DP-5934 | cmcmagumumgacmumumaumumumagumgTT | 1487 | cmacumaaaumaagucmaacuggTT | 1488 | 6197 |
| AL-DP-5935 | agumumgacmumumaumumumagumgaumTT | 1489 | aucmacumaaaumaagucmaacuTT | 1490 | 6201 |
| AL-DP-5936 | umcmagaumgaaggcmcmacmcmcmaumTT | 1491 | augggugcccuucmaucugaTT | 1492 | 6306 |
| AL-DP-5937 | agaumagaumcmaumumaumcmumaggTT | 1493 | ccumagaumaaugaucumaucuTT | 1494 | 6367 |
| AL-DP-5938 | gaumagaumcmaumumaumcmumaggaTT | 1495 | uccumagaumaaugaucumaucTT | 1496 | 6368 |
| AL-DP-5939 | aaumcmcmcmumggaumumgaagcmgcmTT | 1497 | gcgcuucmaauccmagggauuTT | 1498 | 6410 |
| AL-DP-5940 | umcmcmcmumggaumumgaagcmgcmaaTT | 1499 | uugcgcuucmaauccmagggaTT | 1500 | 6414 |
| AL-DP-5941 | ggumcmaagagsaumumumcmcmcmaumTT | 1501 | augggaaauccucuugaccTT | 1502 | 6127 |
| AL-DP-5942 | cmcmacmcmcmaumumcmagggcmaumaumTT | 1503 | aumaugcccugaaugggugg TT | 1504 | 6327 |
| AL-DP-5943 | umumgaumgaumumumagumumgaumumcmTT | 1505 | gaaucmaacumaaaucmaucmaaTT | 1506 | 6353 |
| AL-DP-5944 | cmumcmagumggaumgagacmcmcmumumTT | 1507 | aagggucucmauccmacugagTT | 1508 | 6057 |
| AL-DP-5945 | cmumgaacmcmagumumgacmumumaumumTT | 1509 | aaumaagucmaacugguucmagTT | 1510 | 6188 |
| AL-DP-5946 | gumaaumumcmumgcmumcmumumggumcmaTT | 1511 | ugaccmaagagcmagaauumacTT | 1512 | 6329 |
| AL-DP-5947 | cmagumumgacmumumaumumumagumgaTT | 1513 | ucmacumaaaumaagucmaacugTT | 1514 | 6199 |
| AL-DP-5948 | aaagaumumgcmumumaumgaaacmaTT | 1515 | uguuucmaumaagcmaaucuuuTT | 1516 | 6158 |
| AL-DP-5949 | ggumacmaaagaumumgcmumumaumgTT | 1517 | cmaumaagcmaaucuuugumaccTT | 1518 | 6150 |
| AL-DP-5950 | cmaumumaumcmumaggacmumumgcmaaTT | 1519 | uugcmaaguccumagaumaaugTT | 1520 | 6382 |
| AL-DP-5951 | cmagaumgaaggcmcmacmcmcmaumumTT | 1521 | aaugggugcccuucmaucugTT | 1522 | 6308 |

(continued)

| Agent number | Sense strand | SEQ ID NO. | Antisense strand | SEQ ID NO. | C. a. #[1] |
|---|---|---|---|---|---|
| AL-DP-5952 | umumgacmumumaumumumagumgaumgaTT | 1523 | ucmaucmacumaaaumaagucmaaTT | 1524 | 6205 |
| AL-DP-5953 | cmumumagumumgaumgaumumumagumumTT | 1525 | aacumaaaucmaucmaacumaagTT | 1526 | 6344 |
| AL-DP-5954 | gaacmcmagumumgacmumumaumumumaTT | 1527 | umaaaumaagucmaacugguucTT | 1528 | 6191 |
| AL-DP-5955 | gacmumumaumumumagumgaumgaumumTT | 1529 | aaucmaucmacumaaaumaagucTT | 1530 | 6209 |
| AL-DP-5956 | gcmumaumcmcmagaaaumcmagaumgTT | 1531 | cmaucugauuucuggaumagcTT | 1532 | 6284 |
| AL-DP-5957 | agaumumgcmumumaumgaaacmaaaTT | 1533 | uuuguuucmaumaagcmaaucuTT | 1534 | 6162 |
| AL-DP-5958 | cmagcmumumumgcmcmcmaumcmaumumumgTT | 1535 | cmaaaugaugggcmaaagcugTT | 1536 | 6172 |
| AL-DP-5959 | cmaaagaumumgcmumumaumgaaacmTT | 1537 | guuucmaumaagcmaaucuuugTT | 1538 | 6156 |
| AL-DP-5960 | cmagaumagaumcmaumumaumcmumagTT | 1539 | cumagaumaaugaucumaucugTT | 1540 | 6366 |
| AL-DP-5961 | ggaumumumcmcmaumcmumgumcmcmumgTT | 1541 | cmaggacmagaugggaaauccTT | 1542 | 6142 |
| AL-DP-5962 | umagumumgaumgaumumumagumumgaTT | 1543 | ucmaacumaaaucmaucmaacumaTT | 1544 | 6348 |
| AL-DP-5963 | umaaumumcmumgcmumcmumumggumcmaumTT | 1545 | augaccmaagagcmagaauumaTT | 1546 | 6330 |
| AL-DP-5964 | gumumgacmumumaumumumagumgaugTT | 1547 | cmaucmacumaaaumaagucmaacTT | 1548 | 6203 |
| AL-DP-5965 | aagacmumggagumggumgumumumgTT | 1549 | cmaaacmaccmacuccmagucuuTT | 1550 | 6256 |
| AL-DP-5966 | umgaacmcmagumumgacmumumaumumumTT | 1551 | aaaumaagucmaacugguucmaTT | 1552 | 6189 |

[1] C. a. # = corresponding agent # in Table 2. The agent given under this agent number in Table 2 possesses the same core nucleotide sequence when nucleotide modifications, e.g. 2'-*O*-methyl modifications and phosphorothioate linkages, are disregarded

Example 3: siRNA activity testing

The ability of the iRNA agents represented in Table 4 and Table 5 to inhibit the expression of human Nogo-L or Nogo-R was tested in human cell lines expressing the respective gene product from an expression construct, or in cell lines constitutively expressing the respective gene product. The iRNA agent is transfected into the cells, e.g., by transfection or electroporation, allowed to act on the cells for a certain time, e.g., 24 hours, and levels of Nogo-R or Nogo-L expression were determined by measurement of Nogo-L or Nogo-R mRNA concentrations in cell lysates. These expression levels were then compared to expression levels of Nogo-L or Nogo-R in cells treated equivalently but without addition of the iRNA agent, or to expression levels of housekeeping genes (e.g. GAPDH), and the ability of the iRNA agents representend in Table 4 and Table 5 to inhibit the expression of human Nogo-L or Nogo-R thereby assessed.

Screening for inhibition of Nogo-L expression

One day before transfection, Neuroscreen-1 cells (Cellomics Inc., Pittsburgh, USA) were seeded at 1.5 x $10^4$ cells / well on 96-well collagen-coated plates (Greiner Bio-One GmbH, Frickenhausen, Germany) in 100 μl of growth medium (RPMI 1640, 10% horse serum, 5% fetal calf serum, 100u penicillin / 100 μg/ml streptomycin, 2 mM L-glutamine, Biochrom AG, Berlin, Germany). Transfections were performed in triplicates. For each well 0.5 μl Lipofectamine2000 (Invitrogen GmbH, Karlsruhe, Germany) were mixed with 12 μl Opti-MEM (Invitrogen) and incubated for 15 min at room temperature. 2 μl of a 5 μM solution of siRNA in annealing buffer (20 mM sodium phosphate, pH 6.8; 100 mM sodium chloride) were mixed with 10.5 μl Opti-MEM per well, combined with the Lipofectamine2000-Opti-MEM mixture and again incubated for 15 minutes at room temperature. During this incubation, growth medium was removed from cells and replaced by 75 μl / well of fresh medium. The 25 μl solution of siRNA-Lipofectamine2000-complex were added, resulting in an overall 100 nM siRNA concentration in the 100 μl incubation volume, and the cells were incubated for 24 h at 37°C and 5 % $CO_2$ in a humidified incubator (Heraeus GmbH, Hanau).

mRNA levels in cell lysates were quantitated by a commercially available branched DNA hybridization assay (QuantiGene bDNA-kit, Genospectra, Fremont, USA). Cells were harvested by applying 50 μl additional growth medium and 75 μl of Lysis Mixture (from QuantiGene bDNA-kit) to each well and were lysed at 53°C for 30 min. 50 μl of the lysates were incubated with probes specific to rNogoA, rNogoABC and rGAPDH (sequence of probes see Table 6, Table 7, and Table 8) according to the manufacturer's protocol for the QuantiGene bDNA kit assay. Finally, chemoluminescence was measured in a Victor2-Light (Perkin Elmer, Wiesbaden, Germany) as RLUs (relative light units) and values obtained with NogoA and NogoABC probes, respectively, were normalized to the respective GAPDH values for each well. Mock transfected cells (following the same protocol except that no siRNA was added) served as controls and forcomparison of mRNA levels.

Effective siRNAs from the screen were further characterized by establishment of dose response curves and calculation of $IC_{50}$ concentrations (the concentration at which 50 % inhibition of gene expression would be observed). For dose response assessment, transfections were performed at the following concentrations: 100 nM, 33.3 nM, 11.1 nM, 3.7 nM, 1.2 nM, 0.4 nM, 137 pM, 46 pM, 15 pM, 5 pM and mock (no siRNA) by serially diluting the 5 μM siRNA stock solution with annealing buffer and using 2 μl of the diluted stock according to the above protocol. The $IC_{50}$ was determined by curve fitting using the computer software Xlfit using the following parameters: Dose Response One Site, 4 Parameter Logistic Model, fit = (A+((B-A)/(1+(((10^C)/x)^D)))), inv = ((10^C)/((((B-A)/(y-A))-1)^(1/D))), res = (y-fit).

Table 6: Rat NogoA-specific probes

| Probe type[1] | Nucleotide sequence | SEQ ID NO. |
|---|---|---|
| CE | CATTCATGGCTTCTTCATATGGTTTTTTCTCTTGGAAAGAAAGT | 1553 |
| CE | TGTTCCCAAAGCTTTTAGTGCTATTTTTCTCTTGGAAAGAAAGT | 1554 |
| CE | TCAGGCTCTTTTATTCCTTCCTTTTTTTCTCTTGGAAAGAAAGT | 1555 |
| CE | CAATGGATATATAAGGAGCTTCTGTTTTTTTTCTCTTGGAAAGAAAGT | 1556 |
| CE | TTTGCTATTTCTGAATAATTAGAGAAATTTTTTCTCTTGGAAAGAAAGT | 1557 |
| LE | CCTGAACAGCTGCATTAAAACTTTTTTTAGGCATAGGACCCGTGTCT | 1558 |
| LE | GGGCACCGACTTCTCGAATTTTTTAGGCATAGGACCCGTGTCT | 1559 |
| LE | CCTCCACTAGCTCAGCGTGTTCTTTTTAGGCATAGGACCCGTGTCT | 1560 |
| LE | AATCGAATCATCACTAAATAAGTCAACTTTTTAGGCATAGGACCCGTGTCT | 1561 |
| LE | CTTGTGTTTGTGGGACTTCAGGTTTTTAGGCATAGGACCCGTGTCT | 1562 |
| BL | GCTTTGTTTCTTTAATTAAATCACACG | 1563 |
| BL | CTGGACTTGGCTCAGTGGAGA | 1564 |

(continued)

| Probe type[1] | Nucleotide sequence | SEQ ID NO. |
|---|---|---|
| BL | TGGTTCAGATTCAGGTGAGGAAT | 1565 |

[1]CE = Capture Extender probe; LE = Label Extender probe; BL= blocking probe

Table 7: Rat NogoABC probes

| Probe type[1] | Nucleotide sequence | SEQ ID NO. |
|---|---|---|
| CE | GCCCTTATATATCCTAAAGCTGATAGTCTTTTTCTCTTGGAAAGAAAGT | 1566 |
| CE | CCTCAGTTCTTTTATTGTGCTGTTCTTTTTCTCTTGGAAAGAAAGT | 1567 |
| CE | CGTTCATAAATAACAGGAATACTGAAGAGTTTTTCTCTTGGAAAGAAAGT | 1568 |
| CE | TAATGATCTATCTGCACCTGATGCTTTTTCTCTTGGAAAGAAAGT | 1569 |
| CE | TCCAGGGATTTTTGCTTGGATTTTTCTCTTGGAAAGAAAGT | 1570 |
| LE | ACCGAGAGCAGGGCCAAGTTTTTAGGCATAGGACCCGTGTCT | 1571 |
| LE | TGAATGGGTGGCCTTCATCTTTTTTAGGCATAGGACCCGTGTCT | 1572 |
| LE | GCAACTTCAGATTCTAAATATGCCCTTTTTAGGCATAGGACCCGTGTCT | 1573 |
| LE | CTGCAAACTTCAGGGAATCAACTTTTTAGGCATAGGACCCGTGTCT | 1574 |
| LE | AGACCATTGAACAAGGCACCATTTTTAGGCATAGGACCCGTGTCT | 1575 |
| BL | GATTTCTGGATAGCCTGGATCAC | 1576 |
| BL | ATTTCTGAACCAATTCCTCTGATATA | 1577 |
| BL | ACATGACCAAGAGCAGAATTACTGT | 1578 |
| BL | TAAATCATCAACTAAGAAAAGCCG | 1579 |
| BL | ACATAAGTAAACACCCACATCAACA | 1580 |
| BL | TGAGATCAGAGCTAAAATCAGTAGTGTC | 1581 |
| BL | AACACTCTTGTTTGCAAGTCCTAGA | 1582 |
| BL | TTTTGGCCATGGCATCCTT | 1583 |

[1]CE = Capture Extender probe; LE = Label Extender probe; BL = blocking probe

Table 8: Rat GAPDH probes

| Probe type[1] | Nucleotide sequence | SEQ ID NO. |
|---|---|---|
| CE | CCAGCTTCCCATTCTCAGCCTTTTTCTCTTGGAAAGAAAGT | 1584 |
| CE | TCTCGCTCCTGGAAGATGGTTTTTTCTCTTGGAAAGAAAGT | 1585 |
| CE | CCCATTTGATGTTAGCGGGATTTTTCTCTTGGAAAGAAAGT | 1586 |
| CE | CGGAGATGATGACCCTTTTGGTTTTTCTCTTGGAAAGAAAGT | 1587 |
| LE | GATGGGTTTCCCGTTGATGATTTTTAGGCATAGGACCCGTGTCT | 1588 |
| LE | GACATACTCAGCACCAGCATCACTTTTTAGGCATAGGACCCGTGTCT | 1589 |
| LE | CCCAGCCTTCTCCATGGTGGTTTTTAGGCATAGGACCCGTGTCT | 1590 |
| BL | TTGACTGTGCCGTTGAACTTG | 1591 |
| BL | CCCCACCCTTCAGGTGAGC | 1592 |
| BL | GGCATCAGCGGAAGGGG | 1593 |

[1]CE = Capture Extender probe; LE = Label Extender probe; BL = blocking probe

Table 9 lists the agent number, the position of the nucleotide within the human Nogo-L mRNA sequence (Genebank accession number NM_020532) corresponding to the 5'-most nucleotide of the sense strand of the agent, the amount of total Nogo-L mRNA (i.e., including Nogo-A, Nogo-B, and Nogo-C mRNA) remaining in cells treated with the agent at 100 nM concentration in % of controls, and the amount of Nogo-A mRNA remaining in cells treated with the agent at 100 nM concentration in % of controls. For selected agents with □articular activity in reducing the amount of total Nogo-L mRNA and/or Nogo-A mRNA, $IC_{50}$ values were also obtained and are given in Table 9. The rightmost column of Table 9 lists whether the agent is specific for Nogo-A. Only Nogo-A mRNA comprises positions 769-3177 of Genebank accession number NM_020532, Nogo-B and Nogo-C mRNA lack these sequences. As evident from the relative activity in reducing total Nogo-L mRNA vs. Nogo-A mRNA, and the corresponding $IC_{50}$ values, agents that are

not specific for Nogo-A mRNA show comparable activity towards reducing total Nogo-L mRNA and Nogo-A mRNA, while those specific for Nogo-A are mostly considerably more active towards reducing Nogo-A mRNA compared to total Nogo-L mRNA.

Table 9: Ability of siRNAs specific for Nogo-L to reduce Nogo-L and/or NogoA mRNA levels in cultured cells

| Agent number | Pos. in mRNA | Rem. mRNA Nogo ABC[1] | Rem mRNA Nogo A[1] | $IC_{50}$ Nogo ABC [nM] [2] | $IC_{50}$ Nogo A [nM] [2] | Specific for Nogo A? |
|---|---|---|---|---|---|---|
| AL-DP-5919 | 3422 | 94 ± 4 % | 86 ± 5 % | | | no |
| AL-DP-5920 | 3851 | 55 ± 4 % | 54 ± 4 % | | | no |
| AL-DP-5921 | 3786 | 43 ± 1 % | 50 ± 2 % | | | no |
| AL-DP-5922 | 3846 | 84 ± 7 % | 59 ± 5 % | | | no |
| AL-DP-5923 | 365 | 63 ± 3 % | 71 ± 2 % | | | no |
| AL-DP-5924 | 2080 | 79 ± 5 % | 29 ± 2 % | n.d. | 0,3 | yes |
| AL-DP-5925 | 2082 | 80 ± 6 % | 38 ± 2 % | | | yes |
| AL-DP-5926 | 2367 | 81 ± 3 % | 23 ± 3 % | | | yes |
| AL-DP-5927 | 3787 | 33 ± 4 % | 50 ± 13 % | 77 | 81 | no |
| AL-DP-5928 | 3852 | 35 ± 4 % | 40 ± 5 % | 80 | 77 | no |
| AL-DP-5929 | 2536 | 110 ± 13 % | 30 ± 2 % | n.d | 0,086 | yes |
| AL-DP-5930 | 2001 | 95 ± 6 % | 27 ± 4 % | | | yes |
| AL-DP-5931 | 2002 | 71 ± 4% | 29 ± 4 % | 140 | 1,45 | yes |
| AL-DP-5932 | 2522 | 70 ± 3% | 35 ± 3 % | n.d. | 26 | yes |
| AL-DP-5933 | 2527 | 97 ± 3 % | 42 ± 4 % | | | yes |
| AL-DP-5934 | 2528 | 80 ± 5 % | 25 ± 3 % | n.d. | 0,26 | yes |
| AL-DP-5935 | 2530 | 98 ± 6 % | 38 ± 3 % | | | yes |
| AL-DP-5936 | 3497 | 34 ± 1 % | 42 ± 2% | 0,6 | 56 | no |
| AL-DP-5937 | 3780 | 75 ± 3 % | 78 ± 5 % | | | no |
| AL-DP-5938 | 3781 | 16 ± 1 % | 32 ± 3 % | 0,4 | 1,4 | no |
| AL-DP-5939 | 3847 | 48 ± 5 % | 47 ± 5 % | | | no |
| AL-DP-5941 | 956 | 122 ± 20 % | 34 ± 5 % | n.d. | 0,34 | yes |
| AL-DP-5942 | 3508 | 94 ± 13 % | 65 ± 14 % | | | no |
| AL-DP-5943 | 3633 | 82 ± 23 % | 74 ± 15 % | | | no |
| AL-DP-5944 | 847 | 131 ± 32 % | 48 ± 7% | | | yes |
| AL-DP-5945 | 2523 | 106 ± 18% | 49 ± 9 % | | | yes |
| AL-DP-5946 | 3573 | 39 ± 4% | 38 ± 4 % | 0,15 | 0,65 | no |
| AL-DP-5947 | 2529 | 71 ± 4% | 36 ± 4 % | 123 | 5,9 | yes |
| AL-DP-5948 | 2005 | 82 ± 16% | 25 ± 5 % | n.d. | 0,31 | yes |
| AL-DP-5949 | 2000 | 87 ± 7% | 33 ±4% | n.d. | 0,96 | yes |
| AL-DP-5950 | 3788 | 30 ± 4% | 32 ± 3 % | 0,14 | 1,63 | no |
| AL-DP-5951 | 3498 | 69 ± 11% | 74 ± 9 % | | | no |
| AL-DP-5952 | 2532 | 89 ± 10% | 49 ± 17% | | | yes |
| AL-DP-5953 | 3628 | 29 ± 3% | 51 ± 10% | 3,5 | n.d. | no |
| AL-DP-5954 | 2525 | 82 ± 8% | 47 ± 10% | | | yes |
| AL-DP-5955 | 2534 | 96 ± 21 % | 66 ± 19 % | | | yes |
| AL-DP-5956 | 3485 | 30 ± 3% | 35 ± 4% | | | no |
| AL-DP-5957 | 2007 | 99 ± 15% | 67 ± 20% | | | yes |
| AL-DP-5958 | 2081 | 83 ± 5 % | 30 ± 4% | n.d. | 0,98 | yes |
| AL-DP-5959 | 2004 | 60 ± 11% | 34 ± 11% | 27 | 0,33 | yes |
| AL-DP-5960 | 3779 | 46 ± 1 % | 56 ± 4 % | | | no |
| AL-DP-5961 | 964 | 68 ± 13 % | 84 ± 25% | | | yes |
| AL-DP-5962 | 3630 | 92 ± 7% | 88 ± 2% | | | no |
| AL-DP-5963 | 3574 | 24 ± 6 % | 34 ± 10 % | 0,002 | 0,027 | no |
| AL-DP-5964 | 2531 | 42 ± 4% | 56 ± 14 % | | | yes |
| AL-DP-5965 | 3344 | 57 ± 5% | 76 ± 16% | | | no |

(continued)

| Agent number | Pos. in mRNA | Rem. mRNA Nogo ABC[1] | Rem mRNA Nogo A[1] | $IC_{50}$ Nogo ABC [nM] [2] | $IC_{50}$ Nogo A [nM] [2] | Specific for Nogo A? |
|---|---|---|---|---|---|---|
| AL-DP-5966 | 2524 | 79 ± 5 % | 47 ± 7% | | | yes |

[1] incubation at 100 nM concentration of agent

[2] n.d. means that no $IC_{50}$ value could be obtained since no dependence of the Nogo-L mRNA-reducing activity of the agent on its concentration was observable

In summary, AL-DP-5938 reduced total Nogo-L mRNA by 80% or more, AL-DP-5964, AL-DP-5954, AL-DP-5951, and AL-DP-5957 reduced total Nogo-L mRNA by 70% or more, AL-DP-5927, AL-DP-5936, AL-DP-5928, and AL-DP-5947 reduced total Nogo-L mRNA by 60% or more, AL-DP-5965, AL-DP-5921, AL-DP-5961, and AL-DP-5939 reduced total Nogo-L mRNA by 50% or more, and AL-DP-5920, AL-DP-5966, and AL-DP-5960 reduced total Nogo-L mRNA by 40% or more. Conversely, AL-DP-5926, AL-DP-5934, AL-DP-5949, AL-DP-5930, AL-DP-5931, AL-DP-5924, AL-DP-5959, and AL-DP-5929 reduced Nogo-A mRNA by 70% or more, AL-DP-5938, AL-DP-5951, AL-DP-5950, AL-DP-5964, AL-DP-5960, AL-DP-5942, AL-DP-5957, AL-DP-5932, AL-DP-5948, AL-DP-5947, AL-DP-5925, and AL-DP-5935 reduced Nogo-A mRNA by 60% or more, AL-DP-5928, AL-DP-5936, AL-DP-5933, AL-DP-5939, AL-DP-5955, AL-DP-5945, AL-DP-5953, AL-DP-5946, AL-DP-5927, and AL-DP-5921 reduced Nogo-A mRNA by 50% or more, and AL-DP-5954, AL-DP-5920, AL-DP-5965, AL-DP-5961, and AL-DP-5922 reduced Nogo-A mRNA by 40% or more.

Screening for inhibition of Nogo-R expression

Cloning of rat Nogo-receptor:

Total RNA was extracted from rat brain with Rneasy Mini Kit (Qiagen, Hilden, Germany) according to the manufacturer's protocol. An aliquot of this RNA was reversibly transcribed with Super-Script First Strand Synthesis System for RT-PCR (Invitrogen GmbH, Karlsruhe, Germany) according to the manufacturer's protocol using random-hexamers as primers. 20 ng of the obtained cDNA was used for amplifying Nogo-receptor with two specific primers (rNogoR-sense: 5'-CCACCATGAAGAGGGCGTCCT-3' (SEQ ID NO:1625); rNogoR-antisense: 5'-TCAGCAGGGCCCAAGCACTGT-3' (SEQ ID NO:1626) employing the following for each 50 µl reaction: 4 u Power-Script short (PAN Biotech GmbH, Aidenbach, Germany), 2 mM dNTPs, 1x Opti-Buffer, 1x Hi-Spec additive, 5 mM $MgCl_2$ and 1 µM of each primer (all from PAN Biotech GmbH). After 30 PCR-cycles with denaturing at 94°C, annealing at 50°C and primer extension at 68°C, PCR-probes were loaded on a 0.8% agarose gel and the band of the right size for rat Nogo receptor (1422 basepairs) was eluted with Qiaquick Gel Extraction Kit (Qiagen, Hilden, Germany) according to the manufacturer's protocol. 4 µl of the eluate were ligated with pEF6N5-His TOPO TA Expression Kit (Invitrogen) according to the kit's protocol and transformed into E. coli DH5-alpha (content of the cloning kit), the plasmid was extracted with Pure Link Hipure Plasmid kit (Invitrogen) according to the kit's protocol and analyzed by custom sequencing (GATC Biotech AG, Konstanz, Germany). As compared to the sequence published for rat Nogo-receptor (Genebank accession no. NM_ 053613, designated "Query" below) there are several mistakes in the cloned sequence (designated "Sbjct") as indicated below.

Table 10: Sequence comparison rat Nogo receptor mRNA sequence (Query) (SEQ ID NO:1623) to cloned sequence (Sbjct) (SEQ ID NO:1624).

```
Query: 1     ATGAAGAGGGCGTCCTCCGGAGGAAGCCGGCTGCCGACATGGGTGTTATGGCTACAGGCC 60
             |||||||||||||||||||||||||||||||||| | |||||||||||||||||||||||
Sbjct: 1     ATGAAGAGGGCGTCCTCCGGAGGAAGCCGGCTGCTGGCATGGGTGTTATGGCTACAGGCC 60

Query: 61    TGGAGGGTAGCAACGCCCTGCCCTGGTGCCTGTGTGTGCTACAATGAGCCCAAGGTCACA 120
             ||||||||||||||||||||||||||||||||||||||||||||| ||||||||||||||
Sbjct: 61    TGGAGGGTAGCAACGCCCTGCCCTGGTGCCTGTGTGTGCTACAATGAACCCAAGGTCACA 120

Query: 121   ACAAGCCGCCCCCAGCAGGGCCTGCAGGCTGTACCCGCTGGCATCCCAGCCTCCAGCCAG 180
             |||||| |||||||||||||||||| |||||||||| |||||||||||||||||||||||
Sbjct: 121   ACAAGCTGCCCCCAGCAGGGCCTGCGGGCTGTACCCACTGGCATCCCAGCCTCCAGCCAG 180

Query: 181   AGAATCTTCCTGCACGGCAACCGAATCTCTTACGTGCCAGCCGCCAGCTTCCAGTCATGC 240
             ||||||||||||||||||||||||||||||||||||||||||||||||||||||||||||
Sbjct: 181   AGAATCTTCCTGCACGGCAACCGAATCTCTTACGTGCCAGCCGCCAGCTTCCAGTCATGC 240

Query: 241   CGGAATCTCACCATCCTGTGGCTGCACTCAAATGCGCTGGCCGGGATTGATGCCGCGGCC 300
             ||||||||||||||||||||||||||||||||||||||||||||||||||||||||||||
Sbjct: 241   CGGAATCTCACCATCCTGTGGCTGCACTCAAATGCGCTGGCCGGGATTGATGCCGCGGCC 300

Query: 301   TTCACTGGTCTGACCCTCCTGGAGCAACTAGATCTTAGTGACAATGCACAGCTCCGTGTC 360
             ||||||||||||||||||||||||||||||||||||||||||||||||||||||||||||
Sbjct: 301   TTCACTGGTCTGACCCTCCTGGAGCAACTAGATCTTAGTGACAATGCACAGCTCCGTGTC 360

Query: 361   GTGGACCCCACCACGTTCCGTGGCCTGGGCCACCTGCACACGCTGCACCTAGACCGATGC 420
             ||||||||||||||||||||||||||||||||||||||||||||||||||||||||||||
Sbjct: 361   GTGGACCCCACCACGTTCCGTGGCCTGGGCCACCTGCACACGCTGCACCTAGACCGATGC 420

Query: 421   GGCCTGCAGGAGCTGGGGCCTGGCCTATTCCGTGGGCTGGCAGCTCTGCAGTACCTCTAC 480
             ||||||||||||||||||||||||||||||||||||||||||||||||||||||||||||
Sbjct: 421   GGCCTGCAGGAGCTGGGGCCTGGCCTATTCCGTGGGCTGGCAGCTCTGCAGTACCTCTAC 480
```

```
Query: 481  CTACAAGACAACAACCTGCAGGCACTTCCCGACAACACCTTCCGAGACCTGGGCAACCTC 540
            ||||||||||||||||||||||||||||||||||||||||||||||||||||||||||||
Sbjct: 481  CTACAAGACAACAACCTGCAGGCACTTCCCGACAACACCTTCCGAGACCTGGGCAACCTC 540

Query: 541  ACGCATCTCTTTCTGCATGGCAACCGTATCCCCAGTGTTCCTGAGCACGCTTTCCGTGGC 600
            ||||||||||||||||||||||||||||||||||||||||||||||||||||||||||||
Sbjct: 541  ACGCATCTCTTTCTGCATGGCAACCGTATCCCCAGTGTTCCTGAGCACGCTTTCCGTGGC 600

Query: 601  TTGCACAGTCTTGACCGTCTCCTCTTGCACCAGAACCATGTGGCTCGTGTGCACCCACAT 660
            ||||||||||||||||||||||||||| ||||||||||||||||||||||||||||||||
Sbjct: 601  TTGCACAGTCTTGACCGTCTCCTCTTGTACCAGAACCATGTGGCTCGTGTGCACCCACAT 660

Query: 661  GCCTTCCGGGACCTTGGCCGACTCATGACCCTCTACCTGTTTGCCAACAACCTCTCCATG 720
            ||||||||||||||||| ||||||||||| ||||||||||||||||||||||||||||||
Sbjct: 661  GCCTTCCGGGACCTTGGTCGACTCATGACTCTCTACCTGTTTGCCAACAACCTCTCCATG 720

Query: 721  CTCCCCGCAGAGGTCCTAGTGCCCCTGAGGTCTCTGCAGTACCTGCGACTCAATGACAAC 780
            ||||||||||||||||||||||||||||||||||||||||||||||||||||||||||||
Sbjct: 721  CTCCCCGCAGAGGTCCTAGTGCCCCTGAGGTCTCTGCAGTACCTGCGACTCAATGACAAC 780

Query: 781  CCCTGGGTGTGTGACTGCAGGGCACGTCCGCTCTGGGCCTGGCTGCAGAAGTTCCGAGGT 840
            ||||||||||||||||||||||||||||||||||||||||||||||||||||||||||||
Sbjct: 781  CCCTGGGTGTGTGACTGCAGGGCACGTCCGCTCTGGGCCTGGCTGCAGAAGTTCCGAGGT 840

Query: 841  TCCTCATCCGGGGTGCCCAGCAACCTACCCCAACGCCTGGCAGGCCGTGATCTGAAGCGC 900
            |||||||||| ||||||| |||||||||||||||||||||||||||||||||||||||||
Sbjct: 841  TCCTCATCCGAGGTGCCCTGCAACCTACCCCAACGCCTGGCAGGCCGTGATCTGAAGCGC 900

Query: 901  CTGGCTACCAGTGACTTAGAGGGTTGTGCTGTGGCTTCGGGGCCCTTCCGTCCCTTCCAG 960
            |||||| |||||||||||||||||||||||||||||||||||||||||||||||||||||
Sbjct: 901  CTGGCTGCCAGTGACTTAGAGGGTTGTGCTGTGGCTTCGGGGCCCTTCCGTCCCTTCCAG 960

Query: 961  ACCAATCAGCTCACTGATGAGGAGCTGCTGGGCCTCCCCAAGTGCTGCCAGCCGGATGCT 1020
            |||||||||||||||||||||| |||||||||||||||||||||||||||||||||||||
Sbjct: 961  ACCAATCAGCTCACTGATGAGGGGCTGCTGGGCCTCCCCAAGTGCTGCCAGCCGGATGCT 1020
```

```
Query: 1021 GCAGACAAGGCCTCAGTACTGGAACCCGGGAGGCCGGCGTCTGTTGGAAATGCACTCAAG 1080
Sbjct: 1021 GCAGACAAGGCCTCAGTACTGGAACCCGGGAGGCCGGCGTCTGCTGGAAATGCACTCAAG 1080

Query: 1081 GGACGTGTGCCTCCCGGTGACACTCCACCAGGCAATGGCTCAGGCCCACGGCACATCAAT 1140
Sbjct: 1081 GGACGTGTGCCTCCCGGTGACACTCCACCAGGCAATGGCTCAGGCCCACGGCACATCAAT 1140

Query: 1141 GACTCTCCATTTGGGACTTTGCCCGGCTCTGCAGAGCCCCCACTGACTGCCCTGCGGCCT 1200
Sbjct: 1141 GACTCTCCATTTGGGACTTTGCCGGGCTCTGCAGAGCCCCCACTGATTGCCCTGCGGCCT 1200

Query: 1201 GGGGGTTCCGAGCCCCCGGGACTGCCCACCACGGGCCCCCGCAGGAGGCCAGGTTGTTCC 1260
Sbjct: 1201 GGGGGTTCCGAGCCCCCGGGACTGCCCACCACGGGTCCCCGCAGGAGGCCAGGTTGTTCC 1260

Query: 1261 AGAAAGAACCGCACCCGTAGCCACTGCCGTCTGGGCCAGGCAGGAAGTGGGAGCAGTGGA 1320
Sbjct: 1261 AGAAAGAACCGCACCCGTAGCCACTGCCGTCTGGGCCAGGCAGGAAGTGGGAGCAGTGGA 1320

Query: 1321 ACTGGGGATGCAGAAGGTTCGGGGGCCCTGCCTGCCCTGGCCTGCAGCCTTGCTCCTCTG 1380
Sbjct: 1321 ACTGGGGATGCAGAAGGTTCGGGGGCCCTGCCTGCCCTGGCCTGCAGCCTTGCTCCTCTG 1380

Query: 1381 GGCCTTGCACTGGTACTTTGGACAGTGCTTGGGCCCTGCTGA 1422
Sbjct: 1381 GGCCTTGCACTGGTACTTTGGACAGTGCTTGGGCCCTGCTGA 1422
```

Transfections:

One day before transfection, Hela cells (LCG Promochem, Wesel, Germany) were seeded at 1.5 x $10^4$ cells / well on 96-well plates (Greiner Bio-One GmbH, Frickenhausen, Germany) in 100 μl of growth medium (Ham's F12 Medium, 10% fetal calf serum, 100u penicillin / 100 μg/ml streptomycin, all from Biochrom AG, Berlin, Germany). 4h prior to siRNA transfection, 100 ng ofNogo-R expression plasmid / well were transfected with Lipofectamine2000 (Invitrogen) as described below for the siRNAs, with the plasmid diluted in Opti-MEM to a final volume of 12.5 μl / well, prepared as a mastermix for the whole plate.

siRNA transfections were performed in triplicates. For each well 0.5 μl Lipofectamine2000 (Invitrogen GmbH, Karlsruhe, Germany) were mixed with 12 μl Opti-MEM (Invitrogen) and incubated for 15 min at room temperature. 2 μl of a 5 μM solution of siRNA in annealing buffer (20 mM sodium phosphate, pH 6.8; 100 mM sodium chloride) were mixed with 10.5 μl Opti-MEM per well, combined with the Lipofectamine2000-Opti-MEM mixture and again incubated for 15 minutes at room temperature. During this incubation, growth medium was removed from cells and replaced by 75 μl / well of fresh medium. The 25 μl solution of siRNA-Lipofectamine2000-complex were added, resulting in an overall 100 nM siRNA concentration in the 100 μl incubation volume, and the cells were incubated for 24 h at 37°C and 5 % $CO_2$ in a humidified incubator (Heraeus GmbH, Hanau).

mRNA levels in cell lysates were quantitated by a commercially available branched DNA hybridization assay (QuantiGene bDNA-kit, Genospectra, Fremont, USA). Cells were harvested by applying 50 μl additional growth medium and 75 μl of Lysis Mixture (from QuantiGene bDNA-kit) to each well and were lysed at 53°C for 30 min.50 μl of the lysates were incubated with probes specific to rat Nogo-R and human GAPDH (sequence of probes see below) according to the manufacturer's protocol for the QuantiGene bDNA kit assay. Finally, chemoluminescence was measured in a Victor2-Light (Perkin Elmer, Wiesbaden, Germany) as RLUs (relative light units) and values obtained with the rat Nogo

receptor probes were normalized to the respective human GAPDH values for each well. Cell transfected with an unspecific siRNA (specific for the gene ApoB) served as controls and for comparison of mRNA levels.

siRNAs were characterized by establishment of dose response curves and calculation of $IC_{50}$ concentrations (the concentration at which 50 % inhibition of gene expression would be observed). For dose response assessment, transfections were performed at the following concentrations: 100 nM, 33.3 nM, 11.1 nM, 3.7 nM, 1.2 nM, 0.4 nM, 137 pM, 46 pM, 15 pM, 5 pM and mock (no siRNA) by serially diluting the 5 μM siRNA stock solution with annealing buffer and using 2 μl of the diluted stock according to the above protocol. The $IC_{50}$ was determined by curve fitting using the computer software Xlfit using the following parameters: Dose Response One Site, 4 Parameter Logistic Model, fit = (A+((B-A)/(1+(((10^C)/x)^D)))), inv = ((10^C)/((((B-A)/(Y-A))-1)^(1/D))), res = (y-fit).

Table 11: Rat Nogo-R probes

| Probe type[1] | Nucleotide Sequence | SEQ ID NO. |
|---|---|---|
| CE | CCTGTAGCCATAACACCCATGTTTTTCTCTTGGAAAGAAAGT | 1594 |
| CE | GACCTTGGGCTCATTGTAGCATTTTTCTCTTGGAAAGAAAGT | 1595 |
| CE | GGCTGGGATGCCAGCGGTTTTTCTCTTGGAAAGAAAGT | 1596 |
| CE | CAGGAAGATTCTCTGGCTGGATTTTTCTCTTGGAAAGAAAGT | 1597 |
| CE | GTAAGAGATTCGGTTGCCGTGTTTTTCTCTTGGAAAGAAAGT | 1598 |
| CE | GAGATTCCGGCATGACTGGATTTTTCTCTTGGAAAGAAAGT | 1599 |
| LE | GGCGTTGCTACCCTCCAGGTTTTTAGGCATAGGACCCGTGTCT | 1600 |
| LE | CACACAGGCACCAGGGCAGTTTTTAGGCATAGGACCCGTGTCT | 1601 |
| LE | AACCCCCAGGCCGCAGGGCTTTTTAGGCATAGGACCCGTGTCT | 1602 |
| LE | TCCCGGCCAGCGCATTTTTTTTAGGCATAGGACCCGTGTCT | 1603 |
| LE | GAAGGCCGCGGCATCAATTTTTAGGCATAGGACCCGTGTCT | 1604 |
| LE | TGTGCATTGTCACTAAGATCTAGTTGTTTTTAGGCATAGGACCCGTGTCT | 1605 |
| BL | CGGCAGCCGGCTTCCTC | 1606 |
| BL | CTGGGGGCGGCTTGTTGT | 1607 |
| BL | GTACAGCCTGCAGGCCCTG | 1608 |
| BL | AGCTGGCGGCTGGCAC | 1609 |
| BL | GAGTGCAGCCACAGGATGGT | 1610 |
| BL | CTCCAGGAGGGTCAGACCAGT | 1611 |

[1]CE = Capture Extender probe; LE = Label Extender probe; BL= blocking probe

Table 12: Human GAPDH probes

| Probe type[1] | Nucleotide sequence | SEQ ID NO. |
|---|---|---|
| CE | GAATTTGCCATGGGTGGAATTTTTTCTCTTGGAAAGAAAGT | 1612 |
| CE | GGAGGGATCTCGCTCCTGGATTTTTCTCTTGGAAAGAAAGT | 1613 |
| CE | CCCCAGCCTTCTCCATGGTTTTTTCTCTTGGAAAGAAAGT | 1614 |
| CE | GCTCCCCCCTGCAAATGAGTTTTTCTCTTGGAAAGAAAGT | 1615 |
| LE | AGCCTTGACGGTGCCATGTTTTTAGGCATAGGACCCGTGTCT | 1616 |
| LE | GATGACAAGCTTCCCGTTCTCTTTTTAGGCATAGGACCCGTGTCT | 1617 |
| LE | AGATGGTGATGGGATTTCCATTTTTTTAGGCATAGGACCCGTGTCT | 1618 |
| LE | GCATCGCCCCACTTGATTTTTTTTTAGGCATAGGACCCGTGTCT | 1619 |
| LE | CACGACGTACTCAGCGCCATTTTTAGGCATAGGACCCGTGTCT | 1620 |
| LE | GGCAGAGATGATGACCCTTTTGTTTTTAGGCATAGGACCCGTGTCT | 1621 |
| BL | GGTGAAGACGCCAGTGGACTC | 1622 |

[1]CE = Capture Extender probe; LE = Label Extender probe; BL = blocking probe

Table 11 lists the agent number, the position of the nucleotide within the human Nogo-R mRNA sequence (Genbank accession number NM_023004) corresponding to the 5'-most nucleotide of the sense strand of the agent, the amount of total Nogo-R mRNA remaining in cells treated with the agent at 100 nM concentration in % of controls, and the $IC_{50}$ value. As evident from the relative activity in reducing total Nogo-R mRNA, and the corresponding $IC_{50}$ values, agents AL-DP-5883, AL-DP-5871, AL-DP-5870, AL-DP-5876, and AL-DP-5872, which are devoid of 2'-O-methyl

groups, are somewhat more active than their 2'-O-methyl-modified counterparts sharing the same base nucleotide sequence, AL-DP-5891, AL-DP-5874, AL-DP-5873, AL-DP-5884, and AL-DP-5875.

Table 11: Ability of siRNAs specific for Nogo-R to reduce Nogo-R mRNA levels in cultured cells

| Agent number | Pos. in mRNA[1] | Rem. Nogo-R mRNA at 100 nM agent | $IC_{50}$ Nogo-R [nM] |
|---|---|---|---|
| AL-DP-5883 | 1484 | 55 ± 3% | 16 |
| AL-DP-5891 | 1484 | 69 ± 8 % | 180 |
| AL-DP-5871 | 1482 | 26 ± 9 % | 0,2 |
| AL-DP-5874 | 1482 | 84 ± 2 % | 5053 |
| AL-DP-5870 | 1017 | 25 ± 9% | 0,5 |
| AL-DP-5873 | 1017 | 60 ± 1 % | 119 |
| AL-DP-5876 | 298 | 26 ± 5% | 0,6 |
| AL-DP-5884 | 298 | 38 ± 9% | 4,5 |
| AL-DP-5872 | 1015 | 27 ± 6 % | 0,3 |
| AL-DP-5875 | 1015 | 83 ± 2 % | 5485 |

[1] Position of nucleotide within human Nogo-R mRNA corresponding to the 5'-most nucleotide of the sense strand of the agent

In summary, AL-DP-5870, AL-DP-5871, AL-DP-5872, and AL-DP-5876 were able to reduce the expression of Nogo-R mRNA by 70 % or more, AL-DP-5884 was able to reduce the expression of Nogo-R mRNA by 60 % or more, and AL-DP-5873 and AL-DP-5883 were able to reduce the expression of Nogo-R mRNA by 40 % or more.

Example 4: Stability testing

In order to verify the stability of siRNAs in the biological matrix most relevant to their intended physiological application, cerebrospinal fluid (CSF), we established a method for determining the degradation half life of siRNAs in this medium. This method comprises the incubation of siRNAs with CSF followed by Proteinase K treatment of the CSF sample and the separation of CSF sample constituents on an HPLC.

The example below shows the analyses of CSF samples which were contacted with siRNAs *in vitro.* However, this method can equally be applied to biological samples *ex vivo*, i.e. obtained from a subject which was contacted with an siRNA *in vivo*.

Bovine CSF was obtained from a calf (*Bos bovis*), age 6 months (Prof. Dr. J. Rehage, University of Veterinary Medicine Hannover, Foundation, Hannover, Germany). Porcine CSF was pooled from 3 healthy weaner pigs (Sus scrofa domesticus), age 3-4 months (Prof. Dr. M. Wendt, University of Veterinary Medicine Hannover, Foundation, Hannover, Germany). Rat CSF was pooled from 20 male Sprague Dawley rats (Rattus norvegicus), 175 - 200 g in weight (Charles River Laboratories, L'Arbresle Cedex, France). Proteinase K (20mg/ml) was obtained from peQLab (Erlangen, Germany; Cat.-No. 04-1075) and diluted 1:1 with deionized water (18,2 mΩ) to a final concentration of 10 mg/ml Proteinase K. Proteinase K Buffer (4.0 ml TRIS-HCl 1M pH 7.5, 1.0ml EDTA 0.5M, 1.2 ml NaCl 5M, 4.0 ml SDS 10%) was prepared fresh and kept at 50 °C until use to avoid precipitation.

A 40 mer of poly(L-dT), $(L\text{-}dT)_{40}$ was obtained from Noxxon Pharma AG (Berlin, Germany) and used as an internal standard. Polymers of the L-enantiomers of nucleic acids show an extraordinary stability towards nucleolytic degradation (Klussman S, et al., Nature Biotechn. 1996, 14:1112) but otherwise very similar properties when compared to naturally occuring nucleic acids consisting of R-enantiomers.

Proteinase K treatment of siRNA incubation samples

6 μl of a 50 μM solution of the respective siRNA in phosphate buffered saline (PBS, Sigma-Aldrich Chemie GmbH, Taufkirchen, Germany) was incubated with 54 μl CSF at 37°C for 30 min, 1, 2, 4, 8, 16, 24 or 48 hours. To terminate the siRNA-degradation, 25 μl of Proteinase K buffer were added to incubation samples immediately after expiry of the respective incubation period, the mixture vortexed at highest speed for 5 s (Vortex Genie 2, Scientific Industries, Inc., Bohemia, NY, USA, cat. no. SI 0256), 8 μl Proteinase K (10 mg/ml) were added followed by vortexing for 5 s, and finally the mixture was incubated for 20 min in a thermomixer at 42°C and 1050rpm.

5 μl of a 50 μM solution (250 pmole) of $(L\text{-}dT)_{40}$ were added as an internal standard to each well, the solution was vortexed for 5 s, and the tube centrifuged for 1 min in a tabletop centrifuge to collect all droplets clinging to the inner surfaces of the wells at the bottom. The solution was transferred to a 96 well Captiva 0.2 μm filter plate (Varian, Germany, Cat. No. A5960002) and filtered by centrifugation at 21900 rcf for 45 min.

The incubation wells were washed with 47.5 μl deionized water (18,2 mΩ), the wash filtered through the Captiva Filter Unit at 21900 rcf for 15 min, and the wash step repeated. Approximately 180 μl of the theoretical total volume of 200 μl are on average recovered after the second washing step.

Ion exchange chromatographic separation of siRNA single strands from each other and from degradation products:

A Dionex BioLC HPLC-system equipped with inline-degasser, autosampler, column oven and fixed wavelength UV-detector (Dionex GmbH, Idstein, Germany) was used under denaturing conditions. Standard run parameters were:

| | |
|---|---|
| Column: | Dionex DNA-Pac100; 4 x 250 mm |
| Temperature: | 75°C |
| Eluent A: | 10 mM $NaClO_4$, 20 mM TRIS-HCl, 1 mM EDTA; 10% acetonitrile, pH = 8.0 |
| Eluent B: | 800 mM $NaClO_4$, 20 mM TRIS-HCl, 1 mM EDTA; 10% acetonitrile, pH = 8.0 |
| Detection: | @ 260nm |
| Gradient: | 0 - 1 min: 10%B<br>1 - 11min: 10% -> 35%B<br>11 - 12min: 35%B -> 100%B<br>12 - 14min: 100%B->10%B<br>14 - 16min: 10%B for column reequilibration |
| Injection volume: | 20 μl |

Where separation between the two strands of an siRNA was not satisfactory or a degradation fragment co-eluted with one strand, the chromatographic parameters were adjusted by changing temperature, pH, replacement of $NaClO_4$ by NaBr, the concentration of acetonitrile, and/or adjusting the slope of the eluent gradient until separation was achieved which allowed separate quantitation of the peaks from sense and antisense strand.

Peak areas for full length strands were obtained by integration of the UV detector signal using software supplied by the manufacturer of the instrument (Chromeleon 6.6; Dionex GmbH, Idstein, Germany).

Data analysis:

Integrated sense strand, antisense strand, and internal standard peak areas were obtained for all samples and the normalization control.

A correction factor CF, accounting for liquid losses in the filtration and washing steps, was determined for every sample by calculating the ratio of experimental to theoretical internal standard peak area. The theoretical internal standard peak area is obtained, e.g. from a calibration curve of the internal standard obtained by injecting 50 μl each of a serial dilution of the 50 μM solution of $(L\text{-}dT)_{40}$ onto the HPLC column, and calculation of the theoretical peak area corresponding to 25 pmole $(L\text{-}dT)_{40}$ with the equation obtained by linear least square fit to the peak areas from the dilution series. The correction factor CF to be applied to the peak areas of the sense and antisense strand is the obtained as:

$$CF = PeakArea_{intStd}\,(theoretical)/PeakArea_{intStd}\,(Sample)$$

This treatment assumes that, by virtue of washing the filter twice, virtually complete recovery is achieved in the combined filtrates, and corrects for the variable volume of wash water retained in the filter, such that peak areas from different samples can be compared.

The peak areas obtained for the sense and antisense strand peaks for each time point are then multiplied with the correction factor CF to obtain Normalized Peak Areas ($NPA_{sense,t}$, $NPA_{antisense,t}$):

$$NPA_{sense\ or\ antisense,t} = (Peak\ Area_{sense\ or\ antisense,t}) \times CF$$

To obtain the relative amount of remaining Full Length Product (%FLP) for the sense and antisense strands at time t, the Normalized Peak Area for each strand at time t = 0 min ($NPA_{sense,t=0}$, $NPA_{antisense,t=0}$) is set as 100%, and

the NPAs from other time points are divided by these values.

$$\%FLP_{t=1,2,3\ldots n} = (NPA_{t=1,2,3\ldots n} / NPA_{t=0}) * 100$$

The value obtained from the control sample, where the siRNA was incubated with annealing buffer only, may serve as a control of the accuracy of the method. The %FLP for both strands should lie near 100%, within error margins, regardless of time of incubation.

The degradation half life $t_{1/2}$ may then be calculated for each strand, assuming first order kinetics, from the slope of a linear least square fit to a plot of ln(%FLP) versus time as:

$$t_{1/2} = \ln(0,5)/\text{slope}$$

Stability of siRNAs specific for NogoL and NogoR in rat, bovine and porcine CSF

Table 12 shows the results for select siRNAs of the determination of the relative amount of full length dsRNA present in porcine, rat, and bovine CSF, and PBS, after 48 h of incubation in the respective medium. In addition, the degradation half life was determined for the sense and antisense strands separately for some siRNAs.

Table 12: Stability of various siRNAs specific for NogoL and NogoR in rat, bovine and porcine CSF

| | % full length duplex present after 48 h in | | | | | | |
|---|---|---|---|---|---|---|---|
| Agent number | Porcine CSF | Rat CSF | Bovine CSF | PBS | Specific for | Modification[1] | C. a. #[2] |
| 6000 | 22 | 5 | 40 | 101 | Nogo-R | 0/TT | - |
| AL-DP-5862 | 81 | | | 100 | Nogo-R | 2/TTs | 6000 |
| AL-DP-5871 | 78 | | | 92 | Nogo-R | 0/23 | 6027 |
| AL-DP-5874 | 90 | | | 93 | Nogo-R | 2/23s | 6027 |
| AL-DP-5876 | 0 | | | 100 | Nogo-R | 0/TTs | 6000 |
| AL-DP-5884 | 39 | | | 97 | Nogo-R | 1/TTs | 6000 |
| AL-DP-5934 | 81 | | | 96 | Nogo-L | 3/TTs | 6197 |
| AL-DP-5938 | 71 | | | 100 | Nogo-L | 3/TTs | 6368 |
| AL-DP-5941 | 84 | | | 99 | Nogo-L | 5/TTs | 6127 |
| AL-DP-5946 | 64 | | | 98 | Nogo-L | 4/TTs | 6329 |
| AL-DP-5948 | 0 | | | 108 | Nogo-L | 3/TTs | 6158 |
| AL-DP-5950 | 9 | | | 107 | Nogo-L | 3/TTs | 6382 |

(continued)

| | % full length duplex present after 48 h in | | | | | | |
|---|---|---|---|---|---|---|---|
| Agent number | Porcine CSF | Rat CSF | Bovine CSF | PBS | Specific for | Modification[1] | C. a. #[2] |
| AL-DP-5963 | 79 | | | 100 | Nogo-L | 3/TTs | 6330 |

[1]0 = no 2'-modifications; 1= 5'-nucleotide in 5'-ua-3', 5'-uu-3', 5'-ca-3', and 5'-ug-3' motifs is 2'-modified in sense strand, 5'-nucleotide in 5'-ua-3' and 5'-ca-3' motifs is 2'-modified in antisense strand; 2 = 5'-nucleotide in 5'-ua-3', 5'-uu-3', 5'-ca-3', and 5'-ug-3' motifs is 2'-modified in sense and antisense strand, 3 = all pyrmidine nucleotides are 2'-modified in sense strand, 5'-nucleotide in 5'-ua-3' and 5'-ca-3' motifs is 2'-modified in antisense strand; 4 = all pyrimidine nucleotides are 2'-modified in sense strand, 5'-nucleotide in 5'-ua-3', 5'-uu-3', 5'-ca-3', and 5'-ug-3' motifs is 2'-modified in antisense strand; 5 = all pyrimidine nucleotides are 2'-modified in sense strand, no 2'-modifications in antisense strand; TT = 21 nucleotides and 3'-terminal TT single strand overhangs in sense and antisense strands; TTs = 21 nucleotides and 3'-terminal TT single strand overhangs in sense and antisense strands; 23 = 21 nucleotide sense, 23 nucleotide antisense strand, 2 nucleotide single strand overhang on 3'-end of antisense strand; 23s = 21 nucleotide sense, 23 nucleotide antisense strand, 2 nucleotide single strand overhang on 3'-end of antisense strand, nucleotides comprise 5'-phosphorothioate groups in positions 21 and 22 of antisense strand

[2] C. a. #= corresponding agent # in Table 1 and Table 2. The agent given under this agent number in Table 1 and Table 2 possesses the same core nucleotide sequence when nucleotide modifications, e.g. 2'-O-methyl modifications and phosphorothioate linkages, are disregarded

As is evident from Table 12, the modification of siRNAs in select sites vulnerable to degradation can lead to agents with excellent properties in terms of activity and stability. For example, AL-DP-5871, AL-DP-5938, and AL-DP-5963 all inhibit their respective target gene by more than 70 % in the *in vitro* assays described above, and more than 70 % full length duplex remain after incubation with porcine CSF for 48 h. However, there is some indication that rat CSF is more aggressive towards siRNAs than porcine or bovine CSF.

## Claims

**1.** An iRNA agent comprising

(i) a sense strand, wherein said sense strand consists of the sequence of SEQ ID NO: 1545, in table 5,
(ii) an antisense strand, wherein said antisense strand consists of the sequence of SEQ ID NO: 1546, in table 5.

**2.** The iRNA agent according to claim 1 for use in treating a human subject having a pathological process mediated in part by Nogo-L or Nogo-A.

**3.** The iRNA agent of claim 2, wherein the pathological process is the inhibition of nerve growth or elongation.

**4.** The iRNA agent of claim 2, wherein the pathological process is the inhibition of nerve growth or elongation as a result of nerve injury or damage.

**5.** The iRNA agent of claim 2, wherein the iRNA agent is to be administered in an amount sufficient to reduce the expression of Nogo-L or Nogo-A in a cell or tissue of the subject.

**6.** The iRNA agent of claim 2, wherein the subject is a human.

**7.** A pharmaceutical composition, comprising:

(a) the iRNA agent of claim 1; and
(b) a pharmaceutically acceptable carrier.

**8.** A method of making a pharmaceutical composition, comprising formulating an iRNA agent of claim 1 with a pharmaceutically acceptable carrier.

9. A method of reducing the expression of a Nogo-L or Nogo-A gene in a cell comprising providing the iRNA agent according to claim 1 to said cell, wherein said iRNA agent is contacted with said cell which is outside an organism.

10. The method of claim 9, wherein said cell is a cell of a cell line.

11. A kit comprising the iRNA agent of claim 1, a sterile container in which the iRNA agent is disclosed, and instructions for use.

## Patentansprüche

1. iRNA-Agens, umfassend

(i) einen Sinnstrang, wobei der Sinnstrang aus der Sequenz der SEQ ID NO: 1545 in Tabelle 5 besteht,
(ii) einen Antisinnstrang, wobei der Antisinnstrang aus der Sequenz der SEQ ID NO: 1546 in Tabelle 5 besteht.

2. iRNA-Agens nach Anspruch 1 zur Verwendung in der Behandlung eines menschlichen Individuums, das einen zum Teil durch Nogo-L oder Nogo-A vermittelten pathologischen Prozess hat.

3. iRNA-Agens nach Anspruch 2, wobei der pathologische Prozess die Inhibierung des Nervenwachstums oder der Nervenverlängerung ist.

4. iRNA-Agens nach Anspruch 2, wobei der pathologische Prozess die Inhibierung des Nervenwachstums oder der Nervenverlängerung als Folge einer Nervenverletzung oder eines Nervenschadens ist.

5. iRNA-Agens nach Anspruch 2, wobei das iRNA-Agens in einer Menge verabreicht werden soll, die ausreicht, um die Expression von Nogo-L oder Nogo-A in einer Zelle oder einem Gewebe des Individuums zu reduzieren.

6. iRNA-Agens nach Anspruch 2, wobei das Individuum ein Mensch ist.

7. Pharmazeutische Zusammensetzung, umfassend

(a) das iRNA-Agens nach Anspruch 1 und
(b) einen pharmazeutisch verträglichen Träger.

8. Verfahren zur Herstellung einer pharmazeutischen Zusammensetzung, umfassend die Formulierung eines iRNA-Agens nach Anspruch 1 mit einem pharmazeutisch verträglichen Träger.

9. Verfahren zur Reduktion der Expression eines Nogo-L- oder Nogo-A-Gens in einer Zelle, umfassend die Bereitstellung des iRNA-Agens nach Anspruch 1 für die Zelle, wobei das iRNA-Agens mit der Zelle, die sich außerhalb eines Organismus befindet, kontaktiert wird.

10. Verfahren nach Anspruch 9, wobei die Zelle eine Zelle einer Zelllinie ist.

11. Kit, umfassend das iRNA-Agens nach Anspruch 1, einen sterilen Behälter, in dem das iRNA-Agens enthalten ist, und Instruktionen zur Verwendung.

## Revendications

1. Agent ARNi comprenant

(i) un brin sens, dans lequel ledit brin sens consiste en la séquence de SEQ ID NO: 1545, dans le tableau 5,
(ii) un brin antisens, dans lequel ledit brin antisens consiste en la séquence de SEQ ID NO: 1546, dans le tableau 5.

2. Agent ARNi selon la revendication 1 pour une utilisation dans le traitement d’un sujet humain ayant un processus pathologique médié en partie par Nogo-L ou Nogo-A.

**3.** Agent ARNi selon la revendication 2, dans lequel le processus pathologique est l'inhibition de la croissance ou de l'élongation de nerf.

**4.** Agent ARNi selon la revendication 2, dans lequel le processus pathologique est l'inhibition de la croissance ou de l'élongation de nerf résultant d'une blessure ou d'un dommage nerveux.

**5.** Agent ARNi selon la revendication 2, dans lequel l'agent ARNi doit être administré en une quantité suffisante pour réduire l'expression de Nogo-L ou Nogo-A dans une cellule ou un tissu du sujet.

**6.** Agent ARNi selon la revendication 2, dans lequel le sujet est un humain.

**7.** Composition pharmaceutique, comprenant :

(a) l'agent ARNi selon la revendication 1 ; et
(b) un véhicule acceptable sur le plan pharmaceutique.

**8.** Méthode pour fabriquer une composition pharmaceutique, comprenant la formulation d'un agent ARNi selon la revendication 1 avec un véhicule acceptable sur le plan pharmaceutique.

**9.** Méthode de réduction de l'expression d'un gène Nogo-L ou Nogo-A dans une cellule comprenant la fourniture de l'agent ARNi selon la revendication 1 à ladite cellule, dans laquelle ledit agent ARNi est mis en contact avec lesdites cellules qui est pratiquée en dehors d'un organisme.

**10.** Méthode selon la revendication 9, dans laquelle ladite cellule est une cellule d'une lignée cellulaire.

**11.** Kit comprenant l'agent ARNi selon la revendication 1, un récipient stérile dans lequel l'agent ARNi est enfermé, et des instructions d'utilisation.

# Cholesterol-RNA 3'-conjugates

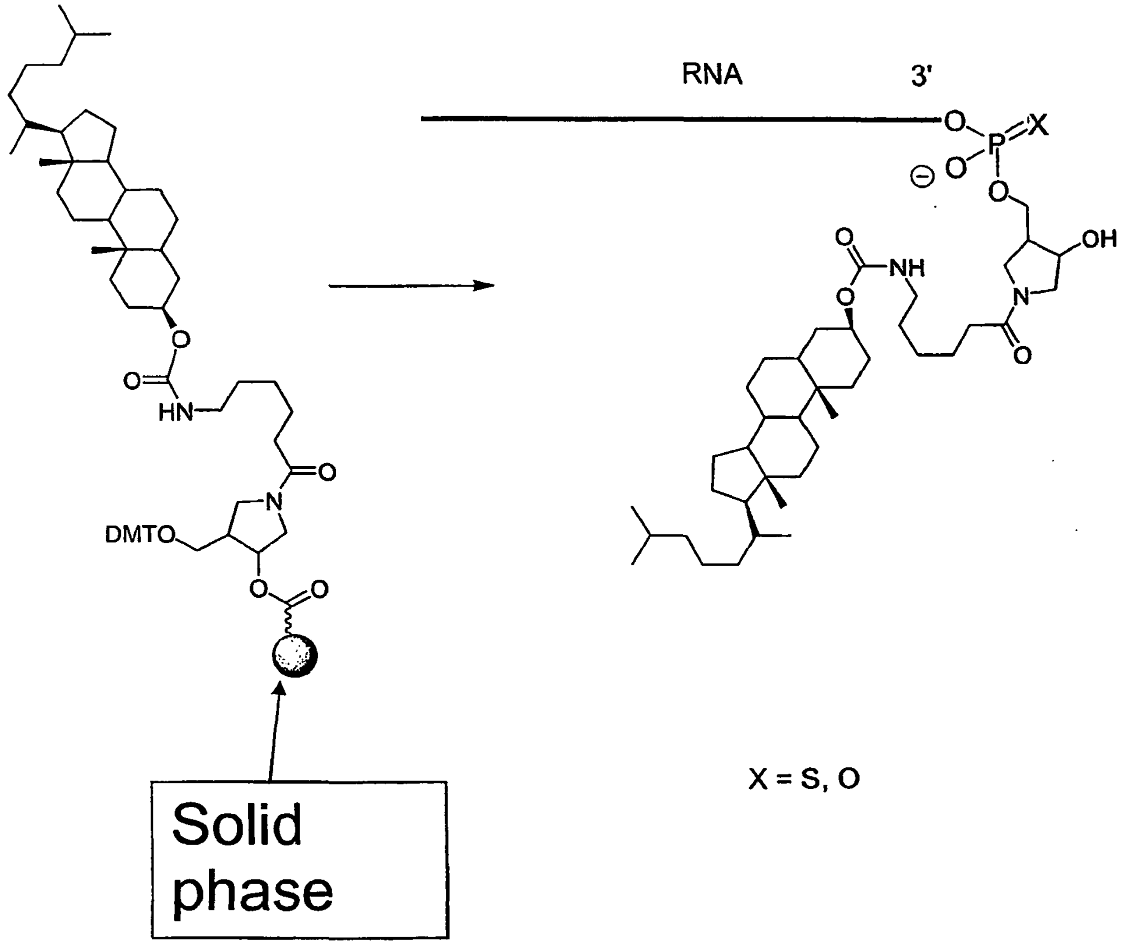


X = S, O

FIG. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 995394547 A, David
- US 5858708 A, Bandman
- WO 9005191 A, Schwab
- US 5684133 A, Schwab
- WO 9417831 A, Schwab
- US 5250414 A, Schwab
- US 6025333 A, Schwab
- US 20030113891 A
- US 20030060611 A
- WO 2004064737 A
- WO 2004094345 A
- WO 2004080406 A
- US 2005107325 A
- US 55991704 P
- US 57474404 P
- US 2005018931 W
- US 5328470 A
- US 6093180 A
- US 09540444 B
- US 09625751 B
- US 5735814 A
- US 5814014 A
- US 6042579 A
- US 09872698 B
- US 09864646 B
- US 6107094 A


### Non-patent literature cited in the description


- **Fire et al.** *Nature,* 1998, vol. 391, 806-811
- **Schwab.** *Neurochem. Res.,* 1996, vol. 21, 755-761
- **Nagase et al.** *DNA Res.,* 1998, vol. 5, 355-364
- **Chen et al.** *Nature,* 2000, vol. 403, 434-439
- **Prinjha et al.** *Nature,* 2000, vol. 403, 383-384
- **Grandpre et al.** *Nature,* 2000, vol. 403, 439-44
- **Nagase et al.** *DNA Res.,* 1998, vol. 31, 355-364
- **Fournier et al.** *Nature,* 2001, vol. 409, 341-346
- **Wang, K.C. et al.** *Nature,* 2002, vol. 420, 74
- **Wong, S.T. et al.** *Nat. Neurosci.,* 2002, vol. 5, 1302
- **Mueller, K. et al.** *Nature Reviews,* 2005, vol. 4, 387
- **GrandPre et al.** *Nature,* 2000, vol. 403, 434-439
- **Spillmann et al.** *J. Biol. Chem.,* 1998, vol. 272, 19283-19293
- **Schnell ; Schwab.** *Nature,* 1990, vol. 343, 269-272
- **Bregman et al.** *Nature,* 1995, vol. 378, 498-501
- **Tatagiba et al.** *Neurosurgery,* 1997, vol. 40, 541-546
- **Savio ; Schwab.** *Proc. Natl. Acad. Sci.,* 1990, vol. 87, 4130-4133
- **Keirstead et al.** *Proc. Natl. Acad. Sci.,* 1992, vol. 89, 11664-11668
- **Dubreuil, C.L et al.** *J. Cell BioL,* 2003, vol. 162, 233
- **Manche et al.** *Mol. Cell. Biol.,* 1992, vol. 12, 5238
- **Lee et al.** *Virology,* 1994, vol. 199, 491
- **Castelli et al.** *J. Exp. Med.,* 1997, vol. 186, 967
- **Zheng et al.** *RNA,* 2004, vol. 10, 1934
- **Heidel et al.** lack of intension response in animals to naked siRNAs. *Nature Biotechn,* 21 November 2004
- **Yang.** *PNAS,* 2002, vol. 99, 9942-9947
- Inducing RNAi with siRNA Cocktails Generated by RNase III. **Byrom et al.** Tech Notes. Ambion, Inc, vol. 10
- **Limbach et al.** *Nucleic Acids Res.,* 1994, vol. 22, 2183-2196
- **Elbashir et al.** *Genes and Dev.,* 2001, vol. 15, 188
- **Nykänen et al.** ATP requirements and small interfering DNA structure in the RNA interference pathway. *Cell,* 2001, vol. 107, 309-321
- **Chen et al.** *Proc. Natl. Acad Sci. USA,* 1994, vol. 91, 3054-3057
- Current protocols in nucleic acid chemistry. John Wiley & Sons, Inc,
- **Prof. Dr. J. Rehage.** *University of Veterinary Medicine Hannover*

- **Prof. Dr. M. Wendt.** *University of Veterinary Medicine Hannover*
- **Klussman S et al.** *Nature Biotechn.,* 1996, vol. 14, 1112